# EUROPEAN PATENT APPLICATION

(11) **EP 4 269 571 A1**
(43) Date of publication of application: **01.11.2023**
(21) Application number: 21911101.0
(22) Date of filing: 24.12.2021
(51) Int. Cl.: C12N 5/10, C12N 7/01, C12N 15/113, C12N 15/12, C12N 15/86

(54) **METHOD FOR PRODUCING NAIVE HUMAN IPS CELLS FROM SOMATIC CELLS**

(30) Priority: 25.12.2020 JP 2020217472
(71) Applicant: Kyoto University, Kyoto-shi, Kyoto 606-8501 (JP); ID Pharma Co., Ltd., Tokyo 102-0071 (JP)
(72) Inventor: KUNITOMI, Akira, Kyoto-shi, Kyoto 606-8501 (JP); SHU, Tsugumine, Tokyo 102-0071 (JP); KAWAGUCHI, Jitsutaro, Tokyo 102-0071 (JP)
(74) Representative: Hoffmann Eitle
(86) International application number: PCT/JP2021/048397
(87) International publication number: WO 2022/138964

(57) **Abstract**

Provided is a method for producing naive induced pluripotent stem cells from human somatic cells, comprising the following steps (1) to (3): (1) introducing one or more vectors containing a reprogramming factor into human somatic cells, (2) culturing said somatic cells in the presence of a naive medium, and (3) after step (2), culturing the resulting cells in the presence of the naive medium under the condition in which the amount of the vectors per the somatic cell is reduced to 30% or less of that at the start of step 3.

## Description

### TECHNICAL FIELD

The present application relates to a method for producing naive induced pluripotent stem cells from human somatic cells.

### BACKGROUND ART

Mouse induced pluripotent stem (iPS) cells and mouse embryonic stem (ES) cells resemble preimplantation blastocyst, and are naive-type cells which are highly undifferentiated. On the other hand, human iPS cells and human ES cells produced by conventional methods resemble postimplantation embryo, and are known to have the characteristics of primed-type cells which are more advanced in development than naive-type cells.

It is noted that compared to mouse iPS cells, which are naive-type, human iPS cells have limited pluripotency, are difficult to be genetically engineered, and have large variations in gene expression and pluripotency among cell lines. It is also noted that one of the main causes of them is that human iPS cells are primed-type cells, which are more advanced in development than naive-type cells. Therefore, a method for producing human pluripotent stem cells having naive-type properties is highly desired for the development of regenerative medicines.

The following methods are reported as methods for producing human naive pluripotent stem cells: (1) a method for converting primed pluripotent stem cells into naive pluripotent stem cells by allowing to express specific genes in the cells and culturing them in a medium containing specific components (Patent Document 1, Non-patent Document 1); (2) a method for converting primed pluripotent stem cells into naive pluripotent stem cells by culturing them in a medium containing specific components (Patent Documents 2-4); and (3) a method for producing naive iPS cells by reprogramming somatic cells (fibroblasts) with a medium containing specific components from the middle of the reprogramming procedures (Non-patent Documents 2 to 4).

### PRIOR ART DOCUMENTS

### PATENT DOCUMENTS

Patent Document 1: WO2016/148253
Patent Document 2: JP2015-136349A
Patent Document 3: WO2016/179243
Patent Document 4: WO2017/170849

### NON-PATENT DOCUMENT

Non-patent Document 1: Takashima Yet al., Cell, 158:1254-1269, 2014
Non-patent Document 2: Theunissen T et al., Cell Stem Cell, 15:471-487, 2014
Non-patent Document 3: Kilens S et al., Nature Communications, 9:360-, 2018
Non-patent Document 4: Liu X et al., Nature Methods, 14:1055-, 2017

### SUMMARY OF THE INVENTION

### PROBLEMS TO BE SOLVED BY THE INVENTION

An objective of the present application is to provide a method for producing naive induced pluripotent stem cells from human somatic cells.

### SOLUTIONS TO THE PROBLEMS

The present application provides the following embodiments.
1. A method for producing naive induced pluripotent stem cells from human somatic cells, comprising the following steps (1) to (3):
   (1) introducing one or more vectors containing a reprogramming factor into human somatic cells,
   (2) culturing said somatic cells in the presence of a naive medium, and
   (3) after step (2), culturing the resulting cells in the presence of the naive medium under the condition in which the amount of the vectors per the somatic cell is reduced to 30% or less of that at the start of step 3.
2. The method according to embodiment 1, wherein the one or more vectors are minus-stranded RNA virus vectors.
3. The method according to embodiment 2, wherein the one or more vectors are paramyxovirus vectors.
4. The method according to embodiment 3, wherein the one or more vectors are Sendai virus vectors.
5. The method according to any one of embodiments 1 to 4, wherein step (2) is started 1 to 10 days after step (1).
6. The method according to any one of embodiments 1 to 5, wherein step (2) is performed for 1 to 20 days.
7. The method according to any one of embodiments 1 to 6, wherein the one or more vectors are selected from the group consisting of a temperature-sensitive vector, a vector containing a target sequence of a microRNA specific for induced pluripotent stem cells, and a temperature-sensitive vector containing a target sequence of a microRNA specific for induced pluripotent stem cells.
8. The method according to embodiment 7, wherein the one or more vectors are temperature-sensitive vectors.
9. The method according to embodiment 8, wherein the one or more vectors are vectors that are temperature-sensitive in the cell culture environment for establishing naive pluripotent stem cells.
10. The method according to embodiment 9, wherein the one or more vectors are Sendai virus vectors with TS7 mutation which has TS mutation (G69E/T116A/A183S mutations in M protein, A262T/G264R/K461G mutations in HN protein, L511F mutation in P protein, and N1197S/K1795E mutations in L protein) and Y942H/L1361C/L1558I mutations in L protein, TS 12 mutation which has TS mutation and D433A/R434A/K437A mutations in P protein, or TS15 mutation which has TS mutation, D433A/R434A/K437A mutations in P protein and L1361C/L1558I mutations in L protein.
11. The method according to any one of embodiments 7 to 10, wherein step (3) comprises culturing the resulting cells at 38°C or higher.
12. The method according to any one of embodiments 7 to 11, wherein the vector containing the target sequence of the microRNA specific for induced pluripotent stem cells is a minus-stranded RNA virus vector, and wherein the target sequence of the microRNA is in the translated region, 5'UTR, or 3'UTR of NP gene or P gene.
13. The method according to embodiment 12, wherein the microRNA is miR-367.
14. The method according to any one of embodiments 1 to 13, wherein the reprogramming factor includes OCT gene, SOX gene, MYC gene and/or KLF gene.
15. The method according to embodiment 14, wherein the one or more vectors include a vector containing OCT gene, SOX gene, and KLF gene, a vector containing MYC gene, and a vector containing KLF gene.
16. The method according to embodiment 15, wherein the vector containing MYC gene is a Sendai virus vector with TS15 mutation.
17. The method according to embodiment 15 or 16, wherein the vector containing OCT gene, SOX gene, and KLF gene, and the vector containing KLF gene are Sendai virus vectors with TS12 mutation.
18. The method according to any one of embodiments 1 to 17, wherein in step (3), the amount of the vectors per the somatic cell is reduced to 30% or less of that at the start of step 3 within 12 days from the start of step 3.
19. The method according to any one of embodiments 1 to 18, wherein the naive medium contains one or more compounds selected from a Leukemia inhibitory factor (LIF), a MEK inhibitor, a GSK3 inhibitor, a cAMP production stimulator, a TGF-β inhibitor, and a PKC inhibitor.
20. The method according to embodiment 19, wherein the naive medium is a medium selected from the group consisting of t2iLGo, 5iLAF, and tt2iLGo.
21. The method according to any one of embodiments 1 to 20, wherein steps (2) and (3) are performed in the absence of feeder cells.
22. The method according to any one of embodiments 1 to 21, wherein the human somatic cells are mononuclear cells or fibroblasts.
23. A kit for producing naive induced pluripotent stem cells from human somatic cells, comprising:
   one or more vectors containing a reprogramming factor, wherein the one or more vectors are selected from the group consisting of a temperature-sensitive vector, a vector containing a target sequence of a microRNA specific for induced pluripotent stem cells, and a temperature-sensitive vector containing a target sequence of a microRNA specific for induced pluripotent stem cells; and
   a naive medium.
24. The kit according to embodiment 23, wherein the one or more vectors are minus-stranded RNA virus vectors.
25. The kit according to embodiment 24, wherein the one or more vectors are paramyxovirus vectors.
26. The kit according to embodiment 25, wherein the one or more vectors are Sendai virus vectors.
27. The kit according to any one of embodiments 23 to 26, wherein the one or more vectors are temperature-sensitive vectors.
28. The kit according to embodiment 27, wherein the one or more vectors are vectors that are temperature-sensitive in the cell culture environment for establishing naive pluripotent stem cells.
29. The kit according to embodiment 28, wherein the one or more vectors are Sendai virus vectors with TS7 mutation which has TS mutation (G69E/T116A/A183S mutations in M protein, A262T/G264R/K461G mutations in HN protein, L511F mutation in P protein, and N1197S/K1795E mutations in L protein) and Y942H/L1361C/L1558I mutations in L protein, TS12 mutation which has TS mutation and D433A/R434A/K437A mutations in P protein, or TS15 mutation which has TS mutation, D433A/R434A/K437A mutations in P protein and L1361C/L1558I mutations in L protein.
30. The kit according to any one of embodiments 23 to 29, wherein the vector containing the target sequence of the microRNA specific for induced pluripotent stem cells is a minus-stranded RNA virus vector, and wherein the target sequence of the microRNA is in the translated region, 5'UTR, or 3'UTR of NP gene or P gene.
31. The kit according to embodiment 30, wherein the microRNA is miR-367.
32. The kit according to any one of embodiments 23 to 31, wherein the reprogramming factor includes OCT gene, SOX gene, MYC gene, and/or KLF gene.
33. The kit according to embodiment 32, wherein the one or more vectors include a vector containing OCT gene, SOX gene, and KLF gene, a vector containing MYC gene, and a vector containing KLF gene.
34. The kit according to embodiment 33, wherein the vector containing MYC gene is a Sendai virus vector with TS15 mutation.
35. The kit according to embodiment 33 or 34, wherein the vector containing OCT gene, SOX gene, and KLF gene, and the vector containing KLF gene are Sendai virus vectors with TS12 mutation.
36. The kit according to any one of embodiments 23 to 35, wherein the naive medium contains one or more compounds selected from a Leukemia inhibitory factor (LIF), a MEK inhibitor, a GSK3 inhibitor, a cAMP production stimulator, a TGF-β inhibitor, and a PKC inhibitor.
37. The kit according to embodiment 36, wherein the naive medium is a medium selected from the group consisting of t2iLGo, 5iLAF, and tt2iLGo.
38. The kit according to any one of embodiments 23 to 37, wherein the human somatic cells are mononuclear cells or fibroblasts.
39. A culture supernatant of the naive induced pluripotent stem cells produced by the method according to any one of embodiments 1 to 22.
40. A cosmetic product containing as raw material a culture supernatant of the naive induced pluripotent stem cells produced by the method according to any one of embodiments 1 to 22.

### EFFECTS OF THE INVENTION

The present application provides a method for producing naive induced pluripotent stem cells from human somatic cells. The present application also provides a set of vectors and kit for producing naive induced pluripotent stem cells from human somatic cells. The naive induced pluripotent stem cells obtained by the method of the present application do not substantially contain the vectors containing reprogramming factors and have high phenotypic stability.

### BRIEF DESCRIPTION OF THE DRAWINGS

Figure 1. Structure of the Sendai virus vector (CytoTune-iPS2.0L) conventionally used for producing naive iPS cells from somatic cells.
Figure 2. Protocol for producing naive iPS cells from human dermal fibroblasts (HDFs). Vertical bars indicate an example of the timing of medium change.
Figure 3A. Phase-contrast microscopy image of HDF-derived naive iPS cells after 45 passages.
Figure 3B. Immunostaining images for pluripotency markers in HDF-derived naive iPS cells after 45 passages. The three side-by-side images are from the same field of view.
Figure 4. Effect of high temperature culture on the amount of the Sendai virus vector genome.
Figure 5. Protocol for producing naive iPS cells from human peripheral blood mononuclear cells (PBMCs). Vertical bars indicate an example of the timing of medium change.
Figure 6. Quantitative evaluation of the amount of the Sendai virus vector genome in PBMC-derived naive iPS cells at each passage from day 14.
Figure 7. Principal component analysis integrating single cell data for each cell type.
Figure 8. Expression patterns of imprinted genes and X chromosome-related genes in each cell type.
Figure 9A. Density bean plot of global methylation of each cell type.
Figure 9B. Principal component analysis of global methylation of each cell type.
Figure 9C. Percentage of 5-methylcytosine to total cytosine in HDF-derived naive iPS cells obtained in this example.
Figure 10. Early differentiation potential of the triploblastic components in HDF- or PBMC-derived naive iPS cells after long-term passages (P16-45). Reset Naive ESCs (in-house) were used as control.
Figure 11A. Phase-contrast image of and immunofluorescent staining for SeV in naive human iPSCs reprogrammed with CytoTune-iPS 2.0L (nCT2.0L_1) on 94 days after SeV infection. Scale bar, 100 µm.
Figure 11B. qRT-PCR analysis of the SeV genome expression in naive iPSCs reprogrammed with the CytoTune 2.0 or 2.0L SeV reprogramming kit. Values are normalized to GAPDH expression and shown as the mean ± s.d. n=3 for each point.
Figure 11C. qRT-PCR analysis of each SeV vector genome expression in naive human iPSCs reprogrammed with CytoTune 2.0 or 2.0L normalized to the GAPDH expression.
Data are shown as the mean ± s.d. n=3 for each point. The inventors used primers designed to specifically detect each SeV vector and SYBR Green in this experiment.
Figure 11D. Schematic structure of the SeV vectors and the modified reprogramming cocktail. The inventors changed the SeV18+KLF4/TSΔF (KLF4/TS) vector in the CytoTune-2.0 or 2.0L reprogramming kit to one of three modified vectors: SeV18+KLF4/TS12ΔF (KLF4/TS12), SeV18+KLF4/PmiR367T2/TSΔF (KLF4/miR/TS) or SeV18+KLF4/PmiR367T2/TS12ΔF (KLF4/miR/TS12). KLF4/miR/TS12 was newly developed in the Examples.
Figure 11E. qRT-PCR analysis of hsa-miRNA367-3p expression in HDFs and PSCs normalized to hsa-miRNA423-3p expression. Data are shown as the mean ± s.d. n=3. ND: not detected even after 40 amplification cycles.
Figure 11F. Experimental design for the induction of naive human iPSCs from HDFs or PBMCs using the modified SeV-OSKL cocktail and incubation temperature shift after the generation of naive human iPSCs.
Figure 11G. Representative phase-contrast image of PBMCs 14 days after CytoTune-iPS 2.0 SeV vector infection. Scale bar, 200 µm.
Figure 12A. Number of naive human iPSC colonies generated from HDFs at day 14. Each of SeV-KLF41TS and the three modified SeV-KLF4 vectors were co-infected with SeV-KOS and SeV-LMYC. Data are shown as the mean ± s.d. n=3. *P<0.05.
Figure 12B. Detection sensitivity of the SeV genome in cells 14 days after SeV vector infection analyzed by qRT-PCR. The X-axis shows the rate of SeV-positive cells among SeV-negative cells.
Figure 12C. qRT-PCR analysis of the SeV genome expression in naive iPSCs derived from HDFs reprogrammed with SeV-KOS, SeV-LMYC and the respective SeV-KLF4 vectors. Data are shown as the mean ± s.d. n=3 of each point.
Figure 12D. qRT-PCR analysis of the SeV genome expression in HDF-derived naive iPSCs (nOSKL_1, 2) and PBMC-derived naive iPSCs (nOSKL_3, 4) reprogrammed with SeV-KOS, SeV-LMYC and SeV-KLF4/miR/TS12 vectors at days 14 and 34. Data are shown as the mean ± s.d. n=3.
Figure 12E. Representative phase-contrast images of naive iPSCs on iMEF feeder cells and feeder-free naive iPSCs, reprogrammed with SeV-KOS, SeV-LMYC and SeV-KLF4/miR/TS12 vectors. Scale bars, 200 µm.
Figure 12F. Cell proliferation rate of established nOSKL_1-4.
Figure 13A. Relative expression of naive pluripotency markers normalized to the GAPDH expression and analyzed by qRT-PCR. The value of nH9 is set to 1.0. Data are shown as the mean ± s.d. n=3.
Figure 13B. Relative expression of primed pluripotency markers normalized to GAPDH expression and analyzed by qRT-PCR. Data are shown as the mean ± s.d. n=3.
Figure 13C. Heatmap of the RNA-seq data depicting expression levels of shared, primed, and naive pluripotency associated marker genes in naive and primed human PSCs.

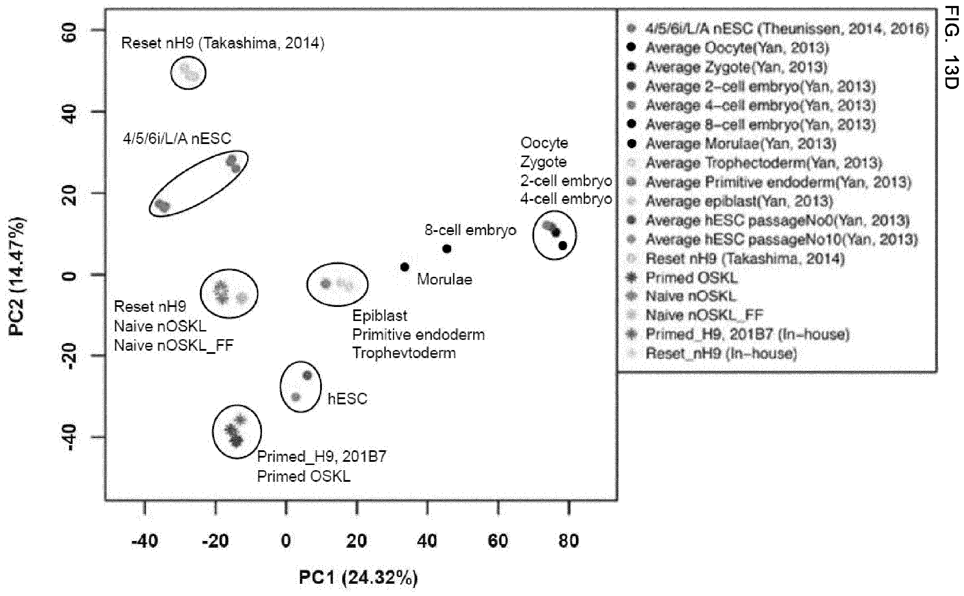

Figure 13D. PCA of RNA-seq data from primed and naive human PSCs in this example compared to reset naive human iPSCs and preimplantation embryo samples from references (Takashima, Y. et al. Resetting transcription factor control circuitry toward ground-state pluripotency in human. Cell 158, 1254-1269, doi: 10.1016/j.cell.2014.08.029 (2014).; Theunissen, T. W. et al. Systematic identification of culture conditions for induction and maintenance of naive human pluripotency. Cell stem cell 15, 471-487, doi:10.1016/j.stem.2014.07.002 (2014).; Yan, L. et al. Single-cell RNA-Seq profiling of human preimplantation embryos and embryonic stem cells. Nature structural & molecular biology 20, 1131-1139, doi:10.1038/nsmb.2660 (2013).).
Figure 14A. Beanplot of the global DNA methylation levels in primed and naive human PSCs analyzed using DNA methylation arrays. Horizontal lines in the beanplot represent mean methylation beta values.
Figure 14B. PCA of global DNA methylation in primed and naive human PSCs using DNA methylation arrays.
Figure 14C. Representative RNA-FISH images of naive and primed human iPSCs detecting X-linked genes UTX (escapes X chromosome inactivation), HUWE1 (subject to X chromosome inactivation), and XIST. UTX was analyzed to ensure only PSCs with normal X chromosome were included in the quantification. Scale bar, 20 µm.
Figure 14D. Quantification of the RNA-FISH patterns for XIST with HUWE1 in cells with bi-allelic UTX expression. 100 cells were analyzed in each cell line. Most naive human iPSCs showed X re-activation as indicated by biallelic HUWE1 expression. Some cells in nOSKL_3 and 4 exhibited biallelic XIST expression in addition to biallelic HUWE1 expression, which is similar to the expression pattern of preimplantation epiblast.
Figure 15A. Heatmap of genes coding proteins of the electron transport chain located in the inner membrane of the mitochondria. These genes reflect the activity of oxidative phosphorylation and are classified by mitochondrion complex and hierarchically clustered.
Figure 15B. Comparison of metabolic capacities of naive and primed iPSCs using extracellular flux analyzer.
Figure 15C. Quantification of spare respiratory capacities of naive and primed iPSCs.
Figure 15D. Extracellular acidification rate (ECAR) and oxygen consumption rate (OCR) in naive and primed iPSCs.
Figure 16A. Schematic representation of the primitive endoderm differentiation.
Figure 16B. Representative phase-contrast images of primitive endoderm from primed ESCs (H9; left panel) and SeV (-) naive iPSCs (nOSKL_4; right panel). Scale bars, 100 µm.
Figure 16C. Quantification of PDGFRA and ANPEP expressions in primed ESCs (H9), naive ESCs (nH9), SeV(-) naive iPSCs (nOSKL_4), and SeV(+) naive iPSCs (nCT2.0L_4) by flow cytometry.
Figure 16D. Median signal values in PDGFRA/ANPEP co-positive cells by flow cytometry.
Figure 17A. Schematic representation of the trophectoderm differentiation.
Figure 17B. Representative phase-contrast images of trophectoderm from primed ESCs (H9; left panel) and SeV (-) naive iPSCs (nOSKL_4; right panel). Scale bars, 200 µm.
Figure 17C. Quantification of TACSTD2, ENPEP, HAVCR1 and HLA-ABC expressions in primed ESCs (H9), naive ESCs (nH9), SeV (-) naive iPSCs (nOSKL_4) and SeV (+) naive iPSCs (nCT2.0L_4) by flow cytometry.
Figure 17D. Percentage of TACSTD2 (+) / ENPEP (+) and HAVCR1 (+) cells by flow cytometry. Each plot shows a single experiment.

### DETAILED DESCRIPTION

In this specification and claims, when a numerical value is accompanied with the term "about", the value is intended to include the range of ± 10% of that value. For example, "about 20" includes "18 to 22". A numerical range includes all values between the two endpoints and the values of both endpoints. When a numerical range is accompanied with "about", "about" is applied to the two endpoints. Accordingly, for example, "about 20 to 30" includes "18 to 33".

### Vector

In the present application, a vector is any vector capable of introducing a gene into a somatic cell, and can be a virus or non-virus vector. For example, the vector is a virus vector. In the present disclosure, a virus vector is a vector that has genomic nucleic acids derived from a virus, and can express a transgene by integrating the transgene into the nucleic acids. For example, the vector of the present application is a non-chromosomal integration virus vector. A non-chromosomal integration virus vector is a virus vector which is derived from a virus and can introduce a gene into a target cell, and refers to a carrier that has no risk of the introduced gene being integrated into a host chromosome (nucleus-derived chromosome). In the present application, a virus vector includes an infectious virus particle, a non-infectious virus particle, virus core, or a complex of virus genome and virus protein which has the ability to express a carried gene upon introduction into a cell. For example, in an RNA virus, a ribonucleoprotein (the core portion of a virus) consisting of a virus genome and a virus protein that binds to the virus genome can be introduced into a cell to express a transgene in the cell (WO00/70055). The introduction into a cell can be carried out using an appropriate transfection reagent. Such a ribonucleoprotein (RNP) is also included in the virus vector of the present application.

In the present application, "no risk of the introduced gene being integrated into a host chromosome" means that the frequency of chromosomal integration of the introduced gene, when the virus vector is introduced into host cells, is sufficiently low. Preferably, the frequency of chromosomal integration is, for example, 5×10⁻⁴ or less, more preferably 10⁻⁴ or less, more preferably 10⁻⁵ or less, more preferably 10⁻⁶ or less, or more preferably 10⁻⁷ or less when human fibrosarcoma-derived cell line HT1080 (ATCC CCL121) is infected with the virus vector at 10 PFU/cell. Anon-chromosomal integration virus vector is preferably an RNA virus. In the present disclosure, an RNA virus refers to a virus that has an RNA genome and has no DNA phase during its lifecycle. In the present disclosure, an RNA virus does not carry reverse transcriptase (i.e., a retrovirus is not included). Accordingly, in virus proliferation, the virus genome is replicated by RNA-dependent RNA polymerase without involving DNA. Since an RNA virus does not have a DNA phase, the use of an RNA virus vector minimizes the risk of chromosomal integration of the introduced gene in the host cells. An RNA virus includes a single-stranded RNA virus (including plus-stranded RNA virus and minus-stranded RNA virus) and double-stranded RNA virus. It also includes a virus with an envelope (an enveloped virus) and a virus without an envelope (a non-enveloped virus), but preferably, a vector derived from an enveloped virus is used. In the present application, an RNA virus specifically includes viruses belonging to the following families:
Arenaviridae family such as Lassa virus;
Orthomyxoviridae family such as influenza virus;
Coronaviridae family such as SARS virus;
Togaviridae family such as rubella virus;
Paramyxoviridae family such as mumps virus, measles virus, Sendai virus, and RS virus;
Picornaviridae family such as poliovirus, Coxsackie virus, and echo virus;
Filoviridae family such as Marburg virus and Ebola virus;
Flaviviridae family such as yellow fever virus, dengue fever virus, hepatitis C virus, and hepatitis G virus;
Bunyaviridae family (including the genera Bunyavirus, Hantavirus, Nairovirus, and Phlebovirus);
Rhabdoviridae family such as rabies virus; and
Reoviridae family.

For example, the non-chromosomal integration virus vector is a minus-stranded RNA virus vector. A minus-stranded RNA virus vector is a vector consisting of a virus containing a minus stranded (an antisense strand to a sense strand encoding a virus protein) RNA as the genome. A minus-stranded RNA is also referred to as a negative-stranded RNA. For example, the minus-stranded RNA virus is a single-stranded minus-stranded RNA virus (also referred to as a non-segmented minus-stranded RNA virus). "Single-stranded negative-stranded RNA virus" refers to a virus having a single-stranded negative-stranded (i.e., minus-stranded) RNAin its genome. Such a virus includes viruses belonging to families such as Paramyxoviridae (including the genera Paramyxovirus, Morbillivirus, Rubulavirus, and Pneumovirus), Rhabdoviridae (including the genera Vesiculovirus, Lyssavirus, and Ephemerovirus), and Filoviridae, and taxonomically belongs to Mononegavirales (Virus vol. 57, no. 1, pp. 29-36, 2007; Annu. Rev. Genet. 32, 123-162, 1998; Fields virology fourth edition, Philadelphia, Lippincott-Raven, 1305-1340, 2001; Microbiol. Immunol. 43, 613-624, 1999; Field Virology, Third edition pp. 1205-1241, 1996).

For example, the minus-stranded RNA virus vector is a paramyxovirus vector. A paramyxovirus vector is a virus vector derived from a Paramyxoviridae family virus. Examples of Paramyxoviridae family virus include Sendai virus. Other examples include Newcastle disease virus, mumps virus, measles virus, respiratory syncytial virus (RS virus), rinderpest virus, distemper virus, simian parainfluenza virus (SV5), and human parainfluenza viruses I, II, and III; influenza virus belonging to the Orthomyxoviridae family; and vesicular stomatitis virus and Rabies virus belonging to the Rhabdoviridae family.

Further examples of the virus which may be used in the present application include Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), Nipah virus (Nipah), human parainfluenza virus-2 (HPIV-2), simian parainfluenza virus 5 (SV5), human parainfluenza virus-4a (HPIV-4a), human parainfluenza virus-4b (HPIV-4b), mumps virus (Mumps), and Newcastle disease virus (NDV). More preferably, examples include viruses selected from the group consisting of Sendai virus (SeV), human parainfluenza virus-1 (HPIV-1), human parainfluenza virus-3 (HPIV-3), phocine distemper virus (PDV), canine distemper virus (CDV), dolphin molbillivirus (DMV), peste-des-petits-ruminants virus (PDPR), measles virus (MV), rinderpest virus (RPV), Hendra virus (Hendra), and Nipah virus (Nipah).

For example, the vector used in the present application is a virus belonging to the Paramyxovirinae family (including the genera Respirovirus, Rubulavirus, and Morbillivirus) or a derivative thereof, and for example, is a virus belonging to the genus Respirovirus (also referred to the genus Paramyxovirus) or a derivative thereof. Examples of the derivative include a virus in which a virus gene is modified so as not to impair the gene transfer ability of the virus, and a chemically modified virus. Examples of the virus of the genus Respirovirus to which the present invention can be applied include human parainfluenza virus type 1 (HPIV-1), human parainfluenza virus type 3 (HPIV-3), bovine parainfluenza virus type 3 (BPIV-3), Sendai virus (also called mouse parainfluenza virus type 1), and simian parainfluenza virus type 10 (SPIV-10). The paramyxovirus is most preferably Sendai virus.

A minus-stranded RNA virus generally contains a complex consisting of RNA and a protein (ribonucleoprotein; RNP) in the interior of the envelope. The RNA included in RNP is (-)-stranded (negative-stranded) single-stranded RNA that is a genome of a minus-stranded RNA virus, and this single-stranded RNA binds to the NP protein, P protein, and L protein to form RNP. The RNA included in this RNP serves as a template for the transcription and replication of the virus genome (Lamb, R.A., and D. Kolakofsky, 1996, Paramyxoviridae: The viruses and their replication, pp.1177-1204. In Fields Virology, 3rd edn. Fields, B. N., D. M. Knipe, and P. M. Howley et al. (ed.), Raven Press, New York, N. Y).

The "NP, P, M, F, HN, and L genes" of a minus-stranded RNA virus refer to the genes encoding nucleocapsid, phosphor, matrix, fusion, hemagglutinin-neuraminidase, and large proteins, respectively. The nucleocapsid (NP) protein is bound to the genomic RNA and is an essential protein in order for the genomic RNA to have template activity. Generally, the NP gene may also be described as "N gene". The phosphor (P) protein is a phosphorylated protein that is a small subunit of RNA polymerase. The matrix (M) protein functions to maintain the virus particle structure in its interior. The fusion (F) protein is a membrane fusion protein involved in the penetration into a host cell, and the hemagglutinin-neuraminidase (HN) protein is a protein involved in the binding with a host cell. The large (L) protein is a large subunit of RNA polymerase. Each of the above-described genes has a transcription control unit, and single mRNA is transcribed from each of the genes, and a protein is transcribed from the mRNA. From the P gene, non-structural protein (C) that is translated by utilizing a different ORF, and protein (V) formed by RNA editing during the reading of P protein mRNA are translated in addition to the P protein. For example, the genes in the viruses belonging to the family Paramyxovirinae are generally described as follows in the order from the 3'-terminus.
Genus Respirovirus N P/C/V M F HN-L
Genus Rubulavirus N P/V M F HN (SH) L
Genus Morbillivirus N P/C/V M F H-L

For example, regarding the accession numbers in the database of the base sequences of various genes of Sendai virus, see M29343, M30202, M30203, M30204, M51331, M55565, M69046, and X17218 for the N gene; M30202, M30203, M30204, M55565, M69046, X00583, X17007, and X17008 for the P gene; D11446, K02742, M30202, M30203, M30204, M69046, U31956, X00584, and X53056 for the M gene; D00152, D11446, D17334, D17335, M30202, M30203, M30204, M69046, X00152, and X02131 for the F gene; D26475, M12397, M30202, M30203, M30204, M69046,X00586, X02808, and X56131 for the HN gene; and D00053, M30202, M30203, M30204, M69040, X00587, and X58886 for the L gene. Furthermore, examples of virus genes encoded by other viruses may include CDV, AF014953; DMV, X75961; HPIV-1, D01070; HPIV-2, M55320; HPIV-3, D10025; Mapuera, X85128; Mumps, D86172; MeV, K01711; NDV, AF064091; PDPR, X74443; PDV, X75717; RPV, X68311; SeV, X00087; SV5, M81442; and Tupaia, AF079780 for the N gene; CDV, X51869; DMV, Z47758; HPIV-1, M74081; HPIV-3, X04721; HPIV-4a, M55975; HPIV-4b, M55976; Mumps, D86173; MeV, M89920; NDV, M20302; PDV, X75960; RPV, X68311; SeV, M30202; SV5, AF052755; and Tupaia, AF079780 for the P gene; CDV, AF014953; DMV, Z47758; HPIV-1, M74081; HPIV-3, D00047; MeV, ABO16162; RPV, X68311; SeV, AB005796; and Tupaia, AF079780 for the C gene; CDV, M12669; DMV Z30087; HPIV-1, S38067; HPIV-2, M62734; HPIV-3, D00130; HPIV-4a, D10241; HPIV-4b, D10242; Mumps, D86171; MeV, AB012948; NDV, AF089819; PDPR, Z47977; PDV, X75717; RPV, M34018; SeV, U31956; and SV5, M32248 for the M gene; CDV, M21849; DMV, AJ224704; HPN-1, M22347; HPIV-2, M60182; HPIV-3, X05303, HPIV-4a, D49821; HPIV-4b, D49822; Mumps, D86169; MeV, AB003178; NDV, AF048763; PDPR, Z37017; PDV, AJ224706; RPV, M21514; SeV, D17334; and SV5, AB021962 for the F gene; CDV, AF112189; DMV, AJ224705; HPIV-1, U709498; HPIV-2. D000865; HPIV-3, AB012132; HPIV-4A, M34033; HPIV-4B, AB006954; Mumps, X99040; MeV, K01711; NDV, AF204872; PDPR, X74443; PDV, Z36979; RPV, AF132934; SeV, U06433; and SV-5, S76876 for the HN (H or G) gene; and CDV, AF014953; DMV, AJ608288; HPIV-1, AF117818; HPIV-2, X57559; HPIV-3, AB012132; Mumps, AB040874; MeV, K01711; NDV, AY049766; PDPR, AJ849636; PDV, Y09630; RPV, Z30698; and SV-5, D13868 for the L gene. A plurality of strains are known for each of the viruses, and genes having sequences other than those exemplified above also exist due to the differences in strains. Sendai virus vectors having virus genes derived from any of these genes are useful as vectors of the present application. Furthermore, in regard to the P protein, the functional site of the protein is a region including the N-binding site, the L-binding site, and the oligomer-forming site on the C-terminal side (in the case of SeV, 320-568 on the C-terminal side of the P protein) (Blanchard L. et al., Virology, (2004) 319, 201-211). It is preferable that the P protein of the present application includes at least this region. For example, the vector of the present application includes a base sequence having at least 90%, preferably at least 95%, at least 96%, at least 97%, at least 98%, or at least 99% identity with the coding sequence of any one of the above-mentioned virus genes (regarding the P gene of SeV, for example, the coding sequence may be a sequence on the C-terminal side, and for example, may be the amino acid sequence from the 479th to 568th amino acids or the amino acid sequence from the 320th to 568th amino acids). Furthermore, the vector of the present application includes, for example, a base sequence encoding an amino acid sequence having at least 90%, preferably at least 95%, at least 96%, at least 97%, at least 98%, or at least 99% identity with the amino acid sequence encoded by the coding sequence of any one of the above-mentioned virus genes (regarding the P protein of SeV, for example, the amino acid sequence may be a sequence on the C-terminal side, and for example, may be the amino acid sequence from the 479th to 568th amino acids or the amino acid sequence from the 320th to 568th amino acids). Furthermore, the vector of the present application includes, for example, a base sequence encoding a polypeptide including an amino acid sequence that has substitutions, insertions, deletions, and/or additions of ten or less, preferably nine or less, eight or less, seven or less, six or less, five or less, four or less, three or less, two or less, or one amino acid with respect to the amino acid sequence encoded by the coding sequence of any one of the above-mentioned virus genes (regarding the P gene of SeV, for example, the amino acid sequence may be a sequence on the C-terminal side, and for example, may be the amino acid sequence from the 479th to 568th amino acids or the amino acid sequence from the 320th to 568th amino acids).

The sequence referred by the database accession number, such as base sequences and amino acid sequences described in the this specification, refer to the sequence on, for example, the filing date and priority date of the present application and can be identified as the sequence on either of the filing date or the priority date of the present application. Preferably, the sequence is identified as the sequence on the filing date of the present application. The sequence at each time point can be identified by referring to the revision history of the database.

Furthermore, the minus-stranded RNA virus of the present application may be derived from a natural strain, a wild-type strain, a mutant strain, a laboratory-subcultured strain, and an artificially established strain. An example thereof is Sendai virus Z strain (Medical Journal of Osaka University, Vol. 6, No. 1, March 1955, p. 1-15). In other words, the virus may be a virus vector having a structure similar to that of a virus isolated from nature, or may be a virus artificially modified by genetic recombination. For example, the virus may have a mutation or deletion in any of the genes of a wild-type virus. For example, a virus having a mutation or deletion in at least one gene encoding an envelope protein or a coat protein of the virus can be suitably used. Such a virus vector is a virus vector that can, for example, replicate the genome in infected cells but cannot form infectious virus particles. Since there is no risk that such a transmission-defective virus vector may spread infection to the surroundings, the virus vector is highly safe. For example, a minus-stranded RNA virus that does not contain at least one gene encoding an envelope protein or a spike protein such as the F protein and/or HN protein, or a combination thereof can be used (WO 00/70055 and WO 00/70070; Li, H.-O. et al., J. Virol. 74 (14) 6564-6569 (2000)). When the proteins necessary for genome replication (e.g., the N protein, P protein, and L protein) are encoded in the genomic RNA, the genome can be amplified in infected cells. In order to produce a defective type virus, for example, the deleted gene product or a protein that can complement the gene product is exogenously supplied to the virus-producing cell (WO 00/70055 and WO 00/70070; LI, H.-O. et al., J. Virol. 74 (14) 6564-6569 (2000)). Furthermore, a method of collecting a virus vector as non-infectious virus-like particles (VLP) without completely complementing the deleted virus protein is also known (WO 00/70070). Furthermore, in the case of collecting a virus vector as RNP (e.g., RNP consisting of the N, L, and P proteins and genomic RNA), the vector can be produced without complementing an envelope protein.

The virus of the present application is not limited to a naturally-occurring virus, and for example, artificially produced viruses are also included in the virus. For example, the virus of the present application also includes a virus in which mutations have been introduced into the nucleic acid sequence in order to optimize codons, chimeric viruses (including, for example, a chimera between homogenous viruses, and a chimeric virus between heterogeneous viruses (for example, a chimera between PIV and SeV)), and the like (J. Virol. 1995, 849-855).

According to the present application, the virus proteins such as the N, L, and P proteins may not be wild-type proteins as long as they maintain the function of expressing genes in transduced cells. For example, a modified protein in which a peptide such as a tag has been appropriately added, a protein having modified codons, and a protein in which a portion of the amino acid sequence of the wild type protein has been deleted so as not to lose the function can be used as appropriate. According to the present application, the N, L, and P proteins also include such modified proteins and deletion type proteins. For example, as long as the P protein has a portion of the C-terminus, other regions are not essential for the expression of the virus vector.

A virus vector is, for example, a complex of RNA which is derived from the (-)-stranded single-stranded RNA genome of the virus and a protein which is derived from a virus protein binding to the (-)-stranded single-stranded RNA of the virus and which binds to the RNA, and is a vector that expresses a carried gene when introduced into cells. The protein that binds to (-)-stranded single-stranded RNA refers to a protein that binds directly and/or indirectly to the (-)-stranded single-stranded RNA and forms a complex with the (-)-stranded single-stranded RNA. The complex includes a complex consisting of (-)-stranded single-stranded RNA derived from a minus-stranded RNA virus, and a protein which is derived from a minus-stranded RNA virus and binds to the RNA (e.g., NP, P, and L proteins). According to the present application, the phrase "derived from a minus-stranded RNA virus" means that the constituents (including proteins and RNAs) of a minus-stranded RNA virus are intact or partially modified. For example, a protein or RNA produced by modifying a protein or RNA of a minus-stranded RNA virus is a protein or RNA "derived from a minus-stranded RNA virus" For example, the vector of the present application may be a virus vector having envelope proteins (F, HN, and M proteins) and having a structure of a virus particle. Furthermore, the vector may also be a RNP vector, which is RNP itself and does not have a virus envelope.

In regard to the minus-stranded RNA virus, the NP, P, and L proteins bind to (-)-stranded single-stranded RNA and perform essential functions in genomic RNA replication and protein expression (in the following description, in some cases, the NP, P, and L proteins are referred to as "genomic RNA-binding proteins"). The NP protein is a protein that binds very strongly to genomic RNA and confers template activity on the genomic RNA. The genomic RNA has template activity for RNA synthesis only when bound to the NP protein and has no template activity at all when not bound to the NP protein. The P protein binds to the genomic RNA as a small subunit of RNA polymerase, and the L protein binds to the genomic RNA as a large subunit of RNA polymerase. Therefore, in a minus-stranded RNA virus, genomic RNA replication does not occur if any one of the NP, P, and L proteins is absent.

The gene contained in the genomic RNA of the vector of the present application may be an intact virus-derived gene sequence, or some mutations may be introduced in the gene. For example, a person skilled in the art can introduce minor mutations that does not impair the function of each protein into each gene on the genomic RNA by known methods. For example, it is possible to introduce site-specific mutations by a PCR method or a cassette mutagenesis method, or random mutations by chemical reagents or random nucleotides.

For example, in envelope proteins or spike proteins, many mutations including attenuated mutations and temperature-sensitive mutations are known. Viruses having these mutated protein genes can be suitably used in the present application. According to the present application, a vector with reduced cytotoxicity can be desirably used. The cytotoxicity of a vector can be measured by, for example, quantifying the release of lactate dehydrogenase (LDH) from vector-infected cells. The lower the LDH release, the lower the cytotoxicity. For example, a vector having significantly reduced cytotoxicity compared to the wild type can be used. For example, regarding the degree of the reduction of cytotoxicity, a vector can be used which significantly reduces the amount of released LDH (e.g., by 20% or more, 25% or more, 30% or more, 35% or more, 40% or more, or 50% or more) compared to the wild type in a culture medium obtained by infecting human-derived HeLa cells (ATCC CCL-2) or simian-derived CV-1 cells (ATCC CCL 70) with the vector at MOI (multiplicity of infection) of 3, and culturing the cells for three days at 35°C to 37°C (e.g., 37° C). Furthermore, mutations that reduce cytotoxicity also include temperature-sensitive mutations.

The temperature-sensitivity, in which the activity is significantly reduced at normal temperatures (e.g., 37 to 38°C) compared to low temperatures (e.g., 30 to 36.5°C), can be determined by measuring the proliferation rate of the virus or the expression level of a carried gene at the normal temperature of the virus host. It is considered that the lower the proliferation rate of the virus and/or the expression level of the carried gene compared to those without mutations, the higher the temperature sensitivity.

The virus vector which may be used for the present application has a deletion or mutation in preferably at least one, and more preferably at least two, three, four, five, or more virus genes. The deletion and mutation may be introduced in any combination into each gene. The mutation may be a function-impaired mutation or a temperature-sensitive mutation. The use of such a modified virus vector can be useful particularly for reducing cytotoxicity in host cells or facilitating the removal of the vector. For example, a virus vector that is suitably used in the present application has at least two virus genes deleted or mutated. Such a virus includes a virus having at least two virus genes deleted, a virus having at least two virus genes mutated, and a virus having at least one virus gene mutated and at least one virus gene deleted. The at least two virus genes mutated or deleted are preferably genes encoding envelope component proteins. For example, it is preferable that the minus-stranded RNA virus vector of the present application is deficient in at least the F gene or M gene. For example, a vector that lacks the F gene, and further lacks the M and/or HN gene, or has further mutation (e.g., temperature-sensitive mutation) in the M and/or HN gene, is suitably used in the present application. Further, the vector used in the present application more preferably has at least three virus genes (preferably at least three genes encoding envelope component proteins; F, HN, and M) deleted or mutated. Such a virus vector includes a virus vector having at least three genes deleted, a virus vector having at least three genes mutated, a virus vector having at least one gene mutated and at least two genes deleted, and a virus vector having at least two genes mutated and at least one gene deleted. According to a more preferred aspect, for example, a vector that lacks the F gene, and further lacks the M and/or HN gene, or has further mutation (e.g., temperature-sensitive mutation) in the M and/or HN gene, is suitably used in the present application. Further, for example, a vector that lacks the F gene, further lacks the M or HN gene, and has further mutation in the remaining M or HN gene (e.g., temperature-sensitive mutation) is suitably used in the present application. Such a mutated type virus can be produced according to known methods.

Examples of the temperature-sensitive mutation of the M gene include amino acid substitution at a site arbitrarily selected from the group consisting of positions 69 (G69), 116 (T116), and 183 (A183) of the M protein of Sendai virus; or amino acid substitution at the corresponding sites of the M protein of a minus-stranded RNA virus (Inoue, M. et al., J. Virol. 2003, 77:3238-3246). A virus having a genome encoding a mutant M protein in which an amino acid at any one of the above-mentioned three sites, preferably amino acids at a combination of any two sites, and more preferably amino acids at all of the three sites in the M protein are substituted by other amino acids, is suitably used in the present application.

The amino acid mutation is preferably substitution to another amino acid with a side chain having different chemical properties, and for example, substitution to an amino acid with a BLOSUM62 matrix (Henikoff, S. and Henikoff, J. G. (1992) Proc. Natl. Acad. Sci. USA 89:10915-10919) score of 3 or less, preferably 2 or less, more preferably 1 or less, and more preferably zero. Specifically, for Sendai virus M protein, G69, T116, and A183 can be substituted by Glu (E), Ala (A), and Ser (S), respectively. For the M protein of other minus-stranded RNA viruses, the amino acids at the corresponding sites can be substituted by Glu (E), Ala (A), and Ser (S), respectively. Further, mutations homologous to the mutations in the M protein of measles virus temperature-sensitive strain P253-505 (Morikawa Y. et al., Kitasato Arch. Exp. Med. 1991; 64; 15-30) can also be utilized. For example, the mutations may be introduced by using oligonucleotides according to known mutagenesis methods.

In this specification, the amino acid sequence of Sendai virus is based on the amino acid sequence of the F gene-deficient Sendai virus from strain Z listed in GenBank accession number: AB855655. Therefore, the Xth amino acid residue of Sendai virus means the amino acid residue corresponding to the Xth amino acid residue in the amino acid sequence listed in GenBank accession number: AB855655. In this specification, "position corresponding to" a given position of an amino acid sequence means the position of the other amino acid sequence that corresponds to the given position of the amino acid sequence in the alignment of the amino acid sequence with the other amino acid sequence. The alignment can be performed, for example, using known genetic analysis software. Specific genetic analysis software includes DNASIS from Hitachi Solutions, GENETYX from Genetyx, and FASTA, BLAST, and ClustalW published by DDBJ.

Examples of the temperature-sensitive mutation of the HN gene include amino acid substitution at a site arbitrarily selected from the group consisting of positions 262 (A262), 264 (G264), and 461 (K461) of the HN protein of Sendai virus; or amino acid substitution at the corresponding sites of the HN protein of a minus-stranded RNA virus (Inoue, M. et al., J. Virol. 2003, 77:3238-3246). A virus having a genome encoding a mutant HN protein in which an amino acid at any one of the above-mentioned three sites, preferably amino acids at a combination of any two sites, and more preferably amino acids at all of the three sites are substituted by other amino acids, is suitably used in the present application. As described above, the substitution of an amino acid is preferably substitution to another amino acid with a side chain having different chemical properties. According to a preferred example, A262, G264, and K461 of the Sendai virus HN protein are substituted by Thr (T), Arg (R), and Gly (G), respectively. For the HN protein of other minus-stranded RNA viruses, the amino acids at the corresponding sites can be substituted by Thr (T), Arg (R), and Gly (G), respectively. Further, for example, with reference to temperature-sensitive vaccine strain Urabe AM9 of mumps virus, mutations can be introduced into the amino acids at positions 464 and 468 of the HN protein (Wright, K. E. et al., Virus Res. 2000: 67; 49-57).

The vector of the present application may also have mutations in the P gene and/or L gene. Specific examples of such mutations include mutation of Glu at position 86 (E86) of the SeV P protein, and substitution of Leu at position 511 (L511) of the SeV P protein with another amino acid. For the P protein of other minus-stranded RNA viruses, substitutions at the corresponding sites may also be included. As described above, the substitution of an amino acid is preferably substitution to another amino acid with a side chain having different chemical properties. Specific examples include substitution of the amino acid at position 86 to Lys and substitution of the amino acid at position 511 to Phe. In regard to the L protein, substitution of Asn at position 1197 (N1197) and/or Lys at position 1795 (K1795) of the SeV L protein to other amino acids, and substitution at the corresponding sites of the L protein of other minus-stranded RNA viruses may be included. As described above, the substitution of an amino acid is preferably substitution to another amino acid with a side chain having different chemical properties. Specific examples include substitution of the amino acid at position 1197 to Ser, and substitution of the amino acid at position 1795 to Glu. The mutations in the P gene and the L gene can significantly enhance the effect of sustained infectivity, suppression of secondary particle release, or suppression of cytotoxicity. Further, these effects can be dramatically enhanced by combining mutations and/or deletions in the envelope protein genes. Furthermore, regarding the L gene, examples include substitution of Tyr at position 1214 (Y1214) and/or Met at position 1602 (M1602) of the SeV L protein to other amino acids, and substitution at the corresponding sites of the L protein of other minus-stranded RNA viruses. As described above, the substitution of an amino acid is preferably substitution to another amino acid with a side chain having different chemical properties. Specific examples include substitution of the amino acid at position 1214 to Phe, and substitution of the amino acid at position 1602 to Leu. The mutations exemplified above can be used in any combinations.

For example, Sendai virus vectors in which at least G at position 69, T at position 116, and A at position 183 of the SeV M protein; at least A at position 262, G at position 264, and K at position 461 of the SeV HN protein; at least L at position 511 of the SeV P protein; and at least N at position 1197 and K at position 1795 of the SeV L protein are substituted by other amino acids, and in which the F gene is deficient or deleted; and an F gene-deficient or F gene-deleted Sendai virus vector having cytotoxicity at a level equal to or lower than these vectors and/or temperature sensitivity at a level equal to or higher than these vectors, are particularly suitable. For other minus-stranded RNA viruses, vectors in which corresponding sites are similarly substituted and the F gene is deficient or deleted; and F gene-deficient or F gene-deleted vectors having cytotoxicity at a level equal to or lower than these vectors and/or temperature sensitivity at a level equal to or higher than these vectors, are preferred. Specific examples of the substitution include substitutions of G69E, T116A, and A183S for the M protein; substitutions of A262T, G264R, and K461G for the HN protein; substitution of L511F for the P protein; and substitutions of N1197S and K1795E for the L protein.

Amino acid mutation may be substitution to other desired amino acids; however, the mutation is preferably substitution to another amino acid with a side chain having different chemical properties, as described above. For example, the amino acids can be classified into groups such as basic amino acids (for example, lysine, arginine, and histidine), acidic amino acids (for example, aspartic acid and glutamic acid), non-charged polar amino acids (for example, glycine, asparagine, glutamine, serine, threonine, tyrosine, and cysteine), non-polar amino acids (for example, alanine, valine, leucine, isoleucine, proline, phenylalanine, methionine, and tryptophan), β-branched amino acids (for example, threonine, valine, and isoleucine), and aromatic amino acids (for example, tyrosine, phenylalanine, tryptophan, and histidine). The amino acid mutation includes the substitution of a certain amino acid to an amino acid other than amino acids in the group to which the amino acid belongs. Specific examples include, but are not limited to, substitution of a basic amino acid to an acidic or neutral amino acid; substitution of a polar amino acid to a non-polar amino acid; substitution of an amino acid having a molecular weight larger than the average molecular weight of the twenty naturally occurring amino acids to an amino acid having a molecular weight smaller than the average molecular weight; and conversely, substitution of an amino acid having a molecular weight smaller than the average molecular weight to an amino acid having a molecular weight larger than the average molecular weight.

Examples of the mutation of the L protein include substitutions of amino acids at sites arbitrarily selected from positions 942 (Y942), 1361 (L1361), and 1558 (L1558) of the SeV L protein, or substitutions of amino acids at the corresponding sites of the L protein of minus-stranded RNA viruses to other amino acids. As described above, the substitution of an amino acid is preferably substitution to another amino acid with a side chain having different chemical properties. Specific examples may include substitution of the amino acid at position 942 to His, substitution of the amino acid at position 1361 to Cys, and substitution of the amino acid at position 1558 to Ile. Particularly, an L protein in which at least position 942 or position 1558 is substituted can be suitably used. For example, a mutant L protein in which the amino acids at position 1558 as well as position 1361 are substituted to other amino acids is also suitable. Further, a mutant L protein in which the amino acids at position 942 as well as position 1558 and/or position 1361 are substituted to other amino acids is also suitable. These mutations can increase the temperature sensitivity of the L protein.

Examples of the mutations in the P protein include substitutions of amino acids at sites arbitrarily selected from positions 433 (D433), 434 (R434), and 437 (K437) of the SeV P protein; or substitutions of amino acids at the corresponding sites of the P protein of minus-stranded RNA viruses to other amino acids. As described above, the substitution of an amino acid is preferably substitution to another amino acid with a side chain having different chemical properties. Specific examples may include substitution of the amino acid at position 433 to Ala (A), substitution of the amino acid at position 434 to Ala (A), and substitution of the amino acid at position 437 to Ala (A). In particular, a P protein in which the amino acids at all of these three sites are substituted can be suitably used. These mutations can increase the temperature sensitivity of the P protein.

The temperature-sensitive mutations that can be included in the vector of the present application are described in detail in WO 2012/029770, WO 2010/008054, and WO 2003/025570. Preferably, the P protein is a mutant P protein in which at least the amino acids at three sites, namely, D at position 433, R at position 434, and K at position 437, are substituted by other amino acids. In addition, an F gene-deficient or F gene-deleted Sendai virus vector encoding a mutant L protein in which at least L at position 1558 is substituted (preferably, a mutant L protein in which at least L at position 1361 is also substituted by another amino acid), and an F gene-deficient or F gene-deleted Sendai virus vector having cytotoxicity at a level equal to or lower than the vector and/or temperature sensitivity at a level equal to or higher than the vector, are suitably used in the present application. Each virus protein may have mutations of amino acids (e.g., ten or less, five or less, four or less, three or less, two or less, or one amino acid) other than the mutations described above. The vectors having the mutations described above are highly temperature sensitive.

The genomic RNA in the vector of the present application may contain all of the envelope protein genes, or may not contain some or all of the envelope protein genes. The envelope protein genes (M gene, F gene, and HN gene) that are contained in the genomic RNA may be of wild type, or may have temperature-sensitive mutations introduced. The temperature-sensitive mutations of the envelope proteins are described in detail in WO 2012/029770, WO 2010/008054, and WO 2003/025570.

For the production of virus vectors, the desired exogenous envelope protein can be expressed in virus-producing cells to produce virus vectors containing it. There are no particular limitations on such a protein, and any desired proteins, such as an adhesion factor, a ligand, and a receptor, that confer infectivity to mammalian cells can be used. A specific example may be the G protein (VSV-G) of vesicular stomatitis virus (VSV). The VSV-G protein may be derived from any VSV strain. For example, the VSV-G protein derived from the Indiana serotype strain (J. Virology 39: 5 19-528 (1981)) can be used, but the VSV-G protein is not limited to it. The virus vector of the present application can contain any combination of envelope proteins derived from other viruses.

Temperature sensitivity according to the present application means that the activity changes depending on the culture temperature. For example, regarding an expression vector, a temperature-sensitive vector is a vector whose expression level changes depending on the culture temperature, and the temperature-sensitive vector whose expression level at a certain temperature within the range of 30 to 36.5°C is significantly higher than that at a temperature 0.5 to 5°C, preferably 1 to 4°C, for example about 3°C higher than such temperature are suitably used. For example, TS7 which has TS mutation (G69E/T116A/A183S mutations in the M protein, A262T/G264R/K461G mutations in the HN protein, L511F mutation in the P protein, and N1197S/K1795E mutations in the L protein) and Y942H/L1361C/L1558I mutations in the L protein, TS12 which has TS mutation and D433A/R434A/K437A mutations in the P protein, TS13 which has TS mutation, D433A/R434A/K437A mutations in the P protein and the L1558I mutation in the L protein, TS 14 which has TS mutation, D433A/R434A/K437A mutations in the P protein and L1361C in the L protein, and TS15 which has TS mutation, D433A/R434A/K437A mutations in the P protein and L1361C/L1558I mutations in the L protein of Sendai virus, detailed in WO2012/029770 and WO2010/008054, are preferred temperature-sensitive mutations.

A specific example of the vector may be an F gene-deleted Sendai virus vector (e.g., Z strain) having TS7, TS12, TS13, TS14, or TS 15 mutation. Specifically, examples include, but are not limited to, the vector obtained by further introducing TS7, TS12, TS13, TS14, or TS15 mutation into SeV18+/TSΔF (WO 2010/008054 and WO 2003/025570), or SeV(PM)/TSΔF.

Note that "TSΔF" refers to a combination of G69E, T116A, and A183S mutations in the M protein; A262T, G264R, and K461G mutations in the HN protein; L511F mutation in the P protein; and N1197S and K1795E mutations in the L protein, and deletion of the F gene.

Preferably, the vector of the present application is a Sendai virus vector derived from Sendai virus Z strain. The genomic sequence of the Z strain is known, and an example of the genomic sequence of a Z strain-derived F gene-deficient Sendai virus vector may be ACCESSION: AB855655. "Derived from strain Z" means that the sequence of strain Z accounts for the highest percentage of the Sendai virus genomic sequence in the genome of the vector. In other words, it means that when non-Sendai virus sequences (sequences of restriction enzyme recognition sites or simple spacers, sequences derived from viruses of other species, sequences of genes to be introduced, and the like) are excluded and only the sequences of the Sendai virus are collected from the genomic sequence of the vector, the sequences of the SeV Z strain account for the highest percentage among the sequences. More preferably, "derived from strain Z" means that the sequence of strain Z accounts for the highest percentage of the minus-stranded RNA virus-derived sequence in the genome of the vector. In other words, it means that when sequences that are not from minus-stranded RNA viruses (sequences of restriction enzyme recognition sites or simple spacers, sequences derived from viruses other than minus-stranded RNA viruses, sequences of genes to be introduced, and the like) are excluded and only the sequences of minus-stranded RNA virus are collected from the genomic sequence of the vector, the sequences of the SeV Z strain account for the highest percentage among the sequences. The percentage is preferably 30% or higher, more preferably 40% or higher, 50% or higher, 60% or higher, 70% or higher, 80% or higher, 90% or higher, 95% or higher, 96% or higher, 97% or higher, 98% or higher, or 99% or higher. Preferably, the genome of a Sendai virus vector derived from the Z strain contains the genomic sequence of non-Z strain Sendai virus (excluding the same sequence as the Z strain) in, for example, only 1,000 or less, 500 or less, 300 or less, 200 or less, 100 or less, 50 or less, 30 or less, or 20 or less consecutive bases. More preferably, the genome of a Sendai virus vector derived from the Z strain does not contain any genomic sequence of non-Z strain Sendai virus (excluding the same sequence as the Z strain).

The vector of the present application may be produced by using known methods. Regarding the specific procedures, the minus-stranded RNA virus can be produced typically by (a) a step of transcribing a cDNA encoding the minus-stranded RNA virus genomic RNA (minus stranded) or the complementary strand thereof (plus stranded) in cells that express virus proteins (NP, P, and L) necessary for virus particle formation; and (b) a step of collecting the virus. The virus is not limited to an infectious particle, and includes a non-infectious particle and RNP as long as it retains the ability to express a gene when introduced into a cell. The virus proteins necessary for virus formation may be expressed from transcribed virus genome RNA or may be supplied from sources other than genomic RNA. For example, the virus proteins can be supplied by introducing expression plasmids encoding the NP, P, and L proteins into cells. When the genomic RNA lacks the virus genes necessary for virus formation, the virus genes can be separately expressed in virus-producing cells to complement virus formation. In order to express a virus protein or RNA genome in a cell, a vector in which a DNA encoding the protein or genomic RNA is linked downstream of an appropriate promoter that functions in a host cell is introduced into the host cell. The transcribed genomic RNA is replicated in the presence of virus proteins to form virions. When a defective type virus that is deficient in genes such as the genes of envelope proteins is produced, the missing proteins or other virus proteins that can complement the function of the missing proteins can be expressed in the virus-producing cells. According to the present application, a vector lacking at least the F gene or having a mutation in the F gene can be suitably used.

The RNP of the present application can be produced by transcribing the genomic RNA (positive-stranded or negative-stranded) included in the vector of the present application in the presence of the NP, P, and L proteins. The formation of RNP can be carried out in, for example, BHK-21 or LLC-MK2 cells. The NP, P, and L proteins may be supplied by virus vectors or by various other methods. For example, the proteins may be supplied by introducing expression vectors containing each gene into cells as described above. Each gene may also be integrated into the chromosome of the host cell. The NP, P and L genes to be expressed to form RNP need not be completely identical to the NP, P, and L genes encoded in the genome of the vector. That is, the amino acid sequence of the proteins encoded by these genes may not be the same as the amino acid sequence of the proteins encoded by RNP genome, and can be mutated or substituted with homologous genes of other viruses as long as they bind to genomic RNA and have transcription and replication activities for the genome in the cells. The wild-type proteins (wild-type NP, P, and/or L proteins) may be expressed.

For example, the production of the minus-stranded RNA virus of the present application can be performed by using the following conventional methods (WO97/16539; WO97/16538; WO00/70055; WO00/70070; WO01/18223; WO03/025570; WO2005/071092; WO2006/137517; WO2007/083644; WO2008/007581; Hasan, M. K. et al., J. Gen. Virol. 78: 2813-2820, 1997, Kato, A. et al., 1997, EMBO J.16: 578-587, and Yu, D. et al., 1997, Genes Cells 2: 457-466; Durbin, A. P. et al., 1997, Virology 235: 323-332; Whelan, S. P. et al., 1995, Proc. Natl. Acad. Sci. USA 92: 8388-8392; Schnell. M. J. et al., 1994, EMBO J. 13: 4195-4203; Radecke, F. et al., 1995, EMBO J. 14: 5773-5784; Lawson, N. D. et al., Proc. Natl. Acad. Sci. USA 92: 4477-4481; Garcin, D. et al., 1995, EMBO J. 14: 6087-6094; Kato, A. et al., 1996, Genes Cells 1: 569-579; Baron, M. D. and Barrett, T., 1997, J. Virol. 71: 1265-1271; Bridgen, A. and Elliott, R. M., 1996, Proc. Natl. Acad.Sci. USA 93: 15400-15404; Tokusumi, T. et al. Virus Res. 2002: 86; 33-38, Li, H.-O. et al., J. Virol. 2000: 74; 6564-6569). For methods for proliferation of viruses and production of recombinant viruses, see also "Detailed Experimental Virology (Uirusu-gaku Jikken-gaku Kakuron)", 2nd revised edition (edited by Gakuyukai of National Institute of Health, Maruzen Co. Ld., 1982).

### Reprogramming factor

In the present application, a reprogramming factor refers to a gene or its product that is used alone or together with multiple factors to direct a differentiated state of a certain cell to a more undifferentiated state, and includes, for example, a gene or its product that is used to induce dedifferentiation of differentiated cells. The reprogramming factor in the present application may include a factor that is essential for nuclear reprogramming, and an auxiliary factor (cofactor) that increases the efficiency of nuclear reprogramming. In the present application, the desired gene used for nuclear reprogramming can be carried into a vector. For example, the vector can carry a gene used for producing pluripotent stem cells. Specifically, examples of the reprogramming factors for inducing pluripotent stem cells are genes that are expressed in ES cells or early embryo but are not expressed or are down-regulated in many differentiated somatic cells, such as ES cell-specific genes. Such genes are preferably genes encoding transcription factors or nucleoproteins. The method for identifying nuclear reprogramming factors is already known (WO 2005/80598), and genes identified by the method have been shown to be useful for reprogramming differentiated cells into pluripotent stem cells (WO 2007/69666).

Examples of such genes include DPPA5 (developmental pluripotency associated 5, ES cell specific gene 1 (ESG1); accession numbers NM_001025290, NM_025274, XM_236761), F-box protein 15 (Fbx15, NM_152676, NM_015798), Nanog (NM_024865, AB093574), ECAT1 (ES cell associated transcript 1; AB211062, AB211060), ERAS (EScell expressed Ras; NM_181532, NM_181548), DNMT3L (DNA (cytosine-5-)-methyltransferase 3-like; NM_013369, NM_019448), ECAT8 (AB211063, AB211061), GDF3 (growth differentiation factor 3; NM_020634, NM_008108), SOX15 (SRY (sex determining region Y)-box 15; NM_006942, NM_009235), DPPA4 (developmental pluripotency associated 4; NM_018189, NM_028610), DPPA2 (NM_138815, NM_028615), FTHL17 (ferritin, heavy polypeptide-like 17; NM_031894, NM_031261), SALL4 (sal-like 4; NM_020436, NM_175303), Oct314 (also called POU5F1; NM_002701, NM_203289, NM_013633, NM_001009178), Sox2 (NM_003106, NM_011443, XM_574919), Rex-1 (ZFP42 (zinc fingerprotein 42 homolog); NM_174900, NM_009556), Utf1 (undifferentiated embryonic cell transcription factor 1; NM_003577, NM_009482), TCL1A (T-cell leukemia/lymphoma 1A; NM_021966, NM_009337), DPPA3 (also called Stella, NM_199286, NM_139218, XM_216263), KLF4 (Kruppel-like factor 4; NM_004235, NM_010637), cateninβ1 (cadherin-associated protein beta 1; NM_001904, NM_007614; including the S33Y mutant), c-Myc (NM_002467, NM_010849; including the T58A mutant), STAT3 (signal transducer and activator of transcription 3; NM_139276, NM_213659), GRB2 (growth factor receptor-bound protein 2; NM_002086, NM_008163), and genes of other members of the families to which these genes belong. These genes have been shown to induce pluripotent stem cells by introducing them into cells (WO 2007/69666). These genes may be integrated individually into separate vectors, or multiple genes can be integrated together into a single vector. Each gene can be integrated into one type of vector, or different types of vectors (including a chromosomal integration virus vector and/or non-virus vector) can be used in combination with a non-chromosomal integration virus vector. The methods for producing reprogrammed cells in the present application are not limited to the methods of using virus vectors for all gene transfer. In other words, the methods of the present application requires the use of at least one non-chromosomal integration virus vector, and include the combined use of other vectors (chromosomal integration virus vectors and/or non-virus vectors) expressing reprogramming factors and/or compounds inducing reprogramming.

Reprogramming may be, for example, the induction of pluripotent stem cells from differentiated cells. Vectors in which genes encoding factors for reprogramming are integrated are used. Examples of reprogramming factors include any one of the genes exemplified above or below.

Examples of genes that are particularly preferable for inducing cellular reprogramming include F-box protein 15 (Fbx15, NM_152676, NM_015798), Nanog (NM_024865, AB093574), ERAS (ES cell expressed Ras; NM_181532, NM_181548), DPPA2 (NM_138815, NM_028615), Oct3/4 (also called POU5F1; NM_002701, NM_203289, NM_013633, NM_001009178), Sox2 (NM_003106, NM_011443, XM_574919), TCL1A (T-cell leukemia/lymphoma 1A; NM_021966, NM_009337), KLF4 (Kruppel-like factor 4; NM_004235, NM_010637), cateninβ1 (cadherin-associated protein beta 1; NM_001904, NM_007614; including the S33Y mutant), and c-Myc (NM_002467, NM_010849; including the T58A mutant), and the genes of other members of the families to which these genes belong. Individual virus vectors can be used in combination.

One of the particularly preferred combination of the genes is the combination comprising at least 4 genes of the Sox gene, the KLF gene, the Myc gene, and the Oct gene (Takahashi, K. and Yamanaka S., Cell 126, 663-676, 2006; Lowry WE et al., Proc Natl Acad Sci USA, 105(8):2883-8, 2008; Masaki, H. et al., Stem Cell Res. 1:105-115, 2008; WO2007/69666). The Sox protein, the KLF protein, the Myc protein, and the Oct protein, and genes thereof refer to the proteins and genes of the members that belong to the Sox family, the KLF family, the Myc family, and the Oct family, respectively. It may be adjusted to express one or more members of each of these four families. For example, for the Sox family genes, any of the Sox1, Sox2, Sox3, Sox15, and Sox17 genes may be used. For the KLF family, KLF4 or KLF2 may be used. For the Myc family, not only the wild-type c-Myc but the T58A mutant, N-Myc, and L-Myc can be used. As such, the genes of the families can be selected in various ways and then used. Reprogramming can be induced by the genes appropriately selected from the above four families.

For example, wild-type c-Myc is poorly expressed from RNA virus vectors such as Sendai virus vector. However, c-MYC gene having one or more, preferably two or more, three or more, four or more, or all five mutations selected from a378g, t1122c, t1125c, a1191g, and a1194g can be stably and highly expressed from the vectors. The position where the gene is inserted into the vectors can be selected as desired.

For example, the Myc gene may be positioned at the back (5' side) of the minus-stranded RNA genome, i.e., at the position closer to the 5' end than the 3' end in the multiple protein coding sequences located on the genome. The Myc gene can be positioned, for example, closest to the 5' end (i.e., at the first position from the 5' side), or at the second or third position from the 5' end. The Myc gene can be positioned, for example, at the second position from the 5' end of the genome. Specifically, the Myc gene may be positioned between the L gene and the HN gene when the L gene is positioned closest to the 5' end of the genome, followed by the HN gene. In the Myc gene, silent mutations can be introduced as appropriate so as not to alter the encoded amino acid sequence and to replace contiguous A or T sequences.

A minus-stranded RNA virus vector in which the Myc gene is positioned at the back (5' side) of the minus-stranded RNA genome can be used in combination with minus-stranded RNA virus vectors containing other reprogramming factors. In this case, in the minus-stranded RNA virus vectors containing other reprogramming factors, the reprogramming factors can be positioned at the forward (3' side) of the minus-stranded RNA genome in the vectors, i.e., at the position closer to 3' end than the 5' end in the multiple protein encoding sequences located on the genome. For example, they may be positioned closest to the 3' end (i.e., at the first position from the 3' end), or at the second or third position from the 3' end. For example, reprogramming factors other than Myc (e.g., the Oct gene, Klf gene, and Sox gene) are positioned at the first or second position, or more preferably at the first position from the 3' end of the genome in each minus-stranded RNA virus vector. Specifically, the reprogramming factors can be positioned closest to the 3' end of the NP gene of the genome.

Specifically, the KLF family includes Klfl (NM_006563, NM_010635), Klf2 (NM_016270, NM_008452), Klf4 (NM_004235, NM_010637), and Klf5 (NM_001730, NM_009769); the Myc family includes c-Myc (NM_002467, NM_010849, including the T58A mutant), NMyc (NM_005378, NM_008709), and L-Myc (NM_005376, NM_005806); the Oct family includes Oct1A (NM_002697, NM_198934), Oct3/4 (NM_002701, NM_203289, NM_013633, NM_001009178), and Oct6 (NM_002699, NM_011141); and the Sox family includes Sox1 (NM_005986, NM_009233), Sox2 (NM_003106, NM_011443, XM_574919), Sox3 (NM_005634, NM_009237), Sox7 (NM_031439, NM_011446), Sox15 (NM_006942, NM_009235), Sox17 (NM_022454, NM_011441), and Sox18 (NM_018419, NM_009236). The non-chromosomal integration virus vector carrying any one of these genes is useful for use in inducing the dedifferentiation of cells in the present application.

One or more virus vectors carrying any one of the Sox, KLF, and Oct genes, or any one of the Sox, Myc, and Oct genes, or a combination of the Sox, Myc and Klf genes, are useful for use in inducing the reprogramming of cells in the present application. For example, if the MYC gene is not expressed, p53 siRNA and UTF1 can be used instead. The virus vector containing each gene can be prepared individually. They can be used in combination.

When one or several of the above genes are, for example, already expressed endogenously in the original differentiated cells, the introduction of the genes can be omitted. Virus vectors containing reprogramming factors may be used only as needed. If the endogenous expression of endogenous reprogramming factors is induced by the expression of other genes or by the treatment with compounds, only virus vectors containing the reprogramming factors that cannot be induced by the induction or treatment may be introduced in combination with the introduction of a vector expressing such other genes or the treatment with the compounds. In the present application, combining vectors so as to endogenously or exogenously express at least the three types of genes of the Oct gene, the Klf gene, and the Sox gene, at least the four types of genes of the Oct gene, the Klf gene, the Sox gene, and the Myc gene, or at least the four types of genes of the Oct gene, the Sox gene, the Nanog gene, and the Lin28 includes, for example, that when a certain reprogramming factor is endogenously expressed in a natural state, or can be induced by the introduction of vectors expressing other genes or by the treatment with compounds or proteins, the virus vector can be combined with such treatment to exogenously expressed only the factors that cannot be endogenously expressed in a natural state or induced by such treatment.

In addition to the above combinations of the four or three types of the genes, the combinations including each of the four types of genes of the Oct gene, the Sox gene, the NANOG gene (NM_024565, AB093574) and the LIN28 gene (NM_024674) are also useful. The combinations further including the Myc gene and the KLF gene are also suitable. The virus vector carrying any one of these genes is useful for use in inducing the dedifferentiation of cells in the present application. One or more virus vectors containing any combination (or all) of these genes can also be suitably used for reprogramming of cells. As described above, when the cells of interest already express some of these genes, the vectors expressing the genes may or may not be introduced.

Other genes can be further combined with the above combination of the genes to increase the reprogramming efficiency. Examples of such genes include TERT (NM_198253, NM_009354) and/or SV40 large T antigen (NC_001669.1, Fiers, W. (5 Nov. 1978) Nature 273: (5658) 113-120). One or more genes selected from the group consisting of HPV16 E6, HPV16 E7, and Bmil (NM_005180, NM_007552) may be further combined. One or any combination of the genes selected from the group consisting of Fbx15 (Mol Cell Biol. 23(8): 2699-708, 2003), Nanog (Cell 113: 631-642, 2003), ERas (Nature 423, 541-545, 2003), DPPA2 (Development 130: 1673-1680, 2003), TCL1A (Development 130: 1673-1680, 2003), and β-Catenin (Nat Med 10 (1): 55-63, 2004) may be expressed. In addition, one or more genes selected from the group consisting of ECAT1 (AB211062, AB211060), DPPA5 (NM_001025290, NM_025274, XM_236761), DNMT3L (NM_013369, NM_019448), ECAT8 (AB211063, AB211061), GDF3 (NM_020634, NM_008108), SOX15 (NM_006942, NM_009235), DPPA4 (NM_018189, NM_028610), FTHL17 (NM_031894, NM_031261), SALL4 (NM_020436, NM_175303), Rex-1 (NM_174900, NM_009556), Utfl (NM_003577, NM_009482), DPPA3 (NM_199286, NM_139218, XM_216263), STAT3 (NM_139276, NM_213659), and GRB2 (NM_002086, NM_008163) may be combined. These factors can also be expressed by using the non-chromosomal integration virus vectors of the present application.

The factors to be introduced may be selected appropriately according to the origin of the cells to be reprogrammed, and may be derived from humans or other mammals such as mice, rats, rabbits, pigs, or primates such as monkeys, and preferably humans. The sequences of the genes and proteins may not be wild-type sequences, and may have any mutations as long as they can induce reprogramming. For example, the gene that encodes an amino acid sequence with one or a small number (e.g., a few, 3 or less, 5 or less, 10 or less, 15 or less, 20 or less, or 25 or less) of amino acid additions, deletions, substitutions, and/or insertions, and that can induce reprogramming may be used in the present application. For example, the polypeptides in which one to several amino acid residues (e.g., 2, 3, 4, 5, 6, 10, 15, or 20 residues) are deleted or added at the N terminus and/or the C terminus, and the polypeptides in which one to several amino acid residues (e.g., 2, 3, 4, 5, 6, 10, 15, or 20 residues) are substituted can be used as long as the biological activity (i.e., the ability to induce reprogramming) is maintained. Examples of the variants that may be used include fragments, analogs, and derivatives of natural proteins, and fusion proteins of natural proteins with other polypeptides (e.g., those with addition of heterologous signal peptides or antibody fragments). Specifically, the examples include the polypeptide comprising the sequence in which one or more amino acids are substituted, deleted and/or added in a wild-type amino acid sequence, and having the biological activity (e.g., the activity to induce reprogramming) equivalent to that of the wild-type protein. When a fragment of a wild-type protein is used, it typically contains a continuous region of 70% or more, preferably 80% or more, 85% or more, more preferably 90% or more, 95% or more, or 98% or more of the wild-type polypeptide (a mature form in the case of a secretory protein).

The variants of an amino acid sequence can be prepared, for example, by introducing mutations to DNA encoding the natural polypeptide (Walker and Gaastra, eds. Techniques in Molecular Biology (MacMillan Publishing Company, New York, 1983); Kunkel, Proc. Natl. Acad. Sci. USA 82:488-492, 1985; Kunkel et al., Methods Enzymol. 154:367-382, 1987; Sambrook et al., Molecular Cloning: A Laboratory Manual (Cold Spring Harbor Laboratory Press, Plainview, N.Y.), 1989; U.S. Pat. No. 4,873,192). An example of guidance for substituting amino acids without affecting the biological activity includes Dayhoff et al. (Dayhoff et al., in Atlas of Protein Sequence and Structure (Natl. Biomed. Res. Found., Washington, D.C.), 1978).

The number of amino acids to be modified is not particularly limited, e.g., 30% or less, preferably 25% or less, more preferably 20% or less, more preferably 15% or less, more preferably 10% or less, 5% or less, or 3% or less of total amino acids of the natural mature polypeptide, such as 15 amino acids or less, preferably 10 amino acids or less, more preferably 8 amino acids or less, more preferably 5 amino acids or less, or more preferably 3 amino acids or less. When an amino acid is substituted, it is expected that the activity of a protein is maintained by substituting it with an amino acid with a similar side chain property. Such a substitution is referred to as a conservative substitution in the present application. Examples of conservative substitutions include substitutions between amino acids within each of the groups, such as basic amino acids (e.g., lysine, arginine, and histidine), acidic amino acids (e.g., aspartic acid and glutamic acid), uncharged polar amino acids (e.g., glycine, asparagine, glutamine, serine, threonine, tyrosine, and cysteine), non-polar amino acids (e.g., alanine, valine, leucine, isoleucine, proline, phenylalanine, methionine, and tryptophan), β-branched amino acids (e.g., threonine, valine, isoleucine), and aromatic amino acids (e.g., tyrosine, phenylalanine, tryptophan, and histidine). The examples also include substitutions between amino acids that have a positive value relationship in the BLOSUM62 substitution matrix (S. Henikoff and J. G. Henikoff, Proc. Acad. Natl. Sci. USA 89: 10915-10919, 1992).

The modified protein exhibits high homology to the amino acid sequence of the wild-type protein. High homology refers to an amino acid sequence having, for example, 70% or higher, 75% or higher, 80% or higher, 85% or higher, 90% or higher, 93% or higher, 95% or higher, or 96% or higher identity. Amino acid sequence identity can be determined using, for example, the BLASTP program (Altschul, S. F. et al., J. Mol. Biol. 215: 403-410, 1990). For example, searches can be preformed using default parameters on the Web page of BLAST at NCBI (National Center for Biotechnology Information) (Altschul S. F. et al., Nature Genet. 3: 266-272, 1993; Madden, T. L. et al., Meth. Enzymol. 266: 131-141, 1996; Altschul S. F. et al., Nucleic Acids Res. 25: 3389-3402,1997; Zhang J. & Madden T. L., Genome Res. 7: 649-656, 1997). An alignment of two sequences can be produced to determine the identity of the sequences, for example, by the Blast 2 sequences program which compares two sequences (Tatiana A et al., FEMS Microbiol Lett. 174: 247-250, 1999). Gaps are treated in the same way as mismatches, and for example, the identity value to the entire amino acid sequence of a natural cytokine (the mature form after secretion) is calculated. Specifically, the proportion of the number of the matched amino acids in the total number of amino acids of the wild-type protein (the mature form in the case of a secreted protein) is calculated.

A silent mutation can be introduced into a gene so that the encoded amino acid sequence is not altered. Particularly, for an AT rich gene, high expression of the gene can be stably obtained by substituting five or more consecutive A or T bases with G or C without changing the encoded amino acid sequence.

Examples of the modified protein or the protein used for reprogramming include a protein encoded by a nucleic acid that hybridizes under stringent conditions with a part or all of the coding region of the gene encoding the wild-type protein, and having an activity (the activity to induce reprogramming) equivalent to that of the wild-type protein. For example, hybridization can be identified by preparing a probe either from a nucleic acid comprising a sequence of the coding region of the wild-type protein gene or a complementary sequence thereof or from a nucleic acid to be hybridized, and by detecting whether or not the probe hybridizes to the other nucleic acid. The stringent hybridization condition is, for example, the condition of performing hybridization in a solution containing 5xSSC, 7% (W/V) SDS, 100 µg/mL denatured salmon sperm DNA, 5×Denhardt's solution (1×Denhardt's solution includes 0.2% polyvinyl pyrrolidone, 0.2% bovine serum albumin, and 0.2% Ficoll) at 60°C, preferably 65°C, and more preferably 68°C, followed by washing with shaking for two hours in 2×SSC, preferably in 1×SSC, more preferably in 0.5xSSC, and more preferably in 0.1×SSC at the same temperature as hybridization.

### Method for producing naive induced pluripotent stem cells

The present application provides a method for producing naive induced pluripotent stem cells from human somatic cells, comprising the following steps (1) to (3):
(1) introducing one or more vectors containing a reprogramming factor into human somatic cells,
(2) culturing said somatic cells in the presence of a naive medium, and
(3) after step (2), culturing the resulting cells in the presence of the naive medium under the condition in which the amount of the vectors per the somatic cell is reduced to 30% or less of that at the start of step 3.

In the present disclosure, the somatic cells are not particularly limited and any somatic cells can be used. Examples of the somatic cells include keratinized epithelial cells (e.g., keratinized epidermal cells), mucosal epithelial cells (e.g., epithelial cells of a tongue surface), exocrine gland epithelial cells (e.g., mammary gland cells), hormone secreting cells (e.g., adrenal medullary cells), cells for metabolism and storage (e.g., hepatocytes), luminal epithelial cells composing a boundary surface (e.g., type I alveolar cells), luminal epithelial cells of a obturator canal (e.g., vascular endothelial cells), cells with having a transport capacity and with cilia (e.g., airway epithelial cells), cells for extracellular matrix secretion (e.g., fibroblasts), contractile cells (e.g., smooth muscle cells), cells of blood and immune system (e.g., peripheral blood mononuclear cells, cord blood, T lymphocytes), cells related to sensation (e.g., rod cells), autonomic neurons (e.g., cholinergic neurons), supporting cells of sensory organ and peripheral neurons (e.g., satellite cells), neurons and glial cells of a central nervous system (e.g., stellate glial cells), pigment cells (e.g., retinal pigment epithelial cells), and their progenitor cells (tissue progenitor cells). The degree of the differentiation of the cells and the age of the animal from which the cells are harvested are not particularly limited. Both undifferentiated progenitor cells (including somatic stem cells) and terminally differentiated mature cells can be used as the source of the somatic cells in the present application. Examples of the undifferentiated progenitor cells include tissue stem cells (somatic stem cells) such as neural stem cells, hematopoietic stem cells, mesenchymal stem cells, and dental pulp stem cells. Preferably, the somatic cells are of human origin.

In the present disclosure, an induced pluripotent stem cell is a cell in which pluripotency is induced by reprogramming a somatic cell using known methods or other methods. Specifically, the induced pluripotent stem cells include cells in which pluripotency is induced by reprogramming differentiated somatic cells such as fibroblasts or peripheral blood mononuclear cells by the expression of a combination of multiple genes selected from a reprogramming gene group including Oct3/4, Sox2, Klf4, Myc (c-Myc, N-Myc, L-Myc), Glis1, Nanog, Sall4, lin28, and Esrrb.

In the present disclosure, "naive induced pluripotent stem cells" refer to induced pluripotent stem cells having characteristics similar to those of preimplantation embryos, and can be defined as induced pluripotent stem cells having one or more of the following characteristics: gene expression patterns equivalent to those of the inner cell mass of preimplantation embryos (specifically, the expression of one or more naive-type specific markers, or naive-type specific subcellular localization of one or more specific gene products), extensive DNA demethylation across the entire genome, reactivation of the X chromosome, and a multilayered and dome-shaped colony morphology. Examples of the naive-type specific markers include TCFP2L1, KLF17, TBX3, PRDM14, SSEA1, CD7, CD75, CD77, CD130, DPPA3, ESRRB, KLF4, and KLF5. Examples of the naive-type-specific subcellular localization of specific gene products includes the nuclear localization of a TFE3 protein.

The produced naive induced pluripotent stem cells may be confirmed by having one or more of the above characteristics, preferably the expression of the above naive-type specific markers and/or the naive-type specific subcellular localization of the specific gene products. In addition, for rapidity and convenience, the produced naive induced pluripotent stem cells may be confirmed by having a dome-shaped colony morphology.

As a method for confirming gene expression patterns, known methods such as RNA-seq, qPCR, immunostaining, FCM, and preferably RNA-seq can be used.

"Primed induced pluripotent stem cells" refer to induced pluripotent stem cells having characteristics similar to those of epiblasts of post-implantation embryos, and can be defined as induced pluripotent stem cells having one or more of the following characteristics: gene expression patterns equivalent to those of epiblasts of post-implantation embryos (specifically, the expression of one or more primed-type specific markers, or primed-type specific subcellular localization of one or more specific gene products), and a single-layered and flattened colony morphology. Examples of the primed-type specific markers include OTX2 or ZIC2. Examples of the primed-type specific subcellular localization of specific gene products include cytoplasmic localization of a TFE3 protein.

The produced primed induced pluripotent stem cells may be confirmed by having one or more of the above characteristics, preferably the expression of the above primed type-specific markers and/or the primed type-specific subcellular localization of the specific gene products. In addition, for rapidity and convenience, the produced primed induced pluripotent stem cells may be confirmed by having a single-layerd and flattened colony morphology.

In step (1), one or more vectors containing a reprogramming factor are introduced into human somatic cells.

The above reprogramming factors can be used as the reprogramming factors. Examples of the reprogramming factors may include an OCT gene, a SOX gene, a MYC gene, and a KLF gene.

The above vectors can be used as the one or more vectors. For example, the one or more vectors may be non-chromosomal integration virus vectors, preferably paramyxovirus vectors, and more preferably Sendai virus vectors.

The one or more vectors containing the reprogramming factor may include, for example, a vector containing an OCT gene, a SOX gene, and a KLF gene, a vector containing a MYC gene, and a vector containing a KLF gene.

The above combination of vectors is introduced into the cells to reprogram the cells. When multiple vectors are introduced in combination, the introductions are preferably performed simultaneously. Specifically, all vectors containing reprogramming factors are preferably added within 48 hours, preferably within 36 hours, more preferably within 24 hours, 18 hours, 12 hours, 10 hours, 8 hours, 6 hours, 3 hours, 2 hours, or 1 hour after the initial addition of the vector or compound. The doses of the vectors can be prepared as appropriate, and the vectors are infected preferably at an MOI of 0.3 to 100, more preferably at an MOI of 0.5 to 50, more preferably at an MOI of 1 to 30, more preferably at an MOI of 1 to 10, and more preferably at an MOI of about 5.

In one aspect, step (2) is started 1 to 15 days, 1 to 10 days, for example, 3 to 9 days after step (1). The human somatic cells can be cultured under appropriate culture conditions until step (2) is started.

In the present application, the medium may be prepared by appropriately adding necessary factors to a basal medium for animal cell culture. Examples of basal media include IMDM medium, Medium 199 medium, Eagle's Minimum Essential Medium (EMEM) medium, αMEM medium, MEM Zinc Option medium, IMEM Zinc Option medium, Dulbecco's modified Eagle's Medium (DMEM) medium, DMEM/F12 medium, Ham's F12 medium, RPMI 1640 medium, Fischer's medium, and a mixed medium thereof. The basal medium may contain serum (e.g., fetal bovine serum (FBS)) or may be a serum-free medium. If necessary, the basal medium may comprise one or more serum substitutes, such as albumin, insulin, transferrin, selenium, KnockOut Serum Replacement (KSR) (Invitrogen) which is a serum substitute used for culturing an ES cell, N2 supplement (Invitrogen), B27 supplement (Invitrogen), a fatty acid, collagen precursor, a trace element, 2-mercaptethanol, and 3'-Thiolglycerol. In addition, the basal medium may comprise one or more substances, such as lipid, an amino acid, L-glutamine, GlutaMAX (Invitrogen), a nonessential amino acid, a vitamin, a growth factor, a low-molecular compound, an antibiotic (such as streptomycin, penicillin, puromycin, mitomycin), an anti-oxidant, pyruvic acid, a buffering agent, an inorganic salt, a cytokine, and equivalents thereof. In one embodiment, the basal medium is DMEM medium or DMEM/F12 medium. Commercially available media may be used as the basal medium. For example, when human hematopoietic cells are cultured, StemSpan ACF (STEMCELL Technologies) can be used. When human hematopoietic cells are cultured, additional factors such as stem cell factor (SCF), thrombopoietin (TPO), Flt3/Flk2, IL-6, and IL-3 may be added to the basal medium.

In one aspect, the somatic cells can be cultured with feeder cells. In the present specification, "feeder cells" refer to cells other than somatic cells that coexist with the somatic cells in the culture of the somatic cells. Examples of the feeder cells include, but are not particularly limited to, fibroblasts such as NIH-3T3 cells, MEF cells, STO cells, and SNL cells; embryonic kidney cells such as HEK293 cells; stromal cells such as ST2 cells, OP9 cells, PA6 cells, and MS-5 cells; epithelial cells such as HeLa cells; placental cells; bone marrow cells; and endometrial cells. The feeder cells are, for example, MEF cells. The feeder cells may be derived from the same species as the somatic cells or from a different species than the somatic cells. The feeder cells may also be treated with growth inhibitors (e.g., mitomycin C) or irradiation (e.g., X-rays or γ-rays) to inhibit cell proliferation. The concentration of the feeder cells used in the present application is not particularly limited as long as the somatic cells can proliferate, and for example, about 5×10³ cells/cm² to about 1×10⁵ cells/cm². The somatic cells may be cultured with feeder cells from the start of the culture or in the middle of the culture. For example, the somatic cells can be cultured with feeder cells from day 2-10 or day 2-5 of the culture. In another aspect, the somatic cells can be cultured in the absence of feeder cells.

For the culture of the somatic cells, the culture temperature can be determined, but not limited, according to the characteristics of the vector within the range of about 30 to 40°C. The culture is performed in a CO₂-containing air atmosphere. The COz concentration is about 2 to 8%, preferably about 3 to 7%, more preferably about 4 to 6%, and most preferably about 5%. After step (1), the culture may be performed under hypoxic conditions. The oxygen concentration is about 3 to 10%, preferably about 4 to 8%, more preferably about 4 to 6%, and most preferably about 5%.

In one aspect, the somatic cells are cultured under a two-dimensional culture condition. For example, when the culture is performed in the presence of feeder cells, the feeder cells may be seeded and cultured on the bottom of a culture vessel or a culture substrate in advance and proliferate to fill the bottom of the culture vessel or the surface of the culture substrate, and then the somatic cells may be seeded and cultured on these feeder cells.

The culture vessels for culturing somatic cells are, for example, those treated with a coating agent. Examples of the coating agents include gelatin, Matrigel (BD), Synthemax (Corning), collagen, laminin, heparan sulfate proteoglycan, entactin, fragments thereof, and combinations thereof.

In step (2), said somatic cells are cultured in the presence of a naive medium.

In step (2), the culture temperature can be determined, but not limited, according to the characteristics of the vector within the range of about 30 to 40°C. The culture is performed in a CO₂-containing air atmosphere. The CO₂ concentration is about 2 to 8%, preferably about 3 to 7%, more preferably about 4 to 6%, and most preferably about 5%. The culture may be performed under hypoxic conditions. The oxygen concentration is about 3 to 10%, preferably about 4 to 8%, more preferably about 4 to 6%, and most preferably about 5%.

In the present application, "naive medium" refers to a medium used to induce naive induced pluripotent stem cells from somatic cells. The basal media described above can be used as the basal medium for the naive medium. Examples of the basal medium for the naive medium include N2B27 medium (a medium obtained by mixing N2 medium, which is DMEM/F12 medium supplemented with N2 supplement, and B27 medium, which is Neurobasal medium supplemented with B27 supplement, at 1:1) supplemented with any combination of additives such as nonessential amino acids (NEAA), L-glutamine, 2-mercaptoethanol, antibiotics (e.g., streptomycin, penicillin, puromycin, mitomycin), and bovine serum albumin (BSA).

In one aspect, the naive medium contains one or more compounds selected from a leukemia inhibitory factor (LIF), a MEK inhibitor, a GSK3 inhibitor, a cAMP production stimulator, a TGF-β inhibitor, and a PKC inhibitor. In particular, the naive medium preferably contains a LIF, and more preferably a LIF and a GSK3 inhibitor. The naive medium is preferably substantially free of bFGF.

The concentration of the LIF in the medium may be 1 ng/ml to 100 ng/ml, or 5 ng/ml to 50 ng/ml, and for example about 10 ng/ml.

In the present application, examples of the MEK inhibitors include, but are not limited to, PD0325901 (N-[(2R)-2,3-dihydroxypropoxy]-3,4-difluoro-2-[(2-fluoro-4-iodophenyl)amino]-benzamide; CAS registry number: 391210-10-9), U0126 (1,4-diamino-2,3-dicyano-l,4-bis[2-aminophenylthio]butadiene; CAS registry number: 109511-58-2), PD98059 (2-(2-amino-3-methoxyphenyl)-4H-1-benzopyran-4-one; CAS registry number: 167869-21-8), PD184352 (2-(2-chloro-4-iodophenylamino)-N-cyclopropylmethoxy-3,4-difluorobenzamide; CAS registry number: 212631-79-3). Among them, PD0325901 is preferred. When PD0325901 is used as the MEK inhibitor, its concentration in the medium may be 50 nM to 100 µM, or 100 nM to 10 µM, and for example about 1 µM.

In the present application, examples of the GSK3 inhibitors include, but are not limited to, CHIR99021 (6-[[2-[[4-(2,4-dichlorophenyl)-5-(5-methyl-1H-imidazol-2-yl)-2-pyrimidinyl]amino]ethyl]amino]-3-pyridine carbonitrile; CAS registry number: 252917-06-9), BIO (6-bromoindirubin-3'-oxime; CAS registry number: 667463-62-9), Kenpaullone (9-bromo-7,12-dihydroindolo [3,2-d][1]benzazepin-6(5H)-one; CAS registry number: 142273-20-9), IM-16 (3-(4-fluorophenylethylamino)-1-methyl-4-(2-methyl-1H-indol-3-yl)-1H-pyrrol-2,5-dione; CAS registry number 1129669-05-1). Among them, CHIR99021 is preferred. When CHIR99021 is used as the GSK3 inhibitor, its concentration in the medium may be 50 nM to 100 µM, or 100 nM to 10 µM, and for example about 1 µM.

In the present application, examples of the cAMP production stimulators include, but are not limited to, forskolin. When forskolin is used as the cAMP production stimulator, its concentration in the medium may be 50 nM to 100 µM, or 100 nM to 10 µM, and for example about 1 µM.

In the present application, examples of the TGF-β inhibitors include, but are not limited to, A83-01 (3-(6-methyl-2-pyridinyl)-N-phenyl-4-(4-quinolinyl)-1H-pyrazole-1-carbothioamide), SB431542 (4-[4-(1,3-benzodioxol-5-yl)-5-(2-pyridinyl)-1H-imidazol-2 -yl]-benzamide). Among them, A83-01 is preferred. When A83-01 is used as the TGF-β inhibitor, its concentration in the medium may be 10 nM to 100 µM, or 100 nM to 10 µM, and for example about 1 µM.

In the present application, examples of the PKC inhibitors include, but are not limited to, Go6983 (3-[1-[3-(dimethylamino)propyl]-5-methoxy-1H-indol-3-yl]-4-(1H-indole-3-yl)-1H-pyrrol-2,5-dione; CAS registry number: 133053-19-7), GF109203X (3-(1 -(3 -dimethylamino)propyl)-1H-indol-3-yl)-4-(1H-indole-3-yl) -1H-pyrrol-2,5-dione; CAS registry number: 133052-90-1). Among them, Go6983 is preferred. When Go6983 is used as the PKC inhibitor, its concentration in the medium may be 50 nM to 100 µM, or 100 nM to 10 µM, and for example about 1 µM to 3 µM.

Commercially available media may be used as the naive medium. For example, t2iLGo (N2B27 + PD0325901 (1 µM) + CHIR99021 (1 µM) + LIF + Go6983 (2-3 µM)), 5iLAF (N2B27 + PD0325901 (1 µM) + CHIR99021 (1 µM) + SB590885 (0.5 µM) + WH-4-023 (1 µM) + Y-27632 (10 µM) + LIF + Activin A), or tt2iLGo (N2B27 + PD0325901 (1 µM) + LIF + Go6983 (2 µM) + XAV939 (2 µM)) can be used.

When the cells are cultured in the absence of feeder cells, the naive medium can be pre-conditioned with specific cells. The above-described cells that can be used as the feeder cells can also be used as the specific cells, and the specific cells may be, for example, irradiated mouse embryonic fibroblasts (iMEFs). The culture period for conditioning the medium may be, for example, about 12 hours to about 2 days. The medium may be obtained by removing cellular components by conventional methods after the culture for conditioning the medium. In one embodiment, when the cells are cultured in the absence of feeder cells, the naive medium may be t2iLGo conditioned with iMEFs.

In step (2), the culture period for the somatic cells may be 1 to 20 days, 3 to 10 days, and for example 4 to 6 days.

In step (3), the resulting cells are cultured in the presence of the naive medium under the condition in which the amount of the vectors per the somatic cell is reduced to 30% or less of that at the start of step 3.

In one aspect, the resulting cells can be cultured with feeder cells. As the feeder cells, the cells described above can be used. In another aspect, the resulting cells can be cultured in the absence of feeder cells.

In step (3), the naive medium can be the same as the naive medium used in step (2). The naive medium may contain one or more compounds selected from a leukemia inhibitory factor (LIF), a MEK inhibitor, a GSK3 inhibitor, a cAMP production stimulator, a TGF-β inhibitor, and a PKC inhibitor. For example, it may include t2iLGo, 5iLAF, or tt2iLGo.

When the cells are cultured in the absence of feeder cells, the naive medium can be pre-conditioned with specific cells. The above-described cells that can be used as the feeder cells can also be used as the specific cells, and the specific cells may be, for example, irradiated mouse embryonic fibroblasts (iMEFs). The culture period for conditioning the medium may be, for example, about 12 hours to about 2 days. The medium may be obtained by removing cellular components by conventional methods after the culture for conditioning the medium. In one embodiment, when the cells are cultured in the absence of feeder cells, the naive medium may be t2iLGo conditioned with iMEFs.

In step (3), the culture temperature can be determined, but not limited, according to the characteristics of the vector within the range of about 30 to 40°C. The culture is performed in a CO₂-containing air atmosphere. The CO₂ concentration is about 2 to 8%, preferably about 3 to 7%, more preferably about 4 to 6%, and most preferably about 5%. The culture may also be performed under hypoxic conditions. The oxygen concentration is about 3 to 10%, preferably about 4 to 8%, more preferably about 4 to 6%, and most preferably about 5%.

In one aspect, the amount of the vectors per the somatic cell may be reduced to 50% or less, 40% or less, 30% or less, 20% or less, 10% or less, 5% or less, or 3% or less of that at the start of step 3 within 30, 20, 15, 12, 10, 8, 5, 3, or 1 days from the start of step 3. For example, the amount of the vectors per the somatic cell may be reduced to 30% or less of that at the start of step 3 within 12 days from the start of step 3.

In one aspect, the one or more vectors are temperature-sensitive vectors, vectors containing a target sequence of a microRNA specific for induced pluripotent stem cells, or temperature-sensitive vectors containing a target sequence of a microRNA specific for induced pluripotent stem cells, and the resulting cells are cultured in the presence of the naive medium, and thereby the amount of the vectors per the somatic cell may be reduced to 50% or less, 40% or less, 30% or less, 20% or less, 10% or less, 5% or less, or 3% or less of that at the start of step 3, e.g., within 30, 20, 15, 12, 10, 8, 5, 3, or 1 days from the start of step 3.

As the temperature-sensitive vectors, the vectors described above can be used. For example, the temperature-sensitive vector may be the vector containing the temperature-sensitive mutation described above. In one aspect, the temperature-sensitive vector is a vector that is temperature-sensitive in the cell culture environment for establishing naive pluripotent stem cells. Examples of the vectors that are temperature-sensitive in the cell culture environment for establishing naive pluripotent stem cells include, but are not limited to, Sendai virus vectors with TS7 mutation which has TS mutation (G69E/T116A/A183S mutations in the M protein, A262T/G264R/K461G mutations in the HN protein, L511F mutation in the P protein, and N1197S/K1795E mutations in the L protein) and Y942H/L1361C/L1558I mutations in the L protein, TS12 mutation which has TS mutation and D433A/R434A/K437A mutations in the P protein, or TS15 mutation which has TS mutation, D433A/R434A/K437A mutations in the P protein and L1361C/L1558I mutations in the L protein, and other minus-stranded RNA virus vectors with equivalent sites similarly substituted.

One or more vectors containing a reprogramming factor may include, for example, a vector containing the OCT gene, the SOX gene, and the KLF gene, a vector containing the MYC gene, and a vector containing the KLF gene. The vector containing the OCT gene, the SOX gene, and the KLF gene, the vector containing the MYC gene, and the vector containing the KLF gene can be Sendai virus vectors, e.g., Sendai virus vectors with TS7, TS12, or TS15 mutation. In one embodiment, the vector containing the MYC gene can be a Sendai virus vector with TS15 mutation, and the vector containing the OCT gene, the SOX gene, and the KLF gene and the vector containing the KLF gene can be Sendai virus vectors with TS12 mutation.

In one aspect, when the one or more vectors are temperature-sensitive vectors, the culture may be performed at the temperature that results in high expression of the vectors in steps (1) and (2) and at the temperature that results in low expression of the vectors in step (3). For example, for the vectors described above, the culture may be performed at 30 to 36.5°C, 34 to 36°C, and for example, about 35°C in steps (1) and (2), and at 37°C or higher, 38°C or higher, 37°C to 45°C, 37°C to 40°C, and for example, about 38°C in step (3). In one example, the culture is performed at about 35°C in steps (1) and (2) and at about 38°C in step (3).

The target sequence of the microRNA specific for induced pluripotent stem cells is not particularly limited as long as the microRNA specific for induced pluripotent stem cells binds to the sequence as a target. Examples of the microRNAs specific for induced pluripotent stem cells include miR-367, miR-302, miR-371, miR-372, miR-373, miR-512, miR-517, miR-518, miR-519, miR-520, miR-525, miR-187, miR-299, miR-499, miR-628, and miR-888.

A microRNA target sequence refers to a sequence to which the microRNA binds as a target. According to the present application, a natural microRNA target sequence or a mutant thereof can be used as a microRNA target sequence, as long as the expression of the target sequence is suppressed by binding it to a microRNA. The microRNA target sequence may or may not be a sequence completely complementary to the microRNA, and contains, for example, at least 10, e.g., 11 or more, 12 or more, 13 or more, 14 or more, 15 or more, 16 or more, 17 or more, 18 or more, 19 or more, 20 or more, 21 or more, or 22 or more contiguous or non-contiguous bases complementary to the microRNA. There is no particular upper limit to the length of the microRNA target sequence. For example, the length of the microRNA target sequence is about 30 bases. Preferably, the complementary bases may be consecutive or have several, e.g., five or less, four or less, three or less, two or less, or one unpaired base. The unpaired bases may be included in the microRNA target sequence and/or in the microRNA.

The microRNA target sequence is preferably a sequence that hybridizes with the microRNA under physiological conditions. For example, the physiological condition is at 150 mM NaCl, 15 mM sodium citrate, pH 7.0, and 37°C. More preferably, the microRNA target sequence is a sequence that hybridizes with the microRNA under stringent conditions. For example, the stringent condition is 1×SSC (1×SSC is 150 mM NaCl, 15 mM sodium citrate, pH 7.0) or 0.5×SSC at 42°C, more preferably 1×SSC or 0.5×SSC at 45°C, and more preferably 1×SSC or 0.5×SSC at 50°C. For example, in hybridization, either RNA including a microRNA sequence or RNA including a microRNA target sequence is labeled, and if necessary, the other RNA is immobilized to a membrane, and then the two RNAs are hybridized. For example, the hybridization condition may be in a solution containing 5×SSC, 7% (WIV) SDS, 100 µg/ml denatured salmon sperm DNA, and 5×Denhardt's solution (1×Denhardt's solution contains 0.2% polyvinylpyrrolidone, 0.2% bovine serum albumin, and 0.2% ficoll), for example, at 37°C, or 45°C, or 50°C. It can be determined whether or not the nucleic acids hybridize under the conditions by performing washing under the above conditions after incubation for a sufficient time (e.g., 3, 4, 5 or 6 hours or more), and detecting whether the labeled nucleic acids hybridize.

Alternatively, the microRNA target sequence preferably has high homology with the complementary sequence of the microRNA sequence. High homology means that base sequences having, for example, 70% or more, 75% or more, 76% or more, 77% or more, 78% or more, 79% or more, 80% or more, 81% or more, 82% or more, 83% or more, 84% or more, 85% or more, 86% or more, 87% or more, 88% or more, 89% or more, 90% or more, 93% or more, 95% or more, 96% or more, 97% or more, 98% or more, or 99% or more identity. The identity of base sequences can be determined by using, for example, the BLAST program (Altschul, S. F. et al., J. Mol. Biol. 215: 403-410, 1990). For example, searches can be performed using default parameters on the Web page of BLAST at NCBI (National Center for Biotechnology Information) (Altschul S. F. et al., Nature Genet. 3: 266-272, 1993; Madden, T. L. et al., Meth. Enzymol. 266: 131-141, 1996; Altschul S. F. et al., Nucleic Acids Res. 25: 3389-3402, 1997; Zhang J. & Madden T. L., Genome Res. 7: 649-656, 1997). An alignment of two sequences can be produced to determine the identity of the sequences, for example, by the Blast 2 sequences program which compares two sequences (Tatiana A et al., FEMS Microbiol Lett. 174: 247-250, 1999). The gaps outside of the complementary base sequence of the microRNA sequence are ignored and the inner gaps are treated in the same way as mismatches to calculate the identity value to the entire base sequence (the total length of the bases obtained by adding the inner gaps of the sequence) of the complementary sequence of the microRNA sequence in the alignment.

Alternatively, the microRNA target sequence preferably consists of the sequence obtained by inserting, substituting, and/or deleting one or several bases in the complementary sequence of the microRNA sequence. Preferably, the microRNA target sequence consists of the sequence obtained by inserting, substituting, and/or deleting eight or less bases, seven or less bases, six or less bases, five or less bases, four or less bases, three or less bases, two or less bases, or one base in the complementary sequence of the microRNA sequence.

Generally, the more mutations are introduced into the microRNA target sequence, the more the binding to the microRNA is suppressed and the more the expression suppression effect is reduced. It is possible to adjust the suppression effect by introducing mutations as needed.

In one aspect, when the vector of the present application is a minus-stranded RNA virus vector, the microRNA target sequence is added to the NP or P gene. When the microRNA target sequence is added to the NP or P gene, the position of the addition is not particularly limited, and the microRNA target sequence can be added to the translated region or untranslated region of the gene. When the microRNA target sequence is added to the translated region, it may be added at either position of the N-terminal site or the C-terminal site, and for example, it is preferably added following the C-terminus. The untranslated region may be the 5'-untranslated region or the 3'-untranslated region in the genome, and is preferably the 5'-untranslated region. When the microRNA target sequence is added to the untranslated region, the position may be selected arbitrarily. For example, when the microRNA target sequence is added to the 5'-untranslated region of the NP gene and/or P gene in the genome, the position may be any position between the stop codon and the E (End) sequence in the translated region of the gene to which it is added. When the microRNA target sequence is added to the 3'-untranslated region in the genome, for example, it can be added to a desired position between the S (Start) sequence and the translation initiation codon in the gene. One or more copies of the microRNA target sequence can be added. One type of microRNA target sequence may be added, and multiple types of microRNA target sequences may also be added. For example, the sequence obtained by arranging two or more, e.g., three or more, four or more, or five or more of one type or multiple types of microRNA target sequences in tandem, can be added. There is no particular upper limit to the number of microRNA target sequences to be added in tandem, and for example it is up to about 10.

When the P gene is modified and the expression of the C protein encoded by the nucleic acid in the coding region of the P protein is inhibited, the C protein may be separately expressed from the vector.

The full-length or fragments of the P protein can be used as appropriate. Only part of the C-terminus is essential in the P protein, and the other regions are not essential for the expression of the virus vector. Specifically, the P protein may be a fragment having a binding site for the L protein and a binding site for the N protein:RNA. Examples of the binding site for the L protein include the sequence of 411th to 445th amino acids of the SeV P protein, and examples of the binding site for the N protein:RNA include the sequences of 479th to 568th amino acids of the SeV P protein (e.g., Accession Nos. AAB06197.1, P04859.1, P14252.1, AAB06291.1, AAX07439.1, BAM62828.1, BAM62834.1, P04860.1, BAM62840.1, BAD74220.1, P14251.1, BAM62844.1, BAM62842.1, BAM62842.1, BAF73480.1, BAD74226.1, BAF73486.1, Q9DUE2.1, BAC79134.1, NP_056873.1, and ABB00297.1). More specifically, for example, a fragment including the sequence of 320th to 568th amino acids of the SeV P protein can be suitably used as a functional P protein in the present application. It can be expected that the deletion form of the P protein is used to reduce the size of the vector and reduce the effect of the host immune response.

When the P protein lacking the coding region of the C protein is used, the C protein may be separately expressed as appropriate, as described above. The C protein includes C', C, Y1, and Y2 proteins (Irie T. et al., PLoS One. (2010) 5: e10719.). To express the C protein, the coding sequence of the C protein may be inserted into the vector as appropriate. The position of the insertion is not particularly limited, but the coding sequence can be inserted at a position immediately before the P protein (3'-side of the coding sequence of the P protein in the genome) or immediately after the P protein (5'-side of the coding sequence of the P protein in the genome). The E-I-S sequence may be added as appropriate for the insertion.

In the case in which the microRNA target sequence is added to the NP gene or P gene, when the microRNA is expressed in a cell into which the vector is introduced, the expression level of the vector is decreased according to the expression of the microRNA, and the removal of the vector is promoted. For example, the expression of the vector is automatically suppressed and the vector is removed when its purpose is achieved by adding to the vector the target sequence of the microRNA that is not expressed at the time of the introduction of the vector but is specifically expressed when the cell is in a certain differentiation state (or undifferentiated state).

In another aspect, one or more vectors may be the vectors in which a degron is added to the P gene, and the resulting cells may be cultured in the presence of the naive medium. In this case, the amount of said vectors per somatic cell may be reduced to 50% or less, 40% or less, 30% or less, 20% or less, 10% or less, 5% or less, or 3% or less of the amount at the start of step 3, e.g., within 30 days, 20 days, 15 days, 12 days, 10 days, 8 days, 5 days, 3 days, or 1 day from the start of step 3.

In the present application, a degron refers to a polypeptide that destabilizes a protein by adding it to the protein. Examples of degrons include a sequence that is stabilized by fusion with a small molecule, a sequence that is destabilized by binding to a small molecule, and a sequence that is destabilized regardless of the presence or absence of a small molecule. Specific examples include DD-tag (US 2009/0215169) derived from FKBP12, which is known as mTOR protein, DDG-tag (US 2012/0178168) derived from dihydrofolate reductase (DHFR), a TetR mutant (WO 2007/032555), a plant-derived auxin-inducible degron (AID) system (WO 2010/125620), a PEST sequence which is known as a decomposition promoting sequence (WO 99/54348), CL1 (WO 2004/025264), a calpain-derived sequence (JP 2009-136154 A), and NDS (JP 2011-101639 A). FKBP12, which is known as a mammalian target of rapamycin (mTOR), is stabilized by binding to a small molecule such as rapamycin and shield1, and destabilized by removing the small molecule and degraded by a proteasome. DHFR is stabilized by trimethoprim, and a TetR mutant is stabilized by doxycycline. The PEST sequence is rich in Pro, Glu, Ser, and Thr, and can be destabilized by adding, for example, the 422-461 sequence on the C-terminal side of mouse ornithine decarboxylase (mODC). The PEST sequence regulates the half-life of a protein, and a desired half-life shortening sequence can also be used (Rechsteiner M, et al., Trends Biochem. Sci. 21, 267-271, 1996). The PEST sequence, for example, is a sequence that is surrounded on both ends by basic amino acids (H, K, or R), contains (i) P, (ii) D and E, or (iii) S and E, and binds to ubiquitinating enzyme E3. The PEST sequence can be identified by, for example, GENETYX (Genetyx). Examples of sequences that have similar effects to PEST include CL1, a calpain partial sequence, and NDS. The AID sequence is destabilized by binding of TIR1, which is a plant ubiquitin ligase, to auxin (IAA).

In the present application, the addition of a degron to the P gene means that the P gene encodes the P protein to which the degron is added. In the present application, the above degrons can be added to the P protein, and specific preferred examples of degrons include mTOR degron, DHFR degron, TetR degron, PEST, and AID. The degrons include natural sequences and sequences derived from them. Particularly preferred examples of degrons include mTOR degron, DHFR degron, TetR degron, and PEST, and the degrons other than the AID sequence are suitable. Specifically, FKBP12 degron (DD), DHFR degron (DDG), TetR degron, and mODC PEST are preferred. Regarding the PEST, d2 derived from the natural sequence, and its modified forms, d1 and d4 are known (WO 99/54348), all of which are included in the PEST and can be used in the present application. The nucleic acid encoding a degron can be produced by DNA synthesis as appropriate.

The degron can be added to a desired position of the P protein as appropriate. For example, the degron can be added to the N-terminus or the C-terminus of the P protein. When the degron is added to the N-terminus of the P protein and the expression of the C protein encoded by the nucleic acid in the coding region of the P protein is inhibited, the C protein may be expressed separately from the vector. When a fragment of the P protein is used as the P protein instead of the full-length P protein, and the fragment does not contain the coding region of the C protein, the degron can be added at any position, e.g., the N-terminus or the C-terminus. In the present application, the degron is preferably added to the C-terminal side of the P protein. The modified P protein to which the degron is added can be produced by a well-known method. Specifically, a sequence encoding the degron may be inserted into the sequence of a virus genome encoding the P protein so that the reading frame is consistent.

After the vector in which the degron is added to the P protein in the present application is introduced into cells and the gene of interest is expressed, the vector can be removed as appropriate according to the characteristics of the degron. For example, when a ligand-controllable degron is used, such as DD, DDG and TetR mutant, the removal can be promoted by not adding a ligand, and the expression of the vector can be prolonged by adding a ligand such as Shield-1. When the degron that functions without a ligand is used, such as the PEST sequence, the removal of the vector can be promoted by continuing to culture the cells into which the vector is introduced.

The vector in which the degron is added to the P gene in this application may be the temperature-sensitive vector as described above, a vector containing a target sequence of a microRNA specific for induced pluripotent stem cells, or a temperature-sensitive vector containing a target sequence of a microRNA specific for induced pluripotent stem cells.

In still other aspect, the one or more vectors may be vectors carrying a suicide gene, and the resulting cells may be cultured in the presence of a naive medium. In this case, the amount of said vectors per somatic cell may be reduced to 50% or less, 40% or less, 30% or less, 20% or less, 10% or less, 5% or less, or 3% or less of the amount at the start of step 3, e.g., within 30 days, 20 days, 15 days, 12 days, 10 days, 8 days, 5 days, 3 days, or 1 day from the start of step 3.

Examples of the suicide gene carried into the vector of the present application include a prodrug-converting enzyme gene for a desired drug that induces cell death or exhibits cytotoxicity. Specific examples of the combination of prodrugs and suicide genes include as follows:
- 5-fluorocytosine and cytosine deaminase
- cyclophosphamide and cytochrome P450
- fludarabine and Escherichia coli PNP (purine-nucleoside phosphorylase)
- CB1954 and nitroreductase
- capecitabine and carboxylesterase.
The suicide gene also includes a gene encoding the desired protein that induces cell death or exhibits cytotoxicity when converted from the inactive form to the active form. For example, a gene encoding a caspase that induces cell death can be suitably used. Specifically, a combination of a desired dimerizing agent and the caspase to which a binding domain of the dimerizing agent is added can be used. Examples of the combination of the dimerizing agent and the caspase may include as follows:
- AP20187 and iCaspase-9.

For example, after the vector carrying a suicide gene in this application is introduced into cells and the cells are cultured, the prodrug is added. The addition of the prodrug results in the death of the cells in which the vector remains, and only the cells in which the vector is lost can be obtained.

The vector carrying a suicide gene in this application may be the temperature-sensitive vector as described above, a vector containing a target sequence of a microRNA specific for induced pluripotent stem cells, or a temperature-sensitive vector containing a target sequence of a microRNA specific for induced pluripotent stem cells. The vector carrying a suicide gene in this application may also be a vector in which a degron is added to the P gene as described above.

In step (3), the culture period is not particularly limited, and for example, can be 4 weeks or less, 3 weeks or less, 2 weeks or less, or 1 week or less, e.g., 20 days or less, 15 days or less, 10 days or less, 5 days or less, or 3 days or less. There is no particular lower limit to the culture period, and for example, it is about 1 day. The decrease in the amount of the vectors can be confirmed by detecting the reporter gene or detecting the virus using an antibody or PCR, and comparing its level with that at the start of step (3).

In the naive induced pluripotent stem cells produced by the method of the present application, the residual amount of the vectors containing the reprogramming factor is very low. Therefore, even if the naive induced pluripotent stem cells are cultured for a long period of time, the properties of the naive induced pluripotent stem cells can be stably maintained. In the method of the present application, the vectors containing the reprogramming factor can be substantially removed in the cells about 30 days after the introduction of the vectors. Thus, the naive induced pluripotent stem cells obtained by the method of the present application do not substantially contain the vectors containing the reprogramming factor and have high phenotypic stability.

### Set of vectors

The present application provides a set of vectors for producing naive induced pluripotent stem cells from human somatic cells, comprising:
one or more vectors containing a reprogramming factor,
wherein the one or more vectors are selected from the group consisting of a temperature-sensitive vector, a vector containing a target sequence of a microRNA specific for induced pluripotent stem cells, and a temperature-sensitive vector containing a target sequence of a microRNA specific for induced pluripotent stem cells. Examples of the reprogramming factors and the vectors used in this aspect are as described above.

### Composition

The present application provides a composition for producing naive induced pluripotent stem cells from human somatic cells, comprising:
one or more vectors containing a reprogramming factor,
wherein the one or more vectors are selected from the group consisting of a temperature-sensitive vector, a vector containing a target sequence of a microRNA specific for induced pluripotent stem cells, and a temperature-sensitive vector containing a target sequence of a microRNA specific for induced pluripotent stem cells. Examples of the reprogramming factors and the vectors used in this aspect are as described above.

The composition of the present application can be produced as a liquid formulation by adding distilled water, a pH regulator, a suspending agent, a solubilizing agent, a stabilizing agent, an isotonic agent, an antioxidant, and a preservative as needed. Examples of pH regulators include hydrochloric acid, sodium hydroxide, lactose, lactic acid, sodium, dibasic sodium phosphate, and monobasic sodium phosphate. Examples of suspending agents include methylcellulose, polysorbate 80, hydroxyethyl cellulose, gum arabic, powdered tragacanth, carboxymethyl cellulose sodium, and polyoxyethylene sorbitan monolaurate. Examples of solubilizing agents include polyoxyethylene hydrogenated castor oil, polysorbate 80, nicotinic-acid amide, and polyoxyethylene sorbitan monolaurate. Examples of stabilizing agents include sodium sulfite, sodium metasulfite, and ether. Examples of isotonic agents include sodium chloride and dextrose. Examples of preservatives include methyl-p-oxybenzoate, ethyl-p-oxybenzoate, sorbic acid, phenol, cresol, and chlorocresol.

### Kit

The present application provides a kit for producing naive induced pluripotent stem cells from human somatic cells, comprising:
one or more vectors containing a reprogramming factor, and
a naive medium, and
wherein the one or more vectors are selected from the group consisting of a temperature-sensitive vector, a vector containing a target sequence of a microRNA specific for induced pluripotent stem cells, and a temperature-sensitive vector containing a target sequence of a microRNA specific for induced pluripotent stem cells. Examples of the reprogramming factors and the vectors used in this aspect are as described above.

In the kit of the present application, the one or more vectors may be packaged separately or together. The kit of the present application may also include a buffer and an instruction.

### Culture supernatant

The present application also provides a culture supernatant of the naive induced pluripotent stem cells produced by the method of the present application. The naive medium described above can be used for the medium. The naive medium can contain one or more compounds selected from Leukemia inhibitory factor (LIF), a MEK inhibitor, a GSK3 inhibitor, a cAMP production stimulator, a TGF-β inhibitor, and a PKC inhibitor. For example, the medium can include t2iLGo, 5iLAF, or tt2iLGo.

The culture period for obtaining the culture supernatant can be 3 to 72 days, e.g., 12 to 48 days. The concentration of the naive induced pluripotent stem cells at the start of the culture can be, for example, 3 × 10³ to 1 × 10⁵ cells/cm², e.g., 1 × 10⁴ to 3 × 10⁴ cells/cm².

The culture temperature for obtaining the culture supernatant is, but is not limited to, about 30 to 40°C, preferably about 37°C. The culture is performed in a CO₂-containing air atmosphere. The CO₂ concentration is about 2 to 8%, preferably about 3 to 7%, more preferably about 4 to 6%, and most preferably about 5%. The culture may also be performed under hypoxic conditions. The oxygen concentration is about 3 to 10%, preferably about 4 to 8%, more preferably about 4 to 6%, and most preferably about 5%.

The culture supernatant of the naive induced pluripotent stem cells can be obtained by separating and removing the cellular components after the culture. In the present disclosure, the culture supernatant includes not only the culture supernatant obtained by separating and removing the cellular components from the culture medium, but also culture supernatant that has been subjected to various treatments (e.g., centrifugation, concentration, solvent substitution, dialysis, freezing, drying, lyophilization, dilution, desalting, and storage) as appropriate.

### Cosmetic product

The present application also provides a cosmetic product containing as raw material the culture supernatant of the naive induced pluripotent stem cells produced by the method of the present application. The culture supernatant of the naive induced pluripotent stem cells produced by the method of the present application is as described above. The cosmetic product of the present application may be in any form or any dosage form as long as it is used in contact with the skin. Specifically, examples of the form or dosage form include, in the broad sense, skin milk, skin cream, foundation cream, massage cream, cleansing cream, shaving cream, cleansing foam, skin lotion, lotion, pack, lipstick, blusher, eye shadow, manicure, soap, body shampoo, hand soap, shampoo, rinse, hair tonic, conditioner, hair cream, hair spray, hair grower, hair restorer, hair dye, hair dressing, depilatory, anti-dandruff agent, dentifrice, denture adhesive, collutory, permanent waving agent, curling agent, styling agent, ointment, cataplasm, tape, bath additive, antiperspirant, and sun protectant. Any type of the form or dosage form can be used as long as it is used in contact with the skin, and the form that can be used as a cosmetic product is particularly preferred. The cosmetic product includes those used in contact with the skin of animals in addition to humans. The cosmetic product of the present application can be in any form, including but not limited to many forms such as solid, liquid, semisolid, gas, powder, granule, tablet, gel, and foam.

The cosmetic product of the present application may contain a pharmaceutically acceptable carrier, excipient, disintegrant, buffer, emulsifier, suspending agent, analgesic agent, stabilizing agent, preservative, antiseptic agent, and saline. Examples of excipients include lactose, starch, sorbitol, D-mannitol, and white soft sugar. Examples of disintegrants include carboxymethylcellulose and calcium carbonate. Examples of buffers include phosphate, citrate, and acetate. Examples of emulsifiers include gum arabic, sodium alginate, and tragacanth. Examples of suspending agents include glyceryl monostearate, aluminum monostearate, methylcellulose, carboxymethylcellulose, hydroxymethylcellulose, and sodium lauryl sulfate. Examples of analgesic agents include benzyl alcohol, chlorobutanol, and sorbitol. Examples of stabilizing agents include propylene glycol and ascorbic acid. Examples of preservatives include phenol, benzalkonium chloride, benzyl alcohol, chlorobutanol, and methylparaben. Examples of antiseptic agents include benzalkonium chloride, p-hydroxybenzoic acid, and chlorobutanol.

### EXAMPLE

The present invention is described in more detail referring to following examples. The present invention, however, is not limited by those Examples in any way.

### Materials and methods

### Cell culture

The inventors obtained human dermal fibroblasts (HDFs) collected under informed consent from the Tokyo Metropolitan Institute of Gerontology. HDFs and irradiated mouse embryonic fibroblasts (iMEFs) were maintained in Dulbecco's modified Eagle medium (DMEM, Nacalai Tesque) supplemented with 10% fetal bovine serum (FBS, Japan Bio Serum). PBMCs purchased from Cellular Technology Limited (CTL) were maintained under the manufacturer's recommended conditions. Primed and naive ES cell (ESC) clones were obtained from WiCELL and Kyoto University. Primed pluripotent stem cells (PSCs) were maintained in StemFit AK02N medium (Ajinomoto) on laminin 511-E8 fragments (iMatrix-511, Nippi)-coated plates. Naive PSCs were cultured on iMEFs and maintained in t2iLGo² medium composed of N2B27 medium (NDiff227, Takara Bio) with 1 µM CHIR99021 (Merck), 1 µM PD0325901 (Merck), 10 µg/mL human LIF (Peprotech), and 2.5 µM Go6983 (Merck). Feeder-free naive induced pluripotent stem cells (iPSCs) were maintained on Matrigel (hESC-qualified, Corning). The inventors changed the medium every other day and added 10 µM Y27632 (Wako) just before every medium change. Naive iPSCs were passaged every 3-4 days using Accutase (Innovative Cell Technologies) and cultured under 5% O₂ throughout. Recombinant DNA experiments in the examples were carried out under the approval of Kyoto University.

### Construction of modified SeV-KLF4 vectors

The insert sequence containing the open reading frames of human KLF4 gene was constructed by PCR from cDNAs using NotI-tagged gene-specific forward and reverse primers including SeV-specific transcriptional regulatory signal sequences. The amplified fragment was inserted into the 18+ region of plasmids containing the genomic sequence of SeV/TS12ΔF, SeVIPmiR367T21TSΔF, and SeV/PmiR367T2/TS12ΔF vectors to produce plasmids pSeV18+KLF4/TS12ΔF, pSeV18+KLF4/PmiR367T2/TSΔF, and pSeV18+KLF4/PmiR367T2/TS12ΔF (collectively, "SeV-KLF4 vector plasmids"), respectively. The sequence of miR367T2 was TCACCATTGCTAAAGTGCAATTcgatTCACCATTGCTAAAGTGCAATT (SEQ ID NO: 1). The recovery and propagation of SeV-KLF4 vectors were performed as follows. First, 293T cells were transfected with SeV-KLF4 vector plasmids and plasmids carrying the T7 RNA polymerase and NP, P, F5R, and L genes. The cells were maintained in DMEM supplemented with 10% heat-inactivated fetal bovine serum (FBS) and cultured for 1-3 days to generate the seed of the SeV-KLF4 vectors. The seeds were cloned and propagated using SeV F-expressing LLC-MK2/F7/A cells in MEM containing trypsin (2.5 µg/ml). Titer (cell infectious units/mL) of the recovered SeV-KLF4 vectors were determined by the immunostaining method using anti-SeV rabbit polyclonal sera as described previously (Fusaki, N., Ban, H., Nishiyama, A., Saeki, K. & Hasegawa, M. Efficient induction of transgene-free human pluripotent stem cells using a vector based on Sendai virus, an RNA virus that does not integrate into the host genome. Proceedings of the Japan Academy, Series B 85, 348-362, doi:10.2183/pjab.85.348 (2009).).

### Reprogramming of human somatic cells into naive iPSCs

In the conventional method, HDFs or PBMCs were reprogrammed with the CytoTune-iPS 2.0 or 2.0L reprogramming kit. In the new method, the inventors replaced the KLF4/TS vector, which was originally contained in the kit, with a new SeV-KLF4 vector, such as KLF4/TS12, KLF4IPmiR367T21TS or KLF4/PmiR367T2/TS12. The incubation temperature was maintained at 35°C until the naive iPSC colony generation. After the first passage of the generated iPSCs (usually day 14), the incubation temperature was changed to 38°C to remove the SeV vectors. After the removal of the SeV vectors (day 34 when SeV-KLF4/PmiR367T2/TS12 was used), the temperature was changed to 37°C. The incubator was set to 5% O₂ after the SeV-vector infection.

HDFs were initially maintained in DMEM (Nacalai Tesque) supplemented with 10% FBS. At day 0, the cells were counted and infected with three SeV vectors: 1) polycistronic KLF4-OCT4-SOX2, 2) CMYC or LMYC and 3) KLF4 at a multiplicity of infection of 5, respectively. From day 1, the medium was changed every other day. At day 5, the cells were reseeded on iMEF feeder cells. The next day the medium was changed to the naive medium.

In the PBMC reprogramming, 5 days before the SeV-vector infection, PBMC culture was started in StemSpan ACF (STEMCELL) with 100 ng/mL human SCF (R&D), 100 ng/mL human TPO (R&D), 100 ng/mL human Flt3/Flk2 (R&D), 50 ng/mL human IL-6 (R&D), and 20 ng/mL human IL-3 (R&D). At day 0, the cells were counted and infected with the three SeV vectors using the same reprogramming procedure as for HDFs. At day 1, the cells were counted and reseeded on the same condition as with HDF reprogramming. From day 2, the inventors added the same amount of PSC medium as PBMC medium every other day. At day 8, the inventors completely changed the medium to the naive medium.

### RNA, DNA and miRNA extraction and quantitative real-time PCR (qRT-PCR)

The inventors removed iMEFs by incubating naive PSCs for 2 hours at 37°C on a gelatin-coated dish before sampling for nucleic acid extraction. Total RNA and DNA were extracted from cell lysates using the AllPrep DNA/RNA Mini Kit (QIAGEN), and the RNA was incubated with RNase-Free DNase Set (QIAGEN) to remove genomic DNA. MicroRNA (miRNA) was extracted using NucleoSpin miRNA (MACHEREY-NAGEL). For qRT-PCR, the reverse transcription reaction was performed with 1 µg of DNase-treated RNA using PrimeScript RT Master Mix (Takara) containing oligo dT primer and random 6 mers. cDNA was synthesized using TaqMan Advanced miRNA Assays (Applied Biosystems). qRT-PCR was performed on StepOne Plus (Applied Biosystems) or QuantStudio3 (Applied Biosystems) using Fast SYBR Green Master Mix (Applied Biosystems) or TaqMan Fast Advanced Master Mix (Applied Biosystems).

### RNA sequencing and data analysis

RNA sequencing libraries were made from 100 ng of total RNA as starting materials with the TruSeq Stranded Total RNA Library Prep Gold (illimina) following the manufacturer's protocol. For Hiseq2500, clusters were generated with the HiSeq PE Cluster Kit v4-cBot (illumina) using illumina cBot. Sequencing was performed with the HiSeq SBS Kit v4 using HiSeq2500 (2 × 126 PE mode). The inventors also used NovaSeq 6000 (2 × 101 PE mode) with the NovaSeq 6000 S1 Reagent Kit v1.5 (illumina) and NextSeq 500 (76 SE mode) with the NextSeq 500/550 High Output Kit v2.5 for the sequencing. FASTQ files were generated from bcl files using bcl2fastq v2.17.1.14 (illumina) and processed using ENCODE long-ma-seq-pipeline v2.3.4. Briefly, the sequenced reads were mapped to the human reference genome (GRCh38) using STAR 2.5.1b with GENCODE v24 gene annotations, the normalized gene expression data was calculated using RSEM 1.2.23, and the gene count data was obtained using featureCounts bundled in Subread 1.5.1. For the characterization of the inventors' PSC lines, the data sets of GSE59435 and GSE75868 obtained from GEO and supplemental data in L. Yan et al. (Yan, L. et al. Single-cell RNA-Seq profiling of human preimplantation embryos and embryonic stem cells. Nature structural & molecular biology 20, 1131-1139, doi:10.1038/nsmb.2660 (2013).) and Y. Takashima et al. (Takashima, Y. et al. Resetting transcription factor control circuitry toward ground-state pluripotency in human. Cell 158, 1254-1269, doi:10.1016/j.cell.2014.08.029 (2014).) were used. The expression values of 4,720 genes included in all data were normalized by quantile among samples, and z-scores for each gene were used for the PCA. Log2-scaled, quantile normalized FPKM values were used for the expression heatmap of PSC markers and oxidative phosphorylation-related genes.

### DNA methylation analysis

The bisulfite conversion of 500 ng genomic DNA was performed using the EZ DNA Methylation Kit (Zymo Research), and the global DNA methylation status was profiled using Infinium Human Methylation 450K or EPIC BeadChip Kit (illumina) according to the manufacturer's protocols. After exporting the DNA methylation values using GenomeStudio V2011.1, data processing was conducted using the "minfi" package in R 3.6.3. In total, 424,444 probes common between 450K and EPIC and not located at known SNP sites were used for the PCA of PSC samples.

### RNA-FISH

Dissociated naive iPSCs were incubated on a gelatin-coated dish for 2 hours at 37°C to remove iMEF feeder cells. Then the cells were seeded on Matrigel-coated slides in PSC medium. On the next day, the cells were fixed in 4% paraformaldehyde for 15 min at room temperature. The slides were treated with 0.2 M HCl for 20 min, permeabilized with 0.2% Triton X-100 for 10 min, digested with pepsin solution (0.005% in 0.1 M HCl) at 37°C for 2-6 min and dehydrated. Bacterial artificial chromosomes (BACs) RP11-155024 and RP11-256P2 were used to generate HUWE1 and UTX RNA FISH probes, respectively. BAC DNAs were labelled by nick-translation with Cy5-dUTP (RP11-155O24) and Cy3-dUTP (RP11-256P2). The labelled probes and the XIST RNA FISH probe (Chromosome Science Labo) were mixed with sonicated salmon sperm DNA and Cot-1 DNA in hybridization solution. The probes were denatured at 85°C for 10 min, applied to the pretreated slides, covered with cover slips and hybridized at 37°C overnight. The slides were then washed with 50% formamide / 2xSSC at 37°C for 20 min, 1xSSC for 15 min at room temperature, counterstained using DAPI and mounted. The FISH images were captured with the CW4000 FISH application program (Leica Microsystems Imaging Solution) using a cooled CCD camera mounted on a Leica DMRA2 microscope.

### Naive PSC-derived primitive endoderm differentiation

For primitive endoderm differentiation, naive PSCs were dissociated and iMEF feeder cells were removed as described above. 500,000 cells were plated in laminin 511-E8 (0.4 µg/cm² iMatrix 511; Nippi) -coated 6 wells with initial primitive endoderm differentiation medium: Ndiff227, 25 ng/ml FGF4 (Peprotech), 1 µg/ml Heparin (Wako), 10 ng/ml BMP4 (R&D), 10 ng/ml PDGFAA (Peprotech), 1 µM XAV939 (Sigma), 3 µM A83-01 (Wako), 0.1 µM RA (Sigma). The following day, the medium was changed to the same medium as above. The next day, the medium was changed to the same medium to which 10 ng/ml IL-6 (R&D) was added. At day 3, the cells were detached and harvested using Trypsin/EDTA (Nacalai) for 5 min and analyzed for PDGFRA and ANPEP expression by flow cytometry.

### Naive PSC-derived trophectoderm (TE) differentiation

For TE differentiation, naive PSCs were dissociated and iMEF feeder cells were removed as described above. 500,000 cells were plated in laminin 511-E8 (0.15 µg/cm² iMatrix 511; Nippi) -coated 6 wells with initial TE differentiation medium: Ndiff227, 2 µM A83-01 (Wako), 2 µM PD0325921 (Sigma) and 10 ng/mL BMP4 (R&D). The following day, the medium was changed to Ndift'2.27, 2 µM A83-01 (Wako), 2 µM PD0325921 (Sigma) and 1 µg/ml JAK inhibitor I (Merck). The next day, the medium was changed again. At day 3, the cells were harvested using Accutase (Innovative Cell Technologies) for 30 min and analyzed for TACSTD2, ENPEP, HAVCR1 and HLA-ABC expression by flow cytometry.

### Flow cytometry

Dissociated cells were stained on ice for 20 min with Alexa Fluor 488-conjugated TROP-2 (TACSTD2), PE-conjugated CD249 (ENPEP), Tim-1 (HAVCR1) biotinylated antibody and Pacific Blue-conjugated HLA-ABC antibody. After washing, APC Streptavidin was applied and incubated on ice for 20 min. Analyses were performed using the BD LSR Fortessa (BD Biosciences) and FACSAria II (BD Biosciences) flow cytometer equipped with FACS Diva software (BD biosciences). The data were analyzed using FlowJo software (LLC).

### Statistics and reproducibility

Exact n values are described in the description of the relevant figures. Statistical significance was determined by two-tailed unpaired Student's t-test using Prism software (GraphPad). P < 0.05 were considered significant and are indicated by asterisks in the figures. Error bars represent the mean ± s.d.

### Data availability

Sequencing and array data were deposited in the GEO under the accession number GSE179476.

### Example 1. Production of Sendai virus vectors (SeVs) used in this example

The structure of the Sendai virus vectors used in this example is shown below. In this specification, "18+" refers to the insertion of a reprogramming factor before the NP gene; "PM" refers to the insertion of a reprogramming factor between the P and M genes; and "HNL" refers to the insertion of a reprogramming factor between the HN and L genes. In this specification, "TS" refers to having G69E, T116A, and A183S mutations in the M protein, A262T, G264R and K461G mutations in the HN protein, L511F mutation in the P protein, and N1197S and K1795E mutations in the L protein. In this example, "TS7" refers to having Y942H, L1361C, and L1558I mutations in the L protein in addition to the TS mutation; "TS12" refers to having D433A, R434A, and K437A mutations in the P protein in addition to the TS mutation; and "TS15" refers to having L1361C and L1558I mutations in the L protein in addition to the TS12 mutation. "ΔF" indicates that the F gene is deleted. "KOS" refers to the carring of all of KLF4, Oct4 and Sox2. For example, an F gene-deleted Sendai virus vector with TS12 mutation carrying KLF4, Oct4 and Sox2 between the P and M genes is described as PM/KOS/TS12ΔF. Similarly, an F gene-deleted Sendai virus vector with the TS mutation carrying KLF4 before the NP gene is described as 18+/KLF4/TSΔF.

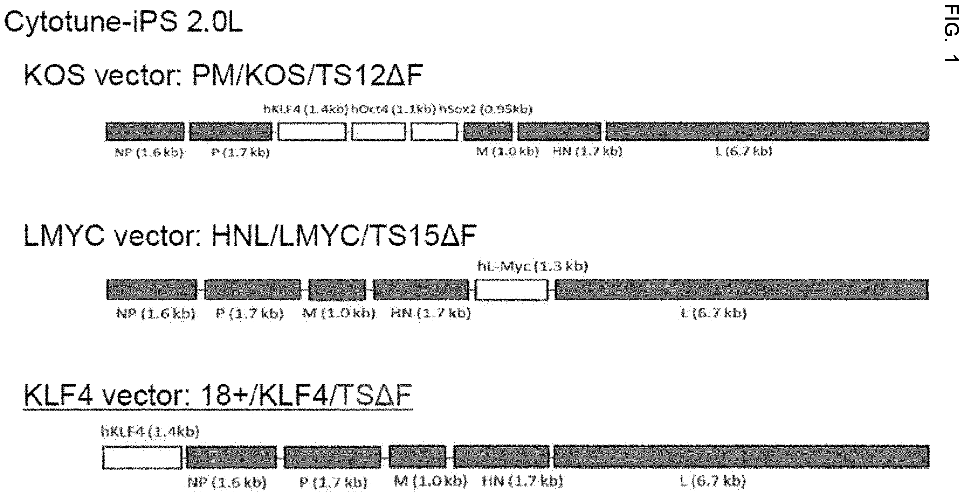

The Sendai virus vector (CytoTune-iPS2.0L) conventionally used to produce naive pluripotent stem cells from somatic cells is shown in Fig. 1. The carried cDNAs are shown in white. The Sendai virus vectors used in this example are the system of the Sendai virus vectors shown in Fig. 1 with one of "18+/KLF4/TS7ΔF", "18+/KLF4/TS12ΔF" or "18+/KLF4/PmiR 367T2/TSΔF" instead of "18+/KLF4/TSΔF" as the KLF4 vector. "PmiR367T2" indicates that miR367 recognition sequences are carried in tandem on the 5' side of the P gene.

The KOS vector "PM/KOS/TS12ΔF" and the LMYC vector "HNLILMYC/TS15ΔF" were produced by conventional methods (WO2012/029770 and WO2010/008054). The "18+IKLF41TS7ΔF" and "18+/KLF4/TS12ΔF" were produced in the same manner as "18+/KLF4/TSΔF" (WO2010/008054) using the TS7 and TS12 vector skeletons, respectively. The "18+/KLF4/PmiR367T2/TSΔF" was produced by conventional methods (WO2017/082174).

### Example 2. Production of naive iPS cells from human dermal fibroblasts (HDFs)

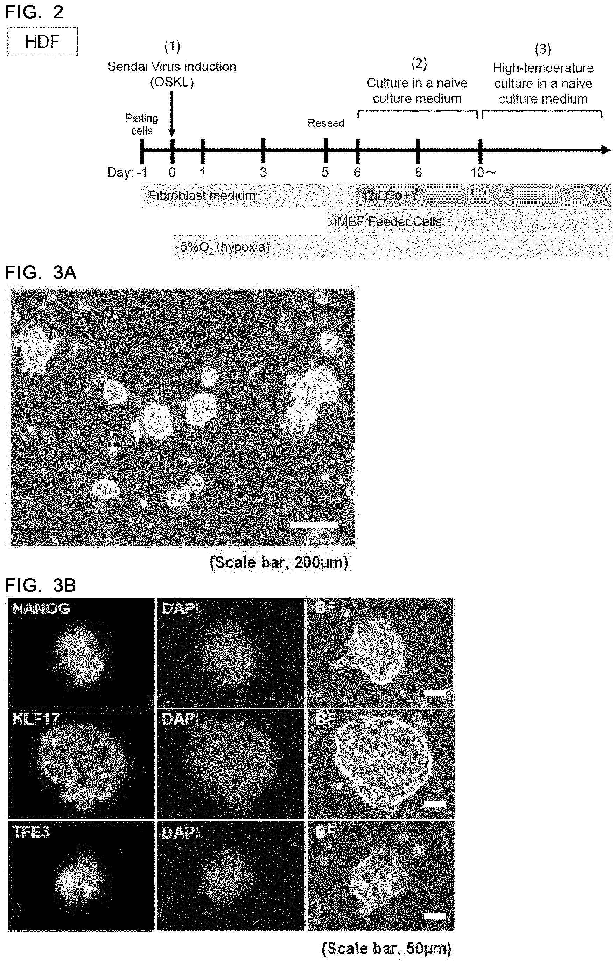

The protocol for producing naive iPS cells from human dermal fibroblasts (HDFs) is shown in Fig. 2.

### Step (1)

For SeV infection and cell counting, human dermal fibroblasts were seeded in equal numbers in a gelatin-coated 12-well or 6-well plate, and cultured in Fibroblast medium (DMEM+10%FBS) at 37°C under normal oxygen conditions (approximately 20%) one day before the infection with the Sendai virus vector. The next day (day 0), the cells seeded for cell counting were detached using cell detachment solution, counted for cell number, infected with the Sendai virus vectors at the dose in accordance with the cell count, and then incubated in a hypoxic (5% O₂) environment at 35°C. The amounts of the Sendai virus vectors for the infection are, for example, KOS: MOI5, LMYC: MOI5, and KLF4: MOI5, and the conditions were studied as appropriate for the cells. After 24 hours (day 1) and at days 2 and 4, the medium was replaced with new Fibroblast medium. The cell culture vessel seeded with iMEFs (irradiated mouse embryonic fibroblasts) was prepared at day 4. The cells were detached using cell detachment solution at day 5 and counted for cell number, and then any number of the cells were seeded onto the cell culture vessel seeded with iMEFs. In this example, the culture was performed under 5% CO₂ conditions.

### Step (2)

The medium was changed to the naive medium (t2iLGo+Y) from day 6 and then changed every other day.

The composition of t2iLGo+Y medium is shown below. All concentrations listed are final concentrations:
NDiff227 (Takara Bio)
CHIR99021 (Sigma-Aldrich) 1 µM
PD0325901 (Sigma-Aldrich) 1 µM
Go6983 (Sigma-Aldrich) 1.25 µM or 2.5 µM
Human recombinant leukemia inhibitory factor (FUJIFILM Wako Pure Chemical Corporation) 10 ng/ml
Y27632 (FUJIFILM Wako Pure Chemical Corporation) 10 µM.
Y27632 may not be added after the next day of the passage.

From about day 10, multilayered and dome-shaped colonies (naive iPS cell colonies) appeared.

### Step (3)

After day 10, when the multilayered and dome-shaped colonies reached sufficient sizes (50 to 200 µm in diameter), passage was performed and high-temperature culture (basically 38°C) was started on the next day.

The t2iLGo+Y medium was changed every other day and the culture was performed in an incubator with 5% oxygen. The passages were performed on every 3 to 4 days. Cell detachment at passaging was performed by removing the medium, washing the cells once with D-PBS (Nacalai Tesque), adding Accutase (Innovative Cell Technologies), and incubating them at 37°C for 7 to 10 minutes. When the only purpose was the maintenance, for example, about 1/5 of the number of the cells obtained by the detachment was seeded into a culture vessel of the same area.

The phase-contrast microscopy image of the cells after 45 passages is shown in Fig. 3A. Many multilayered and dome-shaped colonies were observed, and few single-layered and flattened colonies were observed. Fig. 3B shows images of the dome-shaped colony in Fig. 3A immunostained with naive type specific markers (NANOG, KLF17) and a gene product (TFE3) that has different subcellular localization between naive and primed types. Hoechst indicates nuclear staining; PC indicates phase contrast microscopy image. As shown in Fig. 3B, the cells composing the dome-shaped colony were strongly stained for NANOG and KLF17, and the nuclei were densely stained for TFE3. Accordingly, induced pluripotent stem cells were obtained that expressed naive type specific markers, exhibited naive type specific subcellular localization of specific gene products (the gene expression pattern equivalent to that of inner cell mass of preimplantation embryos), and formed multilayered and dome-shaped colonies (colony morphology characteristic of naive type).

Thus, it was demonstrated that naive iPS cells can be produced from human fibroblasts by the method comprising above steps (1) to (3).

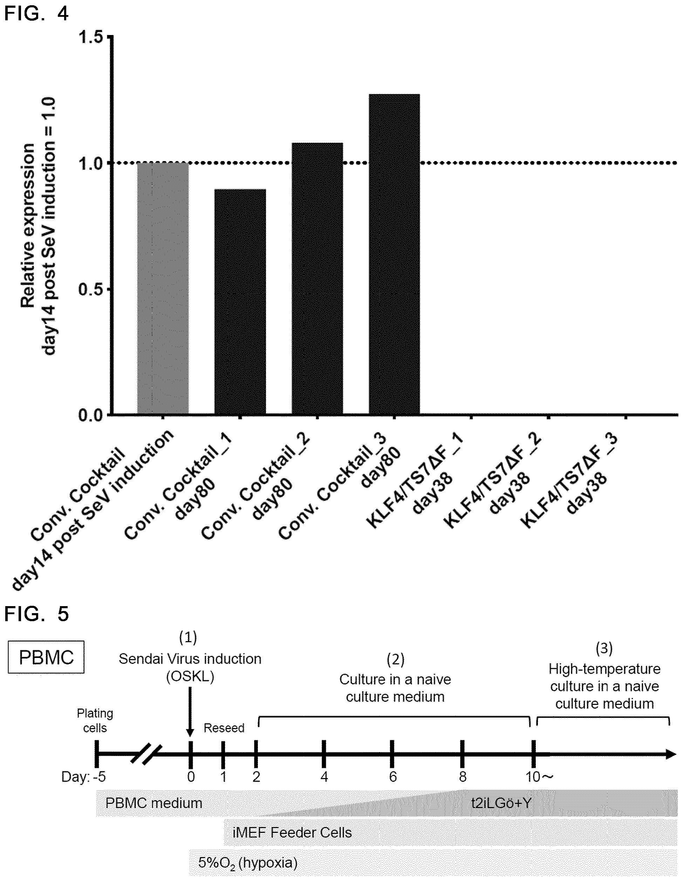

Next, the amount of the Sendai virus vectors remaining in the cells was analyzed. The conventional vector (CytoTune-iPS2.0L) was used for comparison. The intracellular genome amount of the Sendai virus vectors was measured by quantitative PCR. Figure 4 shows the results expressed as relative values with the genome amount at day 14 as 1.0. "Conv. Cocktail" represents CytoTune-iPS2.0L, and "KLF4/TS7ΔF" represents CytoTune-iPS2.0L with "18+IKLF41TS7ΔF" instead of the KLF4 vector of CytoTune-iPS2.0L.

As shown in Fig. 4, the vector used in this example was already reduced to below the detection limit by day 38. Accordingly, it was shown that the vector used in this example was rapidly eliminated by high temperature culture at 38°C (KLF4/TS7ΔF_1 to 3). When "18+/KLF4/TS12" or "18+/KLF4/TS12/miR367T2" was used as the KLF vector and the culture was performed at 38°C, the detection limit was reached 8 to 12 days earlier (data not shown).

In contrast, the conventional vector remained at day 80 at the same level as at day 14 (ConvCocktail_1 to 3). It is known that under culture conditions using normal medium for producing primed induced pluripotent stem cells, even at normal culture temperature (37°C), the vector gradually decreases and almost disappears by day 80. Accordingly, it is strongly suggested that the disappearance rate of the vector may be affected by the cell culture environment.

Therefore, in order to obtain naive pluripotent stem cells not having reprogramming factors derived from the vectors at an early stage, the vector that is temperature-sensitive in the culture using the naive medium or the vector containing a target sequence of microRNA specific for induced pluripotent stem cells can be used. It was shown that the Sendai virus vector with TS7, TS12 or TS15 mutation can be used as one example of said temperature-sensitive vector.

### Example 3 Production of naive iPS cells from human peripheral blood mononuclear cells (PBMCs)

The protocol for producing naive iPS cells from human peripheral blood mononuclear cells (PBMCs) is shown in Fig. 5.

### Step (1)

PBMCs were cultured in a medium for PBMCs at 37°C under normal oxygen conditions (approximately 20%) five days before the infection with the Sendai virus vectors. The medium was not exchanged. The composition of the medium for PBMCs (PBMC medium) is shown below. All concentrations listed are final concentrations:
StemSpan ACF (STEMCELL Technologies)
Recombinant Human SCF (R&D Systems) 100 ng/ml
Recombinant Human TPO (R&D Systems) 100 ng/ml
Recombinant Human Flt3/Flk2 (R&D Systems) 100 ng/ml
Recombinant Human IL-6 (R&D Systems) 50 ng/ml
Recombinant Human IL-3 (R&D Systems) 20 ng/ml.

On the day of the infection (day 0), the number of cells was measured and the cells were infected with the Sendai viruses at the doses in accordance with the number of cells, and then incubated in a hypoxic (5% O₂) environment at 35°C. The amounts of the Sendai viruses to be infected are, for example, KOS: MOI5, LMYC: MOI5, and KLF4: MOI5, and the conditions were studied as appropriate for the cells. For the infection, the cells were generally infected with the viruses in 24-well or smaller wells in order to increase the density of the viruses. On the same day, the cell culture vessel (generally 6 wells) seeded with iMEFs was prepared. Twenty-four hours after the infection (day 1), the cells were collected, the medium containing the viruses was removed, the number of the cells was measured, and then any number of the cells were seeded onto the cell culture vessel seeded with iMEFs. In this example, the culture was performed under 5% CO₂ conditions.

### Step (2)

At day 2, half of the medium for PBMC was removed and the same amount of the naive medium (t2iLGo+Y) was added. Half of the medium was then removed and the same amount of the new medium was added every other day in the same manner. At day 8, the medium was completely aspirated and replaced with the naive medium. The composition of the naive medium (t2iLGo+Y) was the same as that used to produce naive iPS cells from human dermal fibroblasts in Example 2.

### Step (3)

From about day 10, multilayered and dome-shaped colonies (naive iPS cell colonies) appeared. After day 10, when the colonies reached sufficient sizes (50 to 200 µm in diameter), passage was performed and high-temperature culture (basically 38°C) was started on the next day. The methods for maintenance culture and passaging are the same as those used to produce naive iPS cells from human dermal fibroblasts in Example 2.

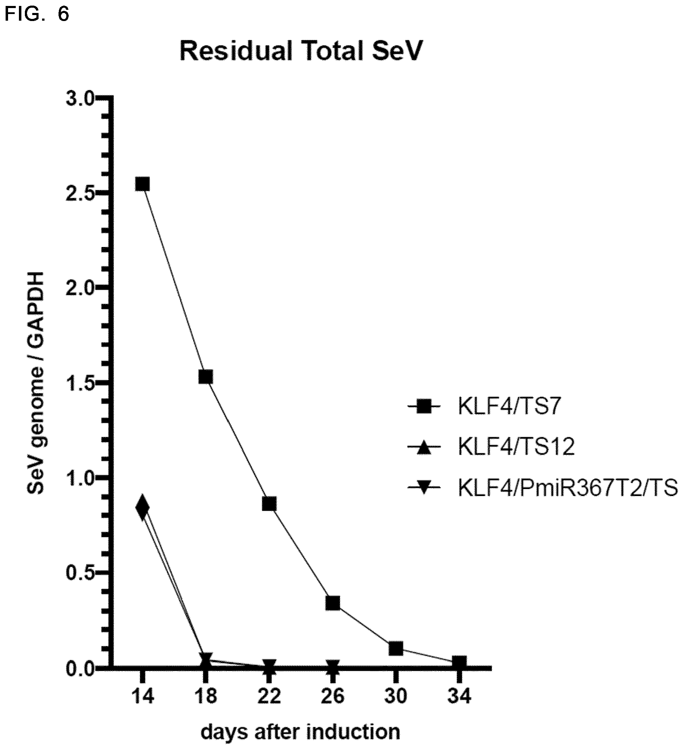

The genome amount of the Sendai virus vectors was quantified by qPCR at each passage from day 14, when the culture at 38°C was initiated (Fig. 6). When 18+/KLF4/TS7ΔF was used as the KLF4 vector, the amount of the vectors was below the detection limit at day 38. When 18+/KLF4/TS12ΔF or 18+IKLF4IPmiR367T21TSΔF was used, the amount of the vectors was below the detection limit at day 30.

Therefore, it was shown that naive induced pluripotent stem cells are produced from human peripheral blood mononuclear cells by the method comprising above steps (1) to (3), and that naive iPS cells substantially free of reprogramming factors are obtained after about 30 to 38 days.

Thus, it was revealed that naive induced pluripotent stem cells (more specifically, naive iPS cells that are substantially free of reprogramming factors) can be promptly produced from various human somatic cells by using the method of the present application.

### Example 4. Phenotypic stability of naive induced pluripotent stem cells

The inventors analyzed the phenotypic stability of the naive induced pluripotent stem cells obtained in Examples 2 and 3 after long-term passages.

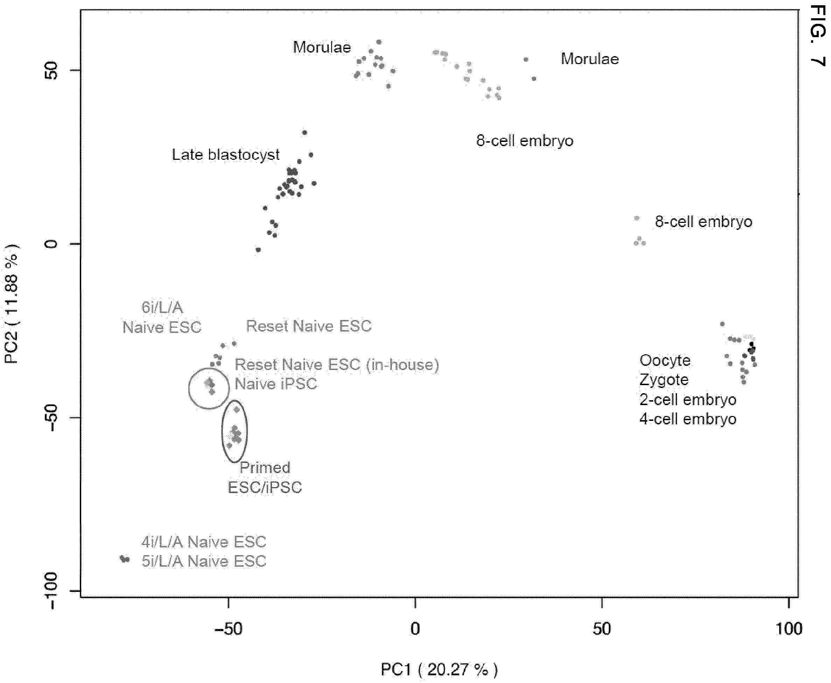

The inventors performed principal component analysis integrating single cell data for reset naive ES cells (Reset Naive ESC (in-house)), which is the standard for naive induced pluripotent stem cells, 8 clones of HDF-derived primed iPS cells established by the inventors, 4 clones of HDF-derived naive iPS cells after long-term passages (P16 to P45) obtained by using the present invention, 2 clones of PBMC-derived naive iPS cells, previously reported naive ES cells (Reset Naive ESC, 6/5/4iLA Naive ESC), and human embryos (Fig. 7). The naive iPS cells established by the inventors are shown in the red circled area, and Reset Naive ESCs (in-house) are shown in the same area, indicating that the global gene expression of the naive iPS cells established by the inventors is equivalent to that of Reset Naive ESCs.

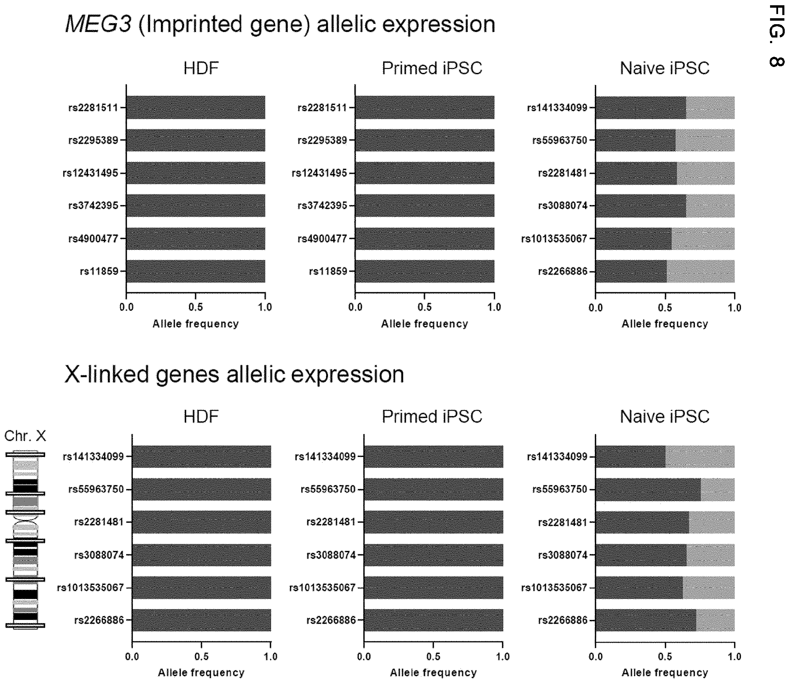

Based on the data obtained from the RNA-seq in Fig. 7, the expression patterns of imprinted genes and X chromosome-related genes in each cell type were compared and verified by SNP analysis (Fig. 8). The HDFs, primed iPS cells, and naived iPS cells were all derived from the same female HDFs. In the HDFs and primed iPS cells, the imprinted genes such as MEG3 generally showed the expression pattern of only one allele, whereas in the naive cells, the imprinting was lost and the genes showed the expression pattern of both alleles. In the female-derived HDFs and primed iPS cells used in this verification, the X chromosome-related genes showed the expression pattern of only one allele due to the inactivation of one X chromosome, whereas in the naive iPS cells, the genes showed the expression pattern of both alleles with X chromosome reactivation.

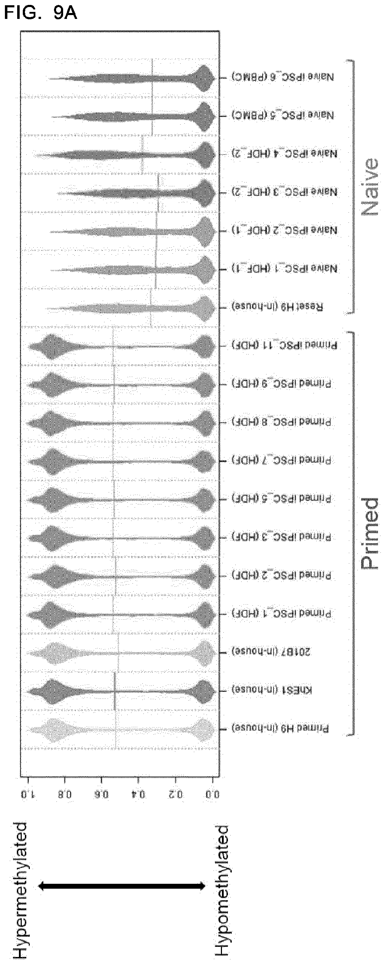

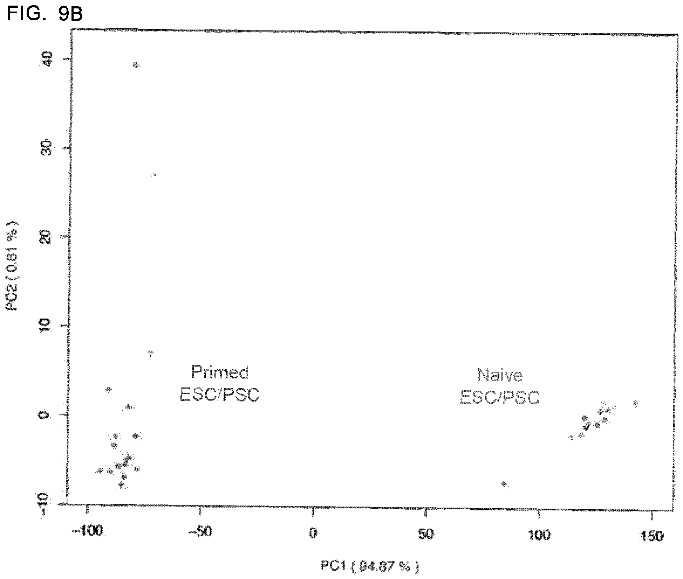

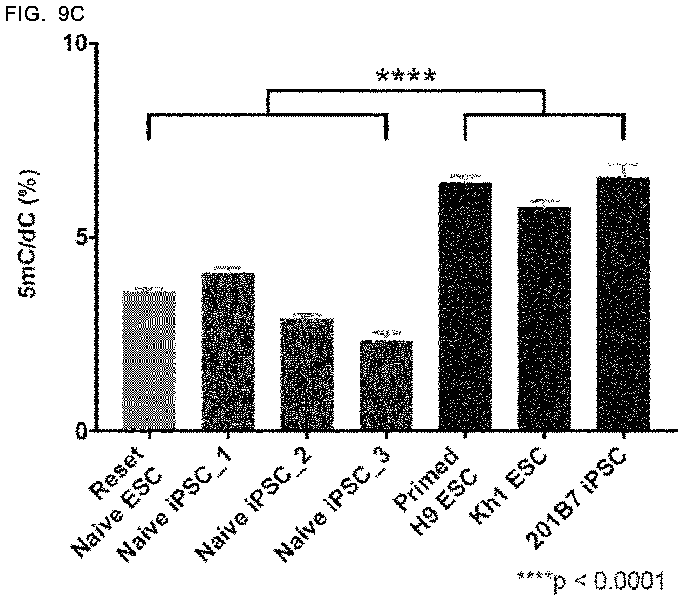

Global methylation of HDF-derived and PBMC-derived naive iPS cells is presented as density bean plots (Fig. 9A) and principal component analysis (Fig. 9B) by methylation arrays using DNA obtained simultaneously with RNA from the cells used for RNA-seq in Fig. 7. "Primed" indicates primed iPS cells established by the applicant, and "Naive" indicates naive iPS cells. In Fig. 9A, the naive cells obtained by the present invention tended to have lower beta values (= hypomethylated) than the primed ESCs, and showed hypomethylation comparable to Reset Naive ESC (in-house). In Fig. 9B, the primed and naive cells are clearly distinguished, indicating that they have different methylation patterns. The percentage of 5-methylcytosine in total cytosine in the HDF-derived naive iPS cells obtained in this example was analyzed by LC-MS/MS (Fig. 9C). The naive iPS cells showed the low percentage comparable to Reset Naive ESCs, suggesting a reduced percentage of methylated cytosine in the genome.

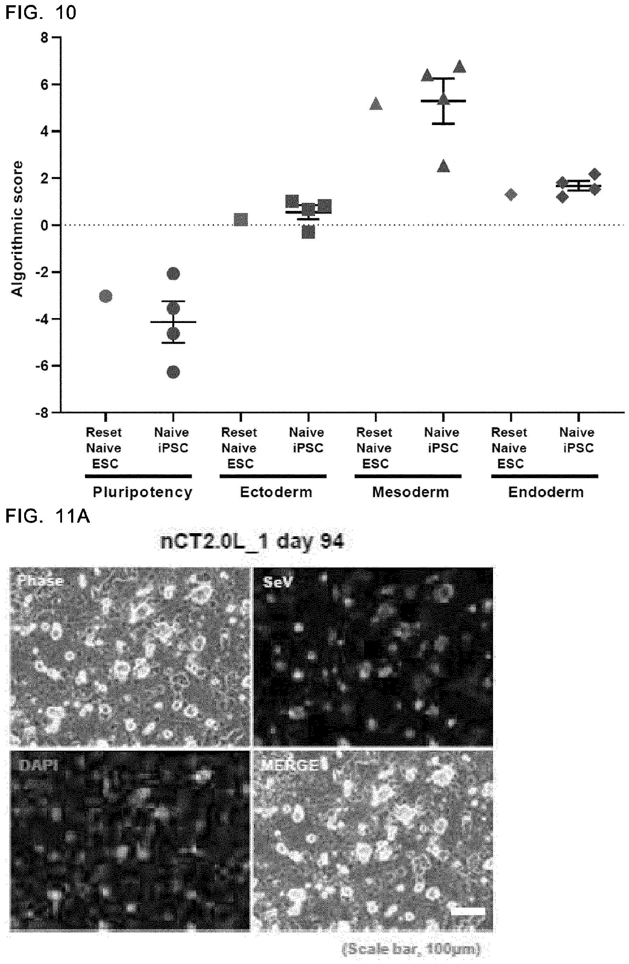

The inventors compared and analyzed the early differentiation potential of the triploblastic components in two clones of HDF-derived naive iPS cells after long-term passages (P16 to 45) used for RNA-seq and methylation arrays, two clones of PBMC-derived naive iPS cells, and Reset Naive ESCs (in-house) as control (Fig. 10). 10 × 10⁶ ES cells/iPS cells were seeded in 96-well plates and cultured in differentiation medium (DMEM+20%FBS) for 24 days under hypoxic (5%O₂) conditions, and the resulting embryoid bodies were collected. RNA was extracted from the collected samples, cDNA was obtained by reverse transcription, and then the early differentiation potential of triploblasts was semi-comprehensively evaluated using TaqMan hPSC Scorecard Panel (ThermoFisher Scientific). The expression levels of pluripotency markers, ectoderm markers, mesoderm markers, and endoderm markers are shown in order from the left end of the figure. The gray plots indicate Reset Naive ESCs, and the red plots indicate the naive iPS cells obtained by the present invention. All clones exhibited the pluripotency comparable to that of Reset Naive ESCs, or better than that of Reset Naive ESCs especially in the mesoderm.

Therefore, it was confirmed that the naive induced pluripotent stem cells obtained by the method of the present application, even after long-term passages of more than 180 days, show the global gene expression pattern, extensive DNA demethylation across the entire genome, X chromosome reactivation, and early differentiation potential of the triploblastic components comparable to those of the standard of naive induced pluripotent stem cells.

Thus, it was shown that true naive induced pluripotent stem cells can be promptly produced by the method of the present application.

### Example 5. Development of modified OSKL-SEV vector reprogramming system

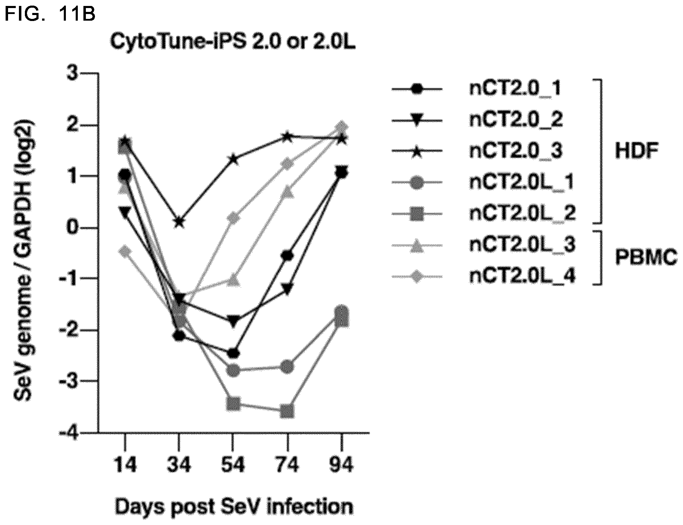

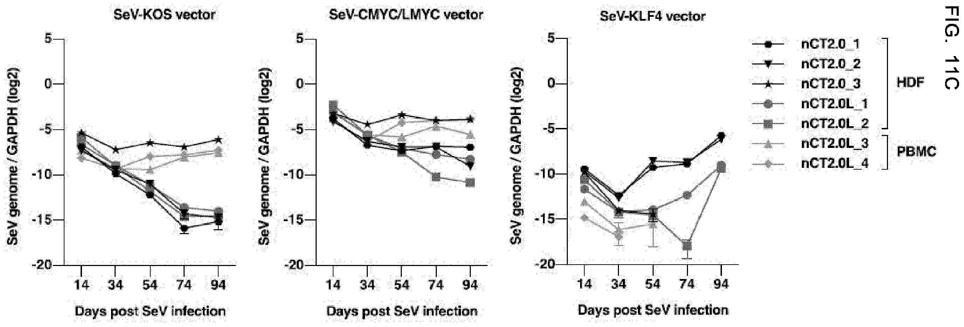

Naive human iPS cells (nCT2.0L_1-4) were established from human dermal fibroblasts (HDFs) by using a commercially available CytoTune-iPS 2.0L SeV reprogramming kit containing SeV(PM)KOS/TS12ΔF (SeV-KOS), SeV(HNL)LMYC/TS15ΔF (SeV-LMYC), and SeV18+KLF4/TSΔF (SeV-KLF4) vectors. On day 94 from SeV introduction, the existence of residual SeV in the established naive human iPSCs was verified by immunostaining (Fig. 11A). As shown in the upper right panel of Fig. 11A, most cells were strongly positive, suggesting a high degree of SeV persistence. Next, the inventors sampled the cells at each passage and measured the amount of remaining SeV genome by quantitative RT-PCR (qRT-PCR). When commercially available CytoTune-iPS 2.0 containing SeV-KOS, SeV(HNL)CMYC/TS15ΔF (SeV-CMYC), and SeV-KLF4 vectors (OSKM) or a CytoTune-iPS 2.0L (OSKL) was used, the amount of SeVs in HDFs or human peripheral blood mononuclear cells (PBMCs) temporarily decreased with each passage, but then increased again, and therefore SeVs were not lost after repeated passages (Fig. 11B). The expression of the SeV genome persisted in all clones, and in some cell lines, the expression level was equal to or even greater than the housekeeping gene GAPDH. By quantifying the expression levels of each of the three residual SeV vector genomes, the inventors found that SeV-KOS and SeV-CMYC/LMYC vectors in all clones and SeV-KLF4 vectors in more than half of the clones persisted at especially high levels (Fig. 11C).

The inventors then considered the reason why these vectors persisted. SeV vectors share virus proteins in mixed infections, and the SeV-TSΔF vector, which forms the backbone of the SeV-KLF4/TS vector, is known to have very weak temperature-sensitivity and therefore difficult to remove from the infected cells by raising temperature unlike SeV-TS12ΔF or SeV-TS15ΔF vectors. The presence or absence of point mutations in each gene in the SeV-TSΔF, SeV-TS12ΔF and SeV-TS15ΔF vectors is shown in Table 1.

**Table 1**

| SeV gene | Mutation | TSΔF | TS12ΔF | TS15ΔF |
|---|---|---|---|---|
| *P* | D433A | | ● | ● |
| | R434A | | ● | ● |
| | K437A | | ● | ● |
| | L511F | ● | ● | ● |
| *M* | G69E | ● | ● | ● |
| | T116A | ● | ● | ● |
| | A183S | ● | ● | ● |
| *HN* | A262T | ● | ● | ● |
| | G264R | ● | ● | ● |
| | K461G | ● | ● | ● |
| *L* | N1195S | ● | ● | ● |
| | K1361C | | | ● |
| | L1558I | | | ● |
| | K1796E | ● | ● | ● |

The inventors therefore hypothesized that the presence of SeV18+KLF4/TSΔF (KLF4/TS) vector in the reprogramming cocktail is the main cause of the persistence of all SeV vector genomes.

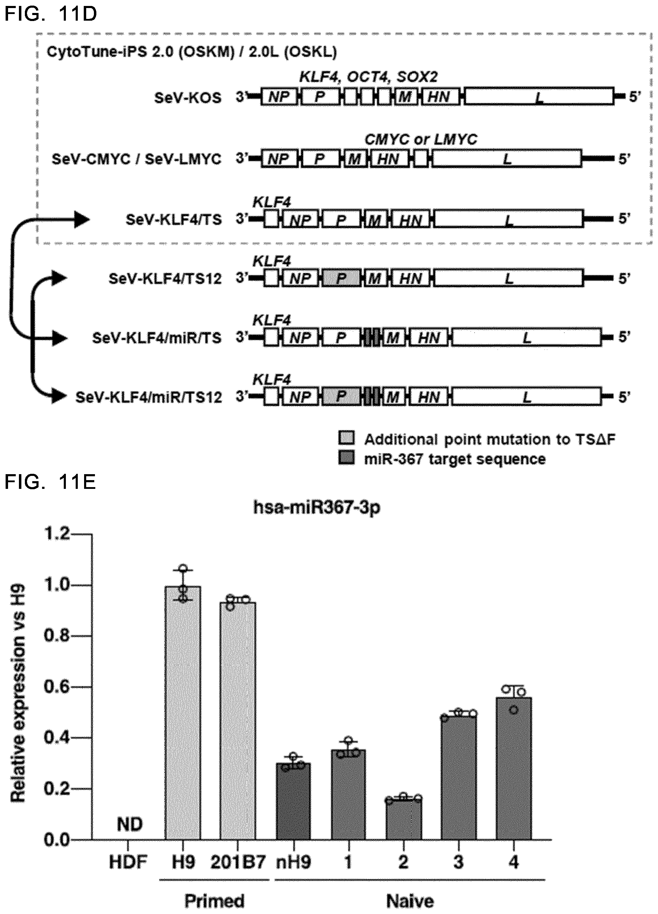

To test this hypothesis, as an alternative to the SeV-KLF4/TS vector, the inventors applied three types of modified SeV-KLF4 vectors: SeV18+KLF4/TS12ΔF (SeV-KLF4/TS12), SeV18+KLF4/PmiR367T2/TSΔF (SeV-KLF4/miR/TS), and SeV18+KLF4/PmiR367T2/TS12 (SeV-KLF4/miR/TS12) vectors (Fig. 11D). The SeV-KLF4/TS12 vector is formed on the basis of the SeV-TS12ΔF vector, which has more point mutations in the P gene to enhance temperature sensitivity (Fig. 11D). The SeV-KLF4/miR/TS vector has hsa-microRNA-367 (miR-367) target sequences (i.e., target sequences of miR-367, not miR-367 itself) inserted in tandem on the 5' side of the P gene of the SeV vector genome (Fig. 11D). miR-367 is known to be specifically and massively expressed in primed human pluripotent stem cells (PSCs), and the inventors confirmed that it is also massively expressed in naive human PSCs compared to HDFs (Fig. 11E). Thus, the inventors presumed that miR-367 receptivity is useful for prompt SeV vector removal in naive human PSCs. SeV-KLF4/miR/TS12 vector incorporates features of both SeV-KLF4/TS12 and SeV-KLF4/miR/TS, which suggests it can further speed up vector removal (Fig. 11D).

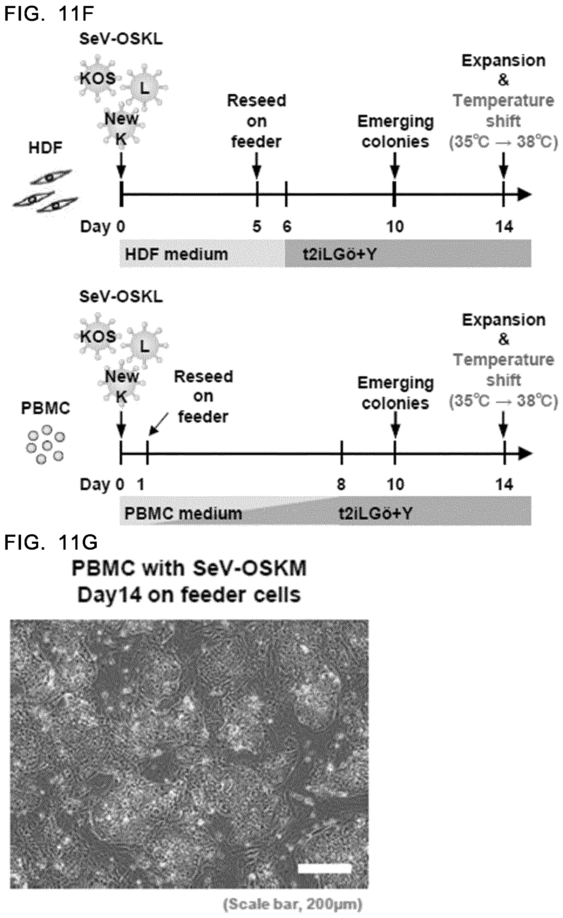

The reprogramming efficiencies of these vector combinations were compared. It was reported that use of a temperature sensitive SeV vector carrying KLF4 gene in the iPSC generation of human fibroblasts failed to generate iPSC at 37°C except for cases where high multiplicity of infection was employed, probably due to the loss of most vectors before the completion of reprogramming. Therefore, the cells were cultured at 35°C from the time of infection until day 14, when enough naive iPSC colonies appeared, and the first passage was then made. After the first passage, the culture temperature was raised to 38°C to remove SeV vectors (Fig. 11F). Reprogramming HDFs using SeV-KOS, SeV-LMYC and the modified SeV-KLF4 vectors led to the appearance of naive iPSC colonies at around day 10, which is consistent with the conventional method using the SeV-KLF4/TS vector.

No previous reports have established naive human iPSCs from human peripheral blood mononuclear cells (PBMCs), and indeed the inventors also could not establish naive human iPSCs from PBMCs using CytoTune-iPS 2.0 (Fig. 11G).

### Example 6. Verification of SeV elimination rate and application to feeder-free naive iPSCs

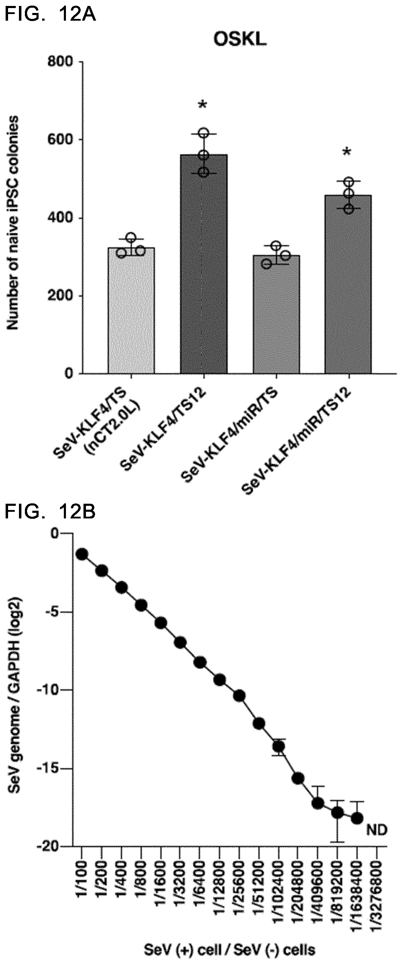

The establishment efficiencies of naive human iPS cells from HDFs using each KLF4 vector were compared (Fig. 12A). Each vector showed no decrease in the establishment efficiency compared to the conventional vector (SeV-KLF4/TS), and OSKL (OSKM but with LMYC replacing CMYC) cocktails containing SeV-KLF4/TS12 or SeV-KLF4/miR/TS12 significantly improved the establishment efficiency compared to the conventional vector.

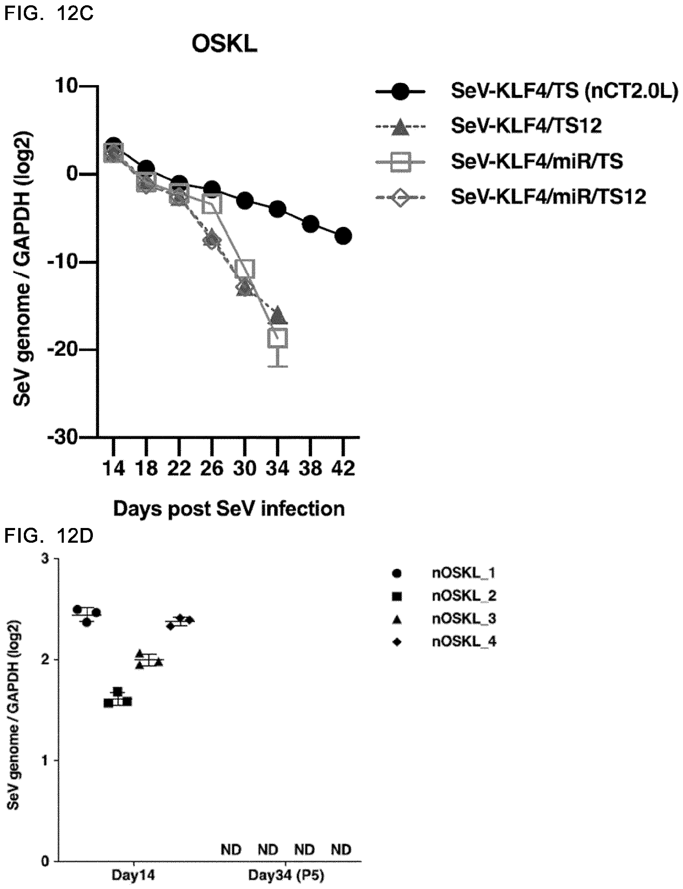

The inventors then detected residual SeV vectors in naive iPSCs generated by these vectors. For the application of iPSC-derived differentiated cells to regenerative medicines, sensitivity to detect one single undifferentiated cell resulting from residual exogenous pluripotent genes out of at least 1×10⁵ cells is required. The inventors' qRT-PCR analysis confirmed a sensitivity that could detect a single SeV-positive cell from 1×10⁶ SeV-negative cells (Fig. 12B). By calculation, even one SeV-positive cell out of 1,638,400 SeV-negative cells can be detected, which is much more sensitive than immunostaining. On this basis, the inventors quantitatively evaluated the residual amount of SeV genome. After the temperature shift at day 14, SeV-KLF4/TS12, SeV-KLF4/miR/TS, and SeV-KLF4/miR/TS12 vectors showed dramatic clearance of the SeV genome, whereas SeV-KLF4/TS levels decreased considerably slower. Notably, SeV-KLF4/miR/TS12 reached the detection limit of qRT-PCR at day 34, which was the fastest of the SeV-KLF4 vectors (Fig. 12C). From these results, the inventors concluded that SeV-KLF4/miR/TS12 vector was the best SeV-KLF4 vector in terms of reprogramming efficiency and vector clearance speed.

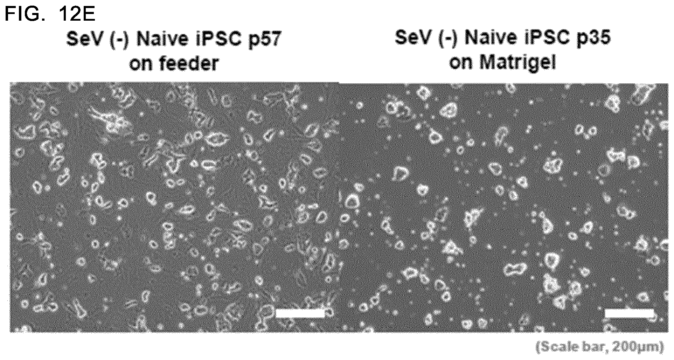

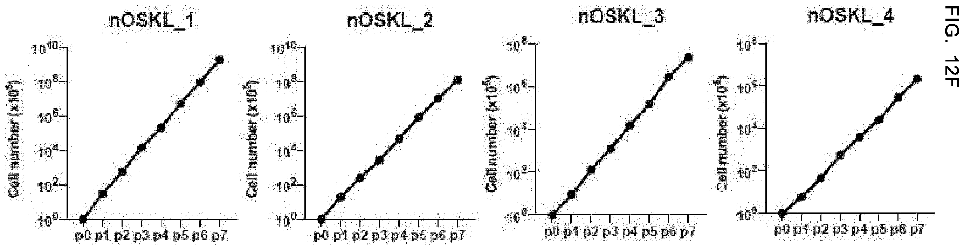

The inventors further tested the SeV-KLF4/miR/TS12 vector in the reprogramming. The inventors were able to generate naive iPSC clones not only from HDFs (nOSKL_1, 2), but also from PBMCs (nOSKL_3, 4) using SeV-KOS, SeV-LMYC and SeV-KLF4/miR/TS12 vectors. The inventors confirmed that SeV disappeared in all nOSKL-iPSC clones at day 34 (Fig. 12D). Interestingly, the inventors also succeeded in establishing and maintaining feeder-free naive iPSCs from HDFs (nOSKL_FF_1, 2) using conditioned medium in which t2iLGo medium was exposed to irradiated mouse embryonic fibroblasts (iMEF) from the start of the reprogramming (Fig. 12E). Next, the cell proliferation rate of nOSKL_1-4 was measured. In all clones, no obvious decrease in cell proliferation rate was observed after passages (Fig. 12F).

### Example 7. Gene expression of established naive iPSCs

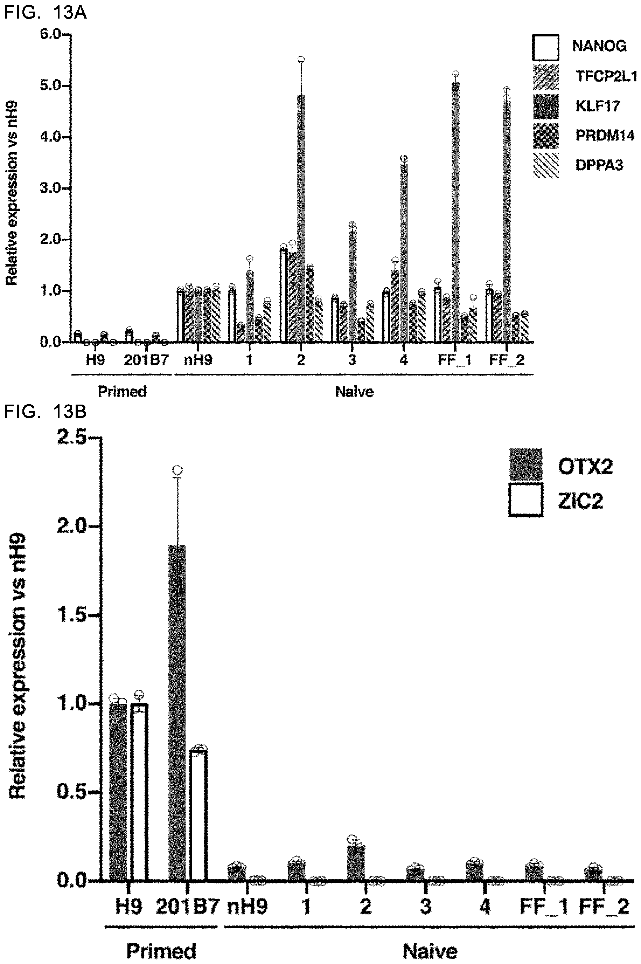

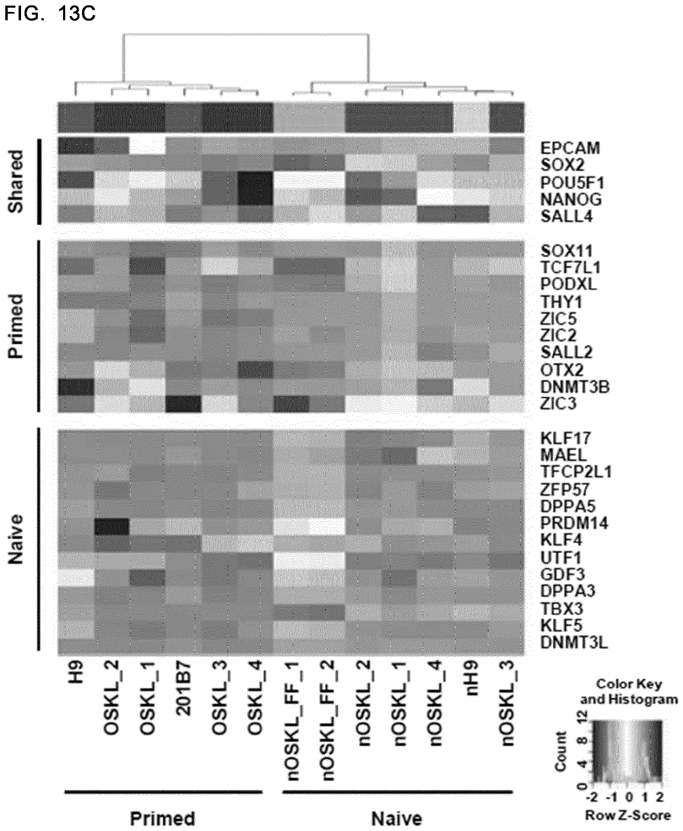

Next, the inventors validated the naive pluripotency specific characteristics of the generated nOSKL-iPSCs. For comparison, the inventors sampled primed human iPSCs (OSKL_1-4) derived from the same somatic cells as nOSKL-iPSCs, primed WA09 H9 embryonic stem cells (ESCs; H9), primed 201B7 iPSCs (201B7), and reset naive H9 ESCs (nH9), which were reset from the primed to naive state by overexpressing KLF2 and NANOG and maintained in the same condition as nOSKL-iPSCs. The expression of naive iPSC-specific markers was verified by qRT-PCR (Fig. 13A). NANOG is known to be expressed in primed iPSCs and markedly increased in naive iPSCs. All naive iPSC lines expressed significantly higher levels of naive iPSC-specific markers compared to primed iPSC lines. Similarly, the expression of primed iPSC-specific markers was verified by qRT-PCR (Fig. 13B). All naive iPSC lines also expressed significantly lower levels of primed iPSC-specific markers compared to primed iPSC lines. RNA-seq was performed on these cell lines to compare the expression of representative pluripotency markers (Fig. 13C). nOSKL-iPSC exhibited pluripotency marker expression almost equivalent to that of nH9. Then the inventors compared the transcriptome of nOSKL-iPSCs with that of primed PSCs and previously published datasets of reset naive PSCs and human inner cell mass (Takashima, Y. et al. Resetting transcription factor control circuitry toward ground-state pluripotency in human. Cell 158, 1254-1269, doi:10.1016/j.cell.2014.08.029 (2014).; Theunissen, T. W. et al. Systematic identification of culture conditions for induction and maintenance of naive human pluripotency. Cell stem cell 15, 471-487, doi:10.1016/j.stem.2014.07.002 (2014).; Yan, L. et al. Single-cell RNA-Seq profiling of human preimplantation embryos and embryonic stem cells. Nature structural & molecular biology 20, 1131-1139, doi:10.1038/nsmb.2660 (2013).). A principal component analysis (PCA) revealed that nOSKL-iPSCs are clearly distinguished from primed PSCs and share almost identical characteristics with nH9 (Fig. 13D). The naive human iPSCs established by the inventors most resembled epiblasts.

### Example 8. Verification of methylation and X chromosome reactivation of established naive iPSCs

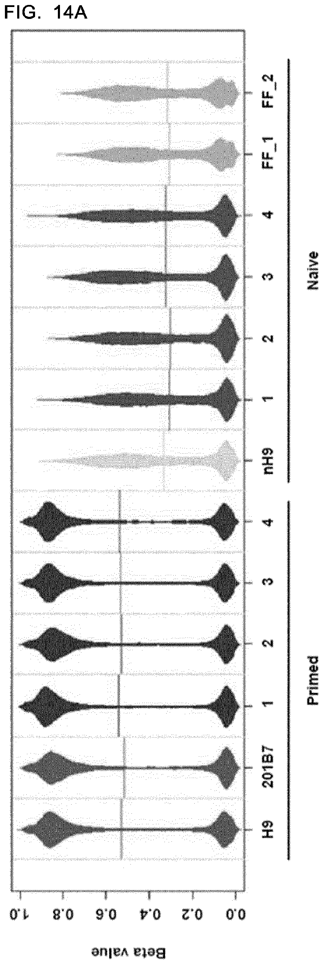

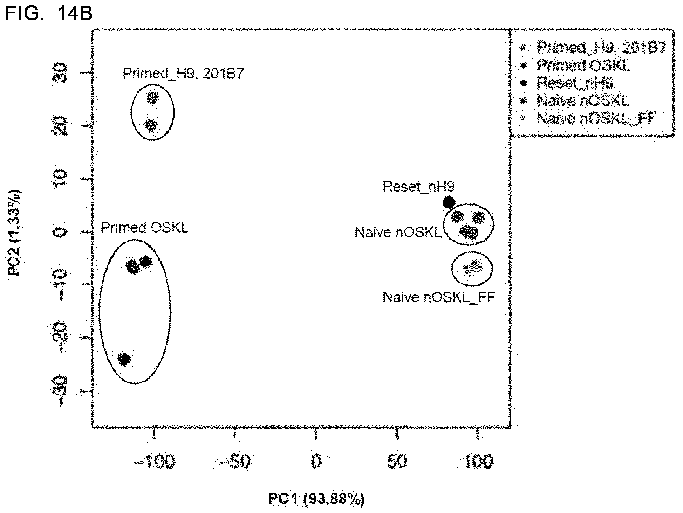

Methylation arrays were performed in the cell lines used for the RNA-seq in Example 7 (Fig. 14A). nOSKL-iPSCs, including feeder-free naive iPSCs (FF_1-2), exhibited a global DNA demethylation state compared to primed iPSCs. PCA of the methylation array data clearly distinguished naive iPSCs from primed iPSCs (Fig. 14B).

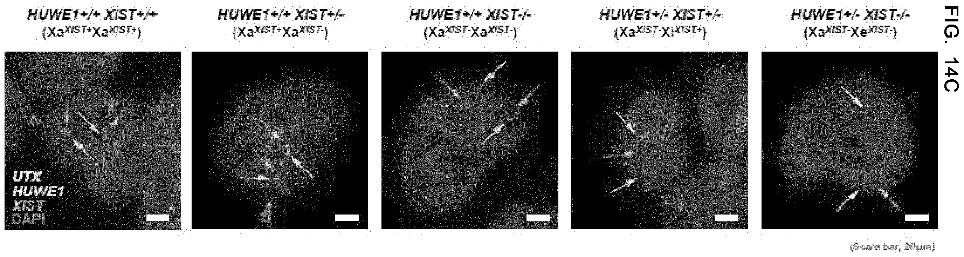

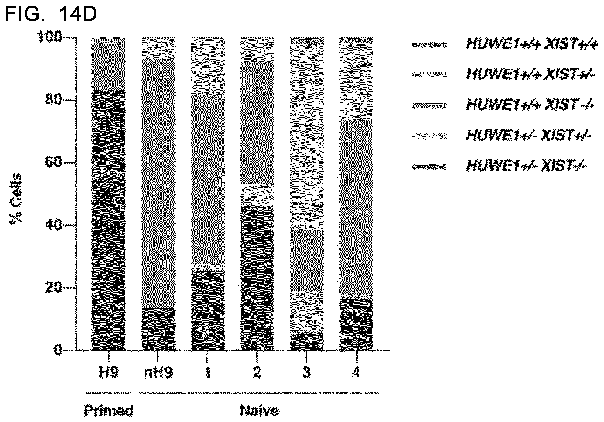

X chromosome reactivation was verified by RNA-FISH (Fig. 14C). HUWE1 is a gene expressed in the activated X chromosome. XIST is an IncRNA that originally acts to inactivate the X chromosome, but XIST is positive in both activated X chromosomes of preimplantation epiblasts which have similar characteristics to naive iPSCs, which is an apparently contradictory finding (far left panel in Fig. 14C). The more the cells resembled the primed iPSC, the more the gene expression pattern changed to that shown in the far right panel in Fig. 14C. Basically, most cells of the primed iPSCs exhibited the gene expression pattern shown in the far right panel in Fig. 14C (HUWEI+/-, XIST-/-). In addition, RNA-FISH was performed on the established clones (Fig. 14D). Although there was variation among clones, nOSKL-iPSCs clearly exibited a pattern similar to that of preimplantation epiblasts compared to primed PSCs. These observations confirmed that nOSKL-iPSCs are in a naive state.

### Example 9. Evaluation of mitochondrial function of established naive iPSCs

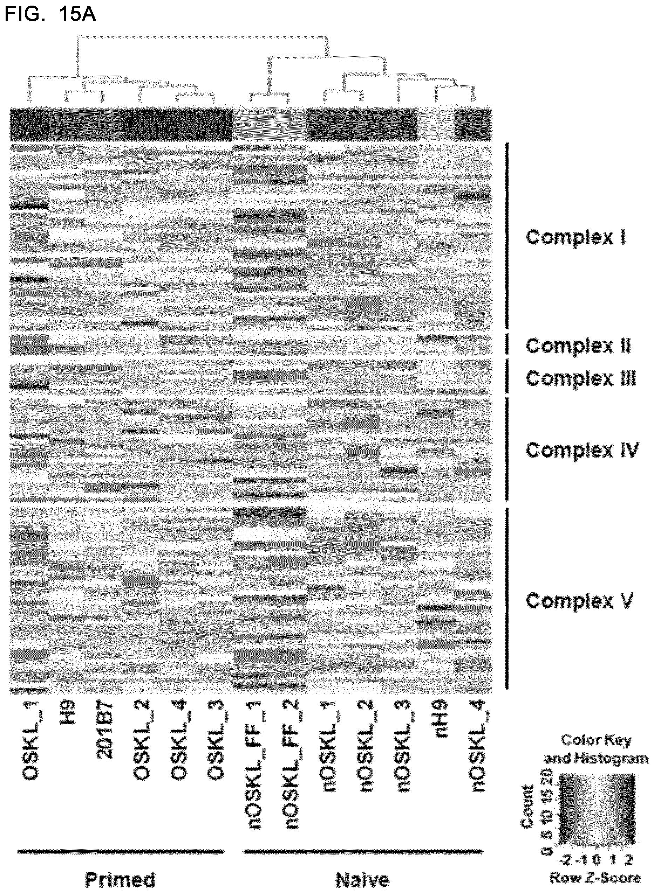

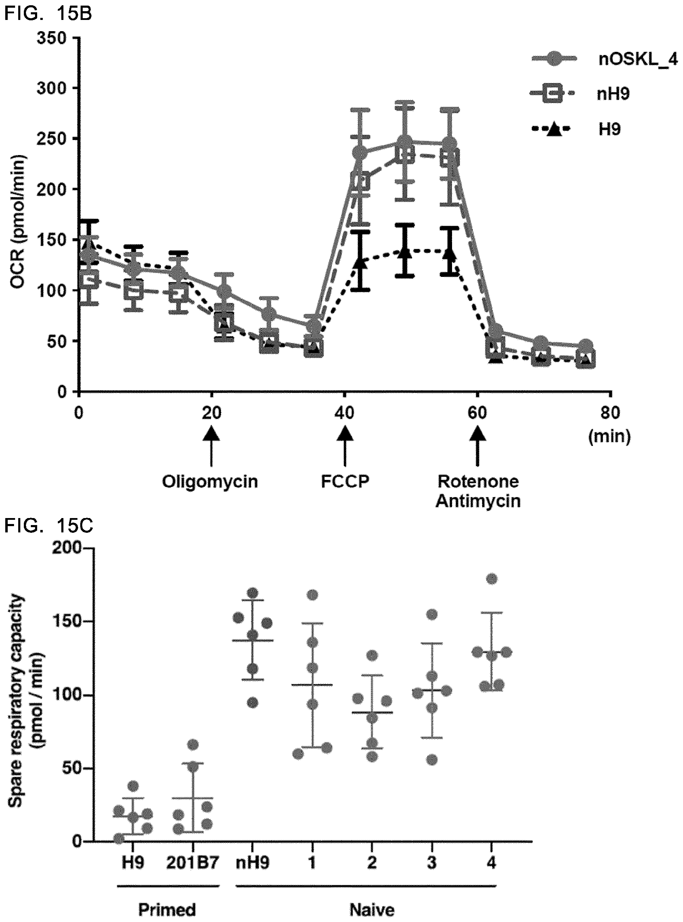

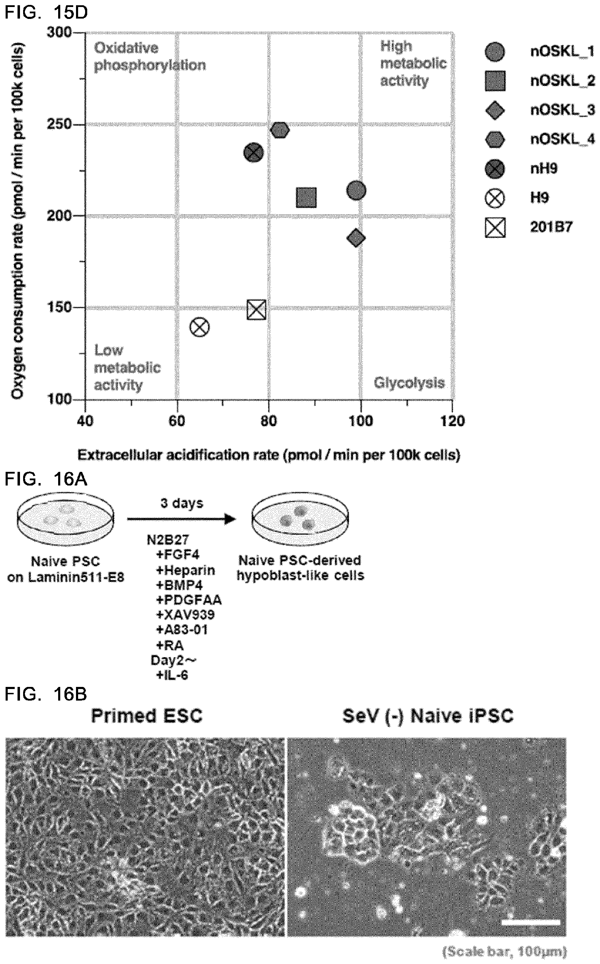

Based on the RNA-seq data from Example 7, the inventors compared the expression of mitochondrial genes associated with oxidative phosphorylation (Fig. 15A). The expression of mitochondrial genes was higher in the naive iPSCs than in the primed iPSCs, suggesting that mitochondrial functions such as aerobic metabolism are activated in the naive iPSCs. Next, the inventors compared the metabolic capacities of the naive and primed iPSCs using extracellular flux analyzer (Fig. 15B). Oxygen consumption rate (OCR) was significantly increased in the naive iPSCs compared to the primed iPSCs after administration of FCCP, suggesting that the naive iPSCs have superior spare respiratory capacity. The inventors quantitatively compared spare respiratory capacity, one of the indicators of aerobic metabolic capacity, of the naive and primed iPSCs (Fig. 15C). As well as nH9, nOSKL-iPSCs showed higher spare respiratory capacity than the primed iPSCs. In addition, extracellular acidification rate (ECAR), an indicator of glycolytic metabolic function, and OCR, an indicator of aerobic metabolic function, were examined (Fig. 15D). The naive iPSCs showed higher ECAR and OCR than the primed iPSCs, suggesting that the naive iPSCs are in a high-energy metabolic state.

### Example 10. Verification of differentiation ability of established naive iPSCs into primitive endoderm

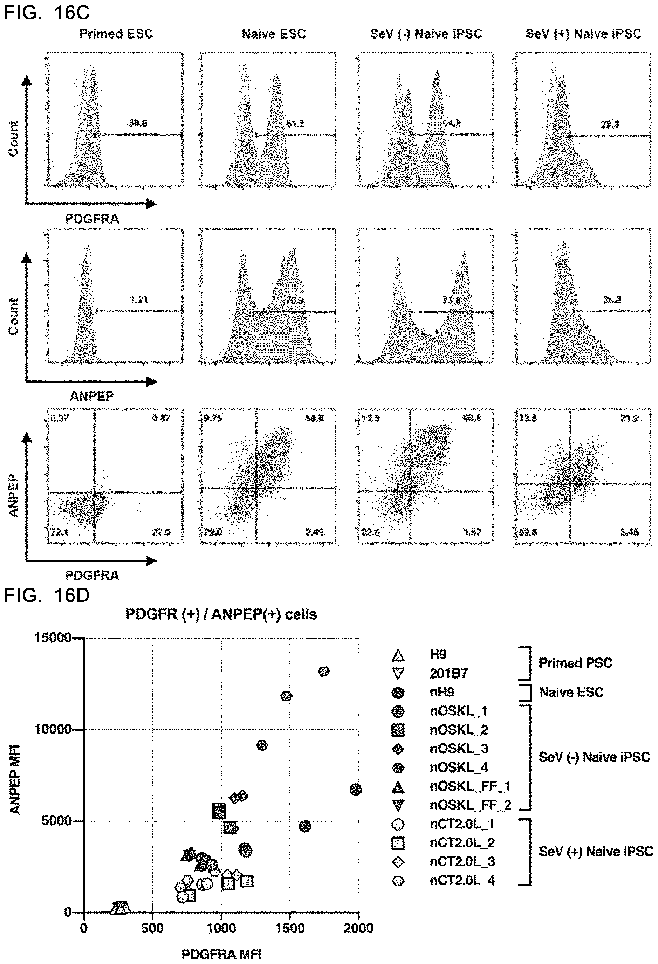

Naive PSCs are similar to preimplantation epiblasts and have the ability to differentiate into extraembryonic tissue. This feature distinguishes naive PSCs from primed PSCs. Primitive endoderm can hardly be differentiated from primed iPSCs, but can be differentiated from naive iPSCs. Therefore, the inventors examined the ability of nOSKL-iPSCs to differentiate into naive PSC-derived primitive endoderm in a defined differentiation medium (Fig. 16A). Microscopic images of primed ESCs and nOSKL-iPSCs on day 3 after the start of differentiation induction are shown in Fig. 16B. nOSKL-iPSCs clearly showed a morphology similar to that of endodermal cells compared to primed ESCs. Next, the inventors compared and verified the expression levels of primitive endoderm-specific markers PDGFRA and ANPEP by using flow cytometry (Fig. 16C). n9 and nOSKL-iPSCs highly expressed primitive endoderm-specific markers PDGFRA and ANPEP, and showed the good differentiation ability into primitive endoderm. The primed PSCs and the naive iPSCs with residual SeVs established with the conventional SeV-KLF4/TS vector (CytoTune-iPSC 2.0L) showed very low expression levels of PDGFRA and ANPEP, indicating the significantly lower differentiation ability into primitive endoderm. The median signal value in PDGFRA/ANPEP co-positive cells was examined (Fig. 16D). The primed iPSCs and the naive iPSCs with residual SeVs are distributed in the lower left side of Fig. 16D, whereas nH9 and the naive iPSCs established by the present method are distributed in the upper right side, suggesting that naive iPSCs established by the present method have the superior differentiation ability similar to nH9.

### Example 11. Verification of differentiation ability of established naive iPSCs into trophectoderm

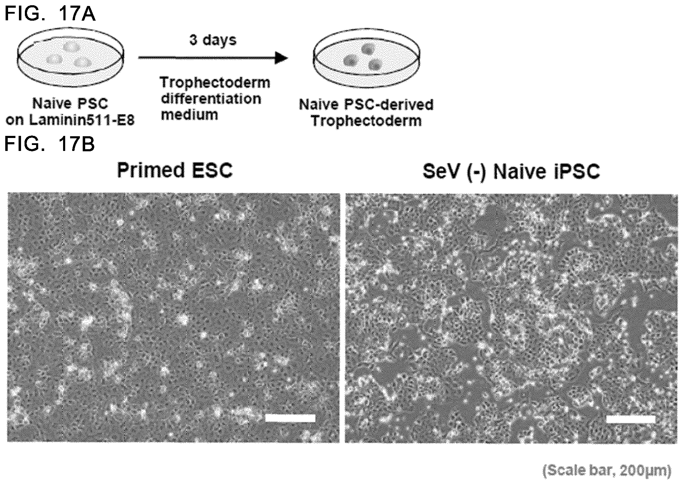

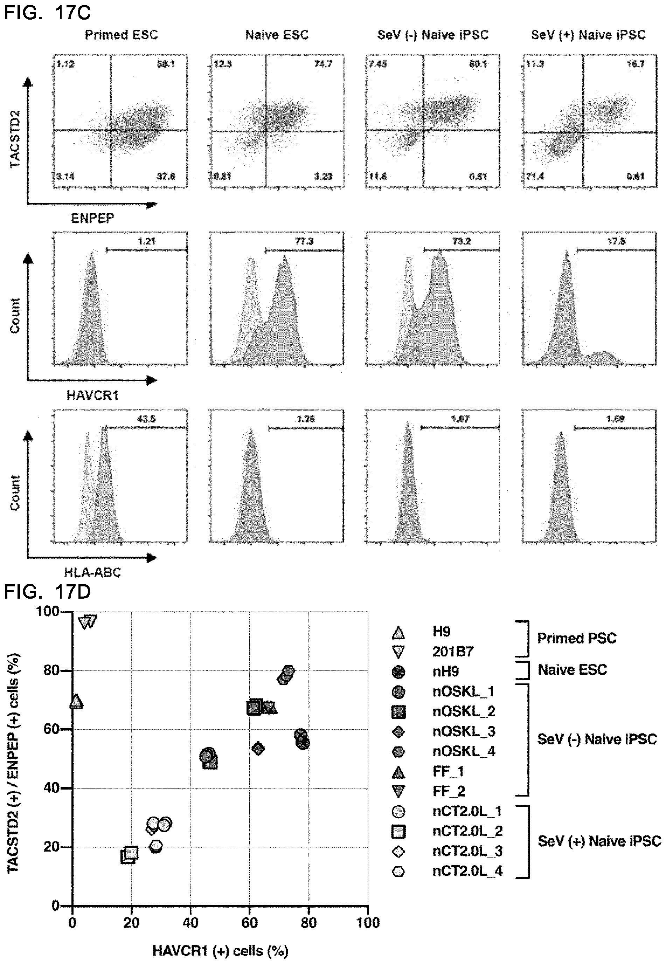

Trophectoderm (TE) also can hardly be differentiated from primed iPSCs, but can be differentiated from naive iPSCs. The inventors examined the ability of nOSKL-iPSCs to differentiate into naive PSC-derived Trophectoderm (TE) in a defined differentiation medium (Fig. 17A). Microscopic images of primed ESCs and nOSKL-iPSCs on day 3 after the start of differentiation induction are shown in Fig. 17B. The morphologies of the primed ESCs and nOSKL-iPSCs were clearly different on day 3 after the start of differentiation induction. Next, the inventors compared and verified the expression levels of trophectoderm-specific markers TACSTD2, ENPEP, and HAVCR1 and negative marker HLA-ABC by using flow cytometry (Fig. 17C). n9 and nOSKL-iPSCs highly expressed TE-specific marker HAVCR1 and did not express HLA-ABC, indicating the good differentiation ability into TE. The primed PSCs did not express HAVCR1 at all, and almost half of them were HLA-ABC positive, suggesting that they were hardly differentiated into TE. The naive iPSCs with residual SeVs established with the conventional SeV-KLF4/TS vector (CytoTune-iPSC 2.0L) showed very low expression levels of TACSTD2, ENPEP and HAVCR1, indicating the significantly lower differentiation ability into TE, as well as the primed iPSCs. The percentages of TACSTD2/ENPEP co-positive cells and HAVCR1-positive cells were examined by using flow cytometry (Fig. 17D). The primed iPSCs and the naive iPSCs with residual SeVs are distributed on the left side of Fig. 17D, whereas nH9 and the naive iPSCs established by the present method are distributed on the right side of Fig. 17D, suggesting that the naive iPSCs established by the present method have the superior differentiation ability similar to nH9. Interestingly, several clones of nOSKL-iPSCs expressed TACSTD2 and ENPEP at higher levels than nH9.

In summary, the inventors developed a dramatically faster SeV genome removal system for the generation of naive human iPSCs from somatic cells by improving the structure of the SeV-KLF4 vector. As a result, the inventors were able to obtain naive human iPSCs with robust transgene independency at early passages and superior naive-specific differentiation ability compared to naive iPSCs generated by the conventional method. In addition, the inventors succeeded in the reprogramming of PBMCs to naive human iPSCs by changing the combination of reprogramming factors to SeV-OSKL. Moreover, the inventors established naive human iPSCs from dermal fibroblasts in a feeder-free environment using iMEF conditioned medium. These innovations greatly expand the application of naive human iPSCs to research on early development and also broaden their clinical applications.

## Claims

1. A method for producing naive induced pluripotent stem cells from human somatic cells, comprising the following steps (1) to (3):
(1) introducing one or more vectors containing a reprogramming factor into human somatic cells,
(2) culturing said somatic cells in the presence of a naive medium, and
(3) after step (2), culturing the resulting cells in the presence of the naive medium under the condition in which the amount of the vectors per the somatic cell is reduced to 30% or less of that at the start of step 3.

2. The method according to claim 1, wherein the one or more vectors are minus-stranded RNA virus vectors.

3. The method according to claim 2, wherein the one or more vectors are paramyxovirus vectors.

4. The method according to claim 3, wherein the one or more vectors are Sendai virus vectors.

5. The method according to any one of claims 1 to 4, wherein step (2) is started 1 to 10 days after step (1).

6. The method according to any one of claims 1 to 5, wherein step (2) is performed for 1 to 20 days.

7. The method according to any one of claims 1 to 6, wherein the one or more vectors are selected from the group consisting of a temperature-sensitive vector, a vector containing a target sequence of a microRNA specific for induced pluripotent stem cells, and a temperature-sensitive vector containing a target sequence of a microRNA specific for induced pluripotent stem cells.

8. The method according to claim 7, wherein the one or more vectors are temperature-sensitive vectors.

9. The method according to claim 8, wherein the one or more vectors are vectors that are temperature-sensitive in the cell culture environment for establishing naive pluripotent stem cells.

10. The method according to claim 9, wherein the one or more vectors are Sendai virus vectors with TS7 mutation which has TS mutation (G69E/T116A/A183S mutations in M protein, A262T/G264R/K461G mutations in HN protein, L511F mutation in P protein, and N1197S/K1795E mutations in L protein) and Y942H/L1361C/L1558I mutations in L protein, TS12 mutation which has TS mutation and D433A/R434A/K437A mutations in P protein, or TS15 mutation which has TS mutation, D433A/R434A/K437A mutations in P protein and L1361C/L1558I mutations in L protein.

11. The method according to any one of claims 7 to 10, wherein step (3) comprises culturing the resulting cells at 38°C or higher.

12. The method according to any one of claims 7 to 11, wherein the vector containing the target sequence of the microRNA specific for induced pluripotent stem cells is a minus-stranded RNA virus vector, and wherein the target sequence of the microRNA is in the translated region, 5'UTR, or 3'UTR of NP gene or P gene.

13. The method according to claim 12, wherein the microRNA is miR-367.

14. The method according to any one of claims 1 to 13, wherein the reprogramming factor includes OCT gene, SOX gene, MYC gene and/or KLF gene.

15. The method according to claim 14, wherein the one or more vectors include a vector containing OCT gene, SOX gene, and KLF gene, a vector containing MYC gene, and a vector containing KLF gene.

16. The method according to claim 15, wherein the vector containing MYC gene is a Sendai virus vector with TS15 mutation.

17. The method according to claim 15 or 16, wherein the vector containing OCT gene, SOX gene, and KLF gene, and the vector containing KLF gene are Sendai virus vectors with TS12 mutation.

18. The method according to any one of claims 1 to 17, wherein in step (3), the amount of the vectors per the somatic cell is reduced to 30% or less of that at the start of step 3 within 12 days from the start of step 3.

19. The method according to any one of claims 1 to 18, wherein the naive medium contains one or more compounds selected from a Leukemia inhibitory factor (LIF), a MEK inhibitor, a GSK3 inhibitor, a cAMP production stimulator, a TGF-β inhibitor, and a PKC inhibitor.

20. The method according to claim 19, wherein the naive medium is a medium selected from the group consisting of t2iLGo, 5iLAF, and tt2iLGo.

21. The method according to any one of claims 1 to 20, wherein steps (2) and (3) are performed in the absence of feeder cells.

22. The method according to any one of claims 1 to 21, wherein the human somatic cells are mononuclear cells or fibroblasts.

23. A kit for producing naive induced pluripotent stem cells from human somatic cells, comprising:
one or more vectors containing a reprogramming factor, wherein the one or more vectors are selected from the group consisting of a temperature-sensitive vector, a vector containing a target sequence of a microRNA specific for induced pluripotent stem cells, and a temperature-sensitive vector containing a target sequence of a microRNA specific for induced pluripotent stem cells; and
a naive medium.

24. The kit according to claim 23, wherein the one or more vectors are minus-stranded RNA virus vectors.

25. The kit according to claim 24, wherein the one or more vectors are paramyxovirus vectors.

26. The kit according to claim 25, wherein the one or more vectors are Sendai virus vectors.

27. The kit according to any one of claims 23 to 26, wherein the one or more vectors are temperature-sensitive vectors.

28. The kit according to claim 27, wherein the one or more vectors are vectors that are temperature-sensitive in the cell culture environment for establishing naive pluripotent stem cells.

29. The kit according to claim 28, wherein the one or more vectors are Sendai virus vectors with TS7 mutation which has TS mutation (G69E/T116A/A183S mutations in M protein, A262T/G264R/K461G mutations in HN protein, L511F mutation in P protein, and N1197S/K1795E mutations in L protein) and Y942H/L1361C/L1558I mutations in L protein, TS12 mutation which has TS mutation and D433A/R434A/K437A mutations in P protein, or TS15 mutation which has TS mutation, D433A/R434A/K437A mutations in P protein and L1361C/L1558I mutations in L protein.

30. The kit according to any one of claims 23 to 29, wherein the vector containing the target sequence of the microRNA specific for induced pluripotent stem cells is a minus-stranded RNA virus vector, and wherein the target sequence of the microRNA is in the translated region, 5'UTR, or 3'UTR of NP gene or P gene.

31. The kit according to claim 30, wherein the microRNA is miR-367.

32. The kit according to any one of claims 23 to 31, wherein the reprogramming factor includes OCT gene, SOX gene, MYC gene and/or KLF gene.

33. The kit according to claim 32, wherein the one or more vectors include a vector containing OCT gene, SOX gene, and KLF gene, a vector containing MYC gene, and a vector containing KLF gene.

34. The kit according to claim 33, wherein the vector containing MYC gene is a Sendai virus vector with TS15 mutation.

35. The kit according to claim 33 or 34, wherein the vector containing OCT gene, SOX gene, and KLF gene, and the vector containing KLF gene are Sendai virus vectors with TS12 mutation.

36. The kit according to any one of claims 23 to 35, wherein the naive medium contains one or more compounds selected from a Leukemia inhibitory factor (LIF), a MEK inhibitor, a GSK3 inhibitor, a cAMP production stimulator, a TGF-β inhibitor, and a PKC inhibitor.

37. The kit according to claim 36, wherein the naive medium is a medium selected from the group consisting of t2iLGo, 5iLAF, and tt2iLGo.

38. The kit according to any one of claims 23 to 37, wherein the human somatic cells are mononuclear cells or fibroblasts.

39. A culture supernatant of the naive induced pluripotent stem cells produced by the method according to any one of claims 1 to 22.

40. A cosmetic product containing as raw material a culture supernatant of the naive induced pluripotent stem cells produced by the method according to any one of claims 1 to 22.
